(19) Europäisches Patentamt

European Patent Office

Office européen des brevets

(11) **EP 1 530 043 A1**

(12) **EUROPEAN PATENT APPLICATION**
published in accordance with Art. 158(3) EPC

(43) Date of publication:
**11.05.2005 Bulletin 2005/19**

(51) Int Cl.7: **G01N 27/416**, G01N 27/327,
G01N 33/53, G01N 37/00,
C12M 1/00, C12M 1/34,
C12Q 1/68

(21) Application number: **04714496.9**

(22) Date of filing: **25.02.2004**

(86) International application number:
**PCT/JP2004/002205**

(87) International publication number:
**WO 2004/077041 (10.09.2004 Gazette 2004/37)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **26.02.2003 JP 2003049614
20.02.2004 JP 2004044368**

(71) Applicant: **Kabushiki Kaisha Toshiba
Tokyo 105-8001 (JP)**

(72) Inventors:
• **O'UCHI, Shin-ichi
(JP)**
• **FUNAKI, Hideyuki
(JP)**

• **HONGO, Sadato
(JP)**
• **HASHIMOTO, Koji
(JP)**
• **GEMMA, Nobuhiro
(JP)**
• **OKADA, Jun
(JP)**
• **TAKAHASHI, Masayoshi
(JP)**

(74) Representative: **Granleese, Rhian Jane
Marks & Clerk
90 Long Acre
London WC2E 9RA (GB)**

(54) **ANALYTIC CHIP FOR QUANTIFYING NUCLEIC ACID CONCENTRATION, ANALYTIC DEVICE FOR QUANTIFYING NUCLEIC ACID CONCENTRATION AND ANALYTIC METHOD FOR QUANTIFYING NUCLEIC ACID CONCENTRATION**

(57)   The present invention includes a plurality of working electrodes (141) on which the same type of nucleic acid probes each having a nucleic acid complementary to a target nucleic acid are immobilized and which have different sensor areas and a normalization circuit which normalizes detection signals obtained by the working electrodes (141) with respect to the respective sensor areas.

FIG. 8

**Description**

Technical Field

The present invention relates to a nucleic acid concentration quantitative analysis chip, a nucleic acid concentration quantitative analysis apparatus, and a nucleic acid concentration quantitative analysis method in which a concentration of a target nucleic acid contained in a specimen is measured.

Background Art

There have heretofore been DNA chips for nucleic acid detection to assay whether or not a specimen contains a target nucleic acid (Patent Document 1: Jpn. Pat. Appln. KOKAI Publication No. 10-146183).

However, to perform gene expression analysis , it is necessary to measure a concentration of a target nucleic acid included in the specimen in a broad measurable range with a high precision. These specifications are not achieved by the above-described DNA chips.

Disclosure of Invention

An object of the present invention is to provide a nucleic acid concentration quantitative analysis chip, a nucleic acid concentration quantitative analysis apparatus, and a nucleic acid concentration quantitative analysis method in which a nucleic acid concentration is measured in a broad dynamic range with a high precision.

In an aspect of the present invention, there is provided a nucleic acid concentration quantitative analysis chip comprising a plurality of nucleic acid sensors having different sensor areas on which nucleic acid probes each having a nucleic acid complementary to a target nucleic acid are immobilized and a first normalization unit which normalizes first detection signals obtained by the nucleic acid sensors with respect to the respective sensor areas.

In another aspect of the invention, there is provided a nucleic acid concentration quantitative analysis apparatus comprising a plurality of nucleic acid sensors having different sensor areas on which nucleic acid probes each having a nucleic acid complementary to a target nucleic acid are immobilized, background level sensors which have the different sensor areas on which the nucleic acid probes having the nucleic acids complementary to the target nucleic acids are not immobilized, a first normalization unit which normalizes first detection signals obtained by the nucleic acid sensors with respect to the respective sensor areas, a second normalization unit which normalizes second detection signals obtained by the background level sensors with respect to the respective sensor areas, a first current-to-voltage conversion unit which converts a first output signal current of the first normalization unit to a first output voltage, a second current-to-voltage conversion unit which converts a second output signal current of the second normalization unit to a second output voltage, an A/D conversion unit which A/D converts the first output voltage to generate first digital data and which A/D converts the second output voltage to generate second digital data, and a subtraction unit which subtracts the second digital data from the first digital data.

In still another aspect of the invention, there is provided a nucleic acid concentration quantitative analysis apparatus comprising a plurality of nucleic acid sensors having different sensor areas on which nucleic acid probes each having a nucleic acid complementary to a target nucleic acid are immobilized, background level sensors which have the different sensor areas on which the nucleic acid probes having the nucleic acids complementary to the target nucleic acids are not immobilized, a first normalization unit which normalizes first detection signals obtained by the nucleic acid sensors with respect to the respective sensor areas, a second normalization unit which normalizes second detection signals obtained by the background level sensors with respect to the respective sensor areas, a first current-to-voltage conversion unit which converts a first output signal current of the first normalization unit to a first output voltage, a second current-to-voltage conversion unit which converts a second output signal current of the second normalization unit to a second output voltage, a subtraction unit which subtracts the second output voltage from the first output voltage, and an A/D conversion unit which A/D converts a third output voltage of the subtraction unit.

In still another aspect of the invention, there is provided a nucleic acid concentration quantitative analysis chip comprising a plurality of nucleic acid sensors having different sensor areas on which nucleic acid probes each having a nucleic acid complementary to a target nucleic acid are immobilized, background level sensors which have the different sensor areas on which the nucleic acid probes having the nucleic acids complementary to the target nucleic acids are not immobilized, a subtraction unit which subtracts a second detection signal of the background level sensor from a first detection signal of the nucleic acid sensor, and a normalization unit which normalizes a subtraction output signal of the subtraction unit.

In still another aspect of the invention, there is provided a nucleic acid concentration quantitative analysis apparatus comprising a nucleic acid concentration quantitative analysis chip including a plurality of nucleic acid sensors having different sensor areas on which nucleic acid probes each having a nucleic acid complementary to a target nucleic acid are immobilized and a first normalization unit which normalizes a detection signal of the nucleic acid sensor with respect to the sensor area to output a normalization signal, and a nucleic acid concentration calculation device which calculates a nucleic acid concentration based on the normalization signal.

In still another aspect of the invention, there is provided a nucleic acid concentration quantitative analysis method comprising normalizing detection signals of a plurality of nucleic acid sensors on which nucleic acid probes each having a nucleic acid complementary to a target nucleic are immobilized and which have different sensor areas with respect to sensor areas to output a normalization signal, and calculating a nucleic acid concentration based on the normalization signal.

Brief Description of Drawings

FIG. 1 is a diagram showing a entire configuration of a nucleic acid concentration quantitative analysis apparatus according to a first embodiment of the present invention;
FIG. 2 is a diagram showing a modification of an appearance configuration of a nucleic acid detection chip according to the embodiment;
FIG. 3 is a block diagram of a measurement circuit of the nucleic acid detection chip according to the embodiment;
FIG. 4 is a diagram showing one example of a detailed configuration of a module 135 according to the embodiment;
FIG. 5 is a diagram showing a detailed configuration of an improved module 135 according to the embodiment;
FIG. 6 is a diagram showing one example of a device sectional view of the nucleic acid detection chip according to the embodiment;
FIG. 7 is a schematic diagram of an electrode area of a working electrode according to the embodiment;
FIG. 8 is a diagram showing a detection result using a probe series according to the embodiment;
FIG. 9 is a flowchart of an operation of a nucleic acid concentration quantitative analysis apparatus according to the embodiment;
FIG. 10 is a flowchart of one example of a concrete process of calibration according to the embodiment;
FIG. 11 is a flowchart showing details of a current value acquisition process according to the embodiment;
FIG. 12 is a flowchart of details of a measurement process according to the embodiment;
FIG. 13 is a diagram showing a detailed configuration of a circuit for performing normalization according to the embodiment;
FIG. 14 is a schematic diagram of the nucleic acid detection chip according to a modification of the nucleic acid concentration quantitative analysis apparatus according to the embodiment;
FIG. 15 is a diagram showing a detailed configuration example of a circuit showing a subtraction circuit according to the embodiment;
FIG. 16 is a diagram showing a modification of the subtraction circuit according to the embodiment;
FIG. 17 is a diagram showing an analysis process flow using a chip with an electrode for background measurement according to the embodiment;
FIG. 18 is a detailed flowchart of a current value acquisition operation according to the embodiment;
FIG. 19 is a schematic diagram of the nucleic acid detection chip according to a further modification of the embodiment;
FIG. 20 is an analysis process flowchart using the chip with the electrode for saturated level calibration according to the embodiment;
FIG. 21 is a detailed flowchart of a current value and bit pattern acquisition operation according to the embodiment;
FIG. 22 is a detailed process flowchart of a measurement process (S2) using the chip with the electrode for saturated level calibration according to the embodiment;
FIG. 23 is a plan view of a modification relating to an electrode arrangement of a three-electrode system according to the embodiment;
FIG. 24 is a plan view of a modification of another electrode arrangement according to the embodiment;
FIG. 25 is a diagram showing one example of a configuration of a compensation circuit according to the embodiment;
FIG. 26 is a diagram showing one example of the compensation circuit according to the embodiment;
FIG. 27 is a top plan view showing a modification of the nucleic acid detection chip according to the embodiment;
FIG. 28 is a perspective view of a cassette for holding the chip according to the embodiment;
FIG. 29 is a flowchart of one example of a saturated level, background level, and threshold value decision algorithm according to the embodiment;
FIG. 30 is a diagram showing a modification of the module according to the embodiment;
FIG. 31 is a schematic diagram of a current detection circuit and normalization circuit according to the embodiment;

FIG. 32 is an explanatory view for describing a problem of a background current according to a second embodiment of the present invention;

FIG. 33 is a diagram showing one example of a circuit configuration for solving a problem according to the embodiment;

FIG. 34 is a diagram showing one example of a current-to-voltage conversion circuit according to the embodiment;

FIG. 35 is a diagram showing one example of the current-to-voltage conversion circuit according to the embodiment;

FIG. 36 is a diagram showing one example of the current-to-voltage conversion circuit according to the embodiment;

FIG. 37 is a diagram showing one example of the current-to-voltage conversion circuit according to the embodiment;

FIG. 38 is a diagram showing one example of the configuration of the module according to a third embodiment of the present invention;

FIG. 39 is a diagram showing one example of the configuration of the module according to a fourth embodiment of the present invention;

FIG. 40 is a diagram showing one example of the configuration of the module according to a fifth embodiment of the present invention;

FIG. 41 is a diagram showing one example of the configuration of the module according to a sixth embodiment of the present invention;

FIG. 42 is a diagram showing one example of the configuration of a capacitor Cb according to the embodiment;

FIG. 43 is an explanatory view of an overlap factor $\gamma$ according to a seventh embodiment of the present invention;

FIG. 44 is a diagram showing a main part section of the nucleic acid concentration quantitative analysis chip according to an eighth embodiment of the present invention;

FIG. 45 is a schematic diagram showing another example of the nucleic acid concentration quantitative analysis chip according to the embodiment;

FIG. 46 is an explanatory view of a nucleic acid concentration range according to the embodiment;

FIG. 47 is a diagram showing a detected graph according to the embodiment;

FIG. 48 is a diagram showing a graph example in which a nucleic acid probe fixing region area is varied according to the embodiment;

FIGS. 49 to 81 are diagrams showing configuration examples of the chip according to the embodiment; and

FIGS. 82 to 85 are diagrams showing an example of a functional block of a nucleic acid concentration quantitative analysis apparatus according to the embodiment.

Best Mode of Carrying Out the Invention

Embodiments of the present invention will hereinafter be described with reference to the drawings.

(First Embodiment)

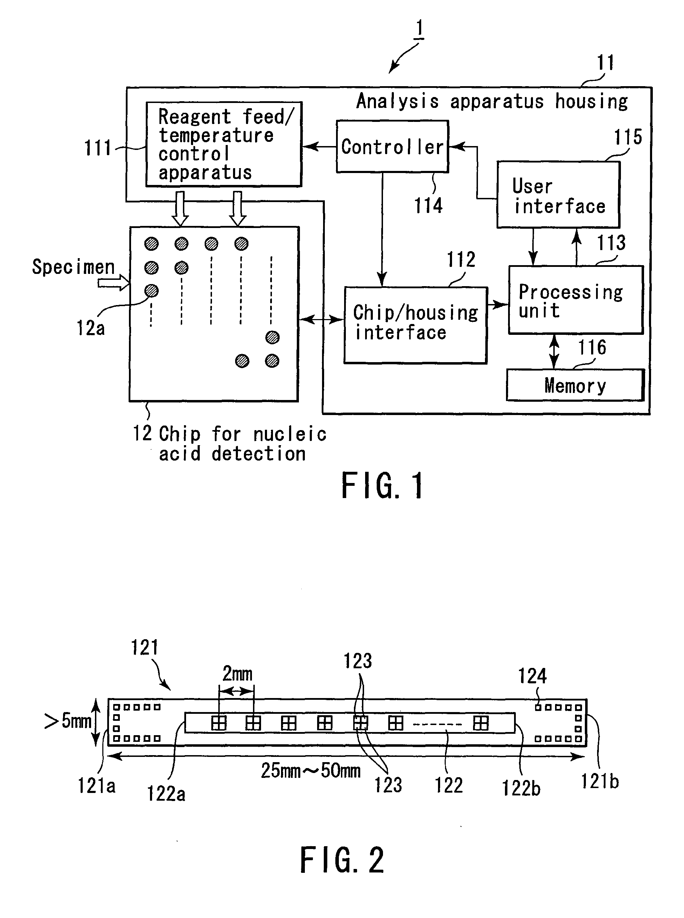

FIG. 1 is a diagram showing an entire configuration of a nucleic acid concentration quantitative analysis apparatus according to a first embodiment of the present invention. As shown in FIG. 1, a nucleic acid concentration quantitative analysis apparatus 1 includes an analysis apparatus housing 11 and a nucleic acid detection chip 12. In this nucleic acid concentration quantitative analysis apparatus 1, the nucleic acid detection chip 12 is attached to the analysis apparatus housing 11 to quantitatively analyze the concentration of a nucleic acid detected from sensors 12a arranged in an array in the nucleic acid detection chip 12.

The analysis apparatus housing 11 includes a reagent feed/temperature control apparatus 111, chip/housing interface 112, processing unit 113, control mechanism 114, user interface 115, and storage unit 116. The reagent feed/temperature control apparatus 111 includes a reagent feed apparatus and a temperature control apparatus. The reagent feed apparatus feeds reagent such as a buffer reagent and an intercalating reagent into the nucleic acid detection chip 12, and remove waste reagent from the nucleic acid detection chip 12. The temperature control apparatus includes a heating apparatus or a cooling apparatus which controls temperatures of the respective sensors 12a of the nucleic acid detection chip 12. The temperature control apparatus keeps the nucleic acid detection chip 12 at a desired temperature based on the detected temperature of a temperature sensor (not shown).

The chip/housing interface 112 is electrically connected to an electronic circuit in the nucleic acid detection chip 12. The chip/housing interface 112 outputs various electric signals obtained from the nucleic acid detection chip 12 to the processing unit 113.

The processing unit 113 effectuates, for example, a function equal to that of a personal computer together with the user interface 115 and storage unit 116. The processing unit 113 includes CPU and the like. The user interface 115 includes input devices such as a keyboard and mouse, a display and the like. A program stored in the processing unit 113 is read and executed, for example, from the storage unit 116. Accordingly, the processing unit 113 functions as analysis means for performing various analysis processes of measured values. As a result, processes such as fitting of a measured peak value can be executed. Obtained analysis processing data is stored in the storage unit 116.

FIG. 2 is a diagram showing a modification of an appearance configuration of the nucleic acid detection chip 12 including an integrated measurement circuit and capable of measuring with low noises for use in the present embodiment. FIG. 1 shows a case where the sensors 12a are arranged in the array. In the modification of FIG. 2, the nucleic acid detection chip 12 has a linear chip shape. A linear trench portion is disposed in the surface of a chip main body 121. This trench portion functions as a channel 122 for housing and passing the reagent or the like. This channel 122 functions as a cell which causes electrochemical reactions such as a hybridization between a target nucleic acid in the specimen solution and probe nucleic acid. Both the chip main body 121 and the channel 122 have elongated shapes along a direction in which the reagent or air flows. The chip main body 121 has a length of about 25 to 50 mm in a longitudinal direction, and a width smaller than 5 mm vertically to the longitudinal direction, that is, in a direction vertical to the direction in which the reagent or air flows. A plurality of electrolysis electrodes 123 are linearly arranged in the channel 122. These electrolysis electrodes 123 are arranged every four electrodes, for example, at a substantially equal interval of about 2 mm. Each of these electrolysis electrodes 123 functions as a sensor which detects various electrochemical reactions. The electrode for electrolysis 123 includes a set of a working electrode, counter electrode, and reference electrode as described above. Alternatively, for example, one counter electrode or one reference electrode may be disposed for a plurality of working electrodes, or the working electrode, counter electrode, and reference electrode may be disposed respectively.

If a channel end 122a is assumed to be on an uppermost stream side of the reagent or air in the channel 122, the reagent or air flows toward a channel end 122b from the channel end 122a. Needless to say, the reagent or air may also be reversed to the channel end 122a from the channel end 122b depending on a measurement method, but in either case the reagent or air flows to the other end from one end along the longitudinal direction.

A plurality of bonding pads 124 are disposed on the ends 121a and 121b of the chip main body 121. Each of the bonding pads 124 is electrically connected to the electrolysis electrodes 123 in the chip main body 121. The chip/housing interface 112 is electrically connected to the bonding pads 124 to perform the measurement. Accordingly, the electric signal detected by the electrolysis electrodes 123 can be obtained from the bonding pads 124, and output to the analysis apparatus housing 11.

In general, an operation of passing the solution onto or from the electrode surface functioning as the sensor on the chip, that is, the solution feed operation has to be performed, for example, in the DNA chip for detecting the nucleic acid. If a capacity of the channel for feeding the solution is large, a total amount of specimens increases. If the sensors are arranged on the chip in a two-dimensional array, the channel has to be disposed in a meandered shape, or a broad channel has to be disposed. In a meandered channel, resistance against the reagent solution flow is large, and efficiency of the solution feed is remarkably impaired. To solve the problem, as shown in FIG. 2, the chip main body 121 and channel 122 are linearly disposed. If the sensors are linearly arranged along the channel 122, degradation of the solution feed efficiency or a local fluctuation of the measured value can be avoided.

Furthermore, a reagent dispensing port of a spotting robot for use in dropping the probe nucleic acid onto the chip is also one-dimensionally disposed for each aggregate of four electrolysis electrodes 123 in the channel 122. Accordingly, all the probe nucleic acid can be dropped by one positioning. As a result, the efficiency of a chip preparation process can be enhanced.

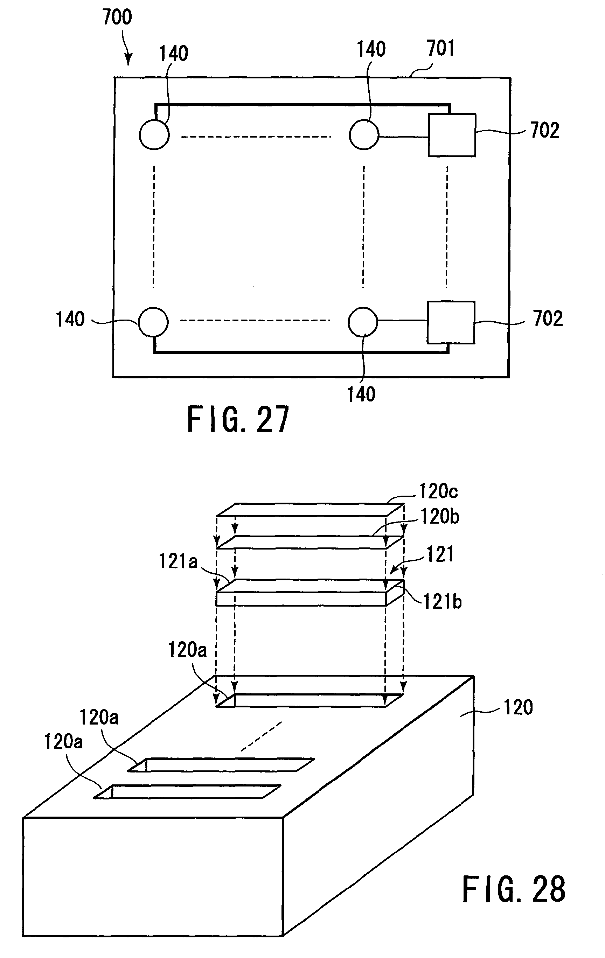

Alternatively, for the linear nucleic acid detection chip 12 shown in FIG. 2, for example, as shown in FIG. 28, a plurality of chips for nucleic acid detection 12 are fitted and fixed at equal intervals and in parallel into trench portions 120a of a cassette for holding the chips 120 in which the chips for nucleic acid detection 12 are held. Moreover, the chip surface is sealed with a glass plate or the like via a rubber ring for sealing the solution.

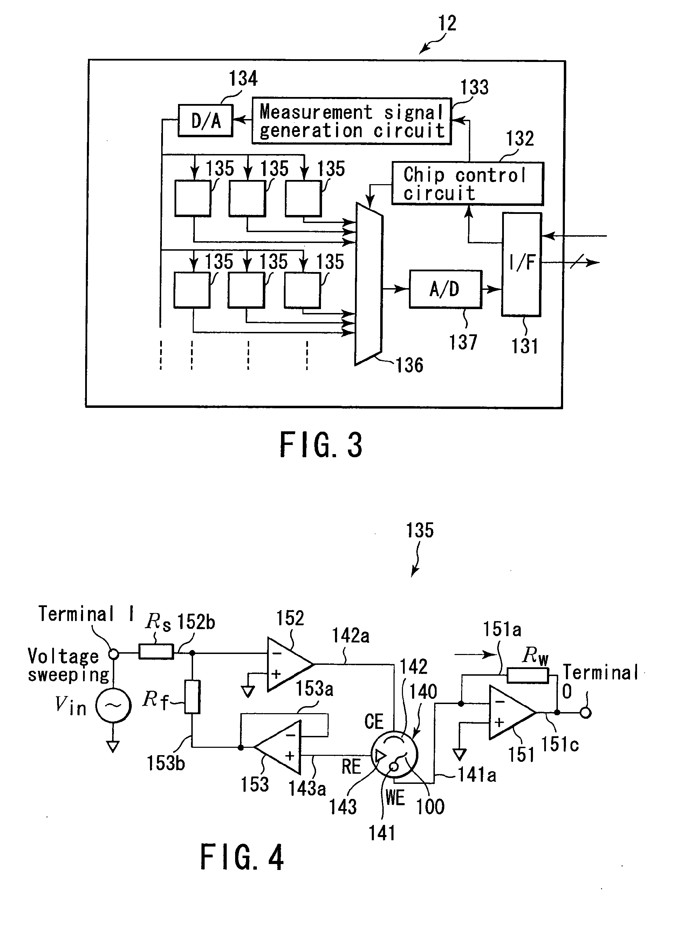

FIG. 3 is a block diagram of a measurement circuit of the nucleic acid detection chip 12. As shown in FIG. 3, in the chip 12, an interface 131, a chip control circuit 132, a measurement signal generation circuit 133, a D/A converter 134, a plurality of modules 135, a selector 136, and an A/D converter 137 are integrated.

The interface 131 executes a reception/transmission of an electric signal from/to the outside of the chip. The chip control circuit 132 controls the measurement signal generation circuit 133 and selector 136 based on a command of measurement start sent from the outside of the chip 12 via the interface 131. The measurement signal generation circuit 133 performs a voltage sweeping based on the command of the chip control circuit 132. Concretely, the measurement signal generation circuit 133 generates a digital voltage sweep signal, and outputs the signal to the D/A converter 134.

The D/A converter 134 D/A converts the digital voltage sweep signal to an analog measurement signal to output the signals to the plurality of modules 135. Various measurement circuits are integrated in the module 135. The module 135 consists of the circuits, for instance, such as: a Potentiostat circuit including a tree-electrode system to control the voltage applied to the reagent, a circuit to copy a current output from a probe, a circuit to convert the current to the voltage, a circuit to subtract a background signal from output signal, and the like.

The configuration of the module 135 is variously modified in accordance with a method, purpose or the like of the measurement. For example, the processing unit 113 may perform a process corresponding to subtraction without including the circuit for background signal subtraction. That is, in this case, a sensor 12a including a conventional sensor and a sensor for background level detection, a normalization circuit which normalizes an output current of the sensor 12a, and a current-to-voltage converter which converts a output current of the normalization circuit to a voltage signal, are implemented in the module 135. Moreover, an output voltage of the current-to-voltage converter is output as measurement data to the outside of a nucleic acid detection chip 12 via a selector 136, A/D converter 137, and interface 131. The measurement data is further output to a processing unit 113 via a chip/housing interface 112. The processing unit 113 subtracts background measurement data from the sensor for background level detection from conventional measurement data from the conventional sensor in measurement data from this chip/housing interface 112.

When the modules 135 are used to detect DNA, the following procedure is performed. First, the sensor 12a disposed in the module 135 is immersed in the specimen to cause the hybridization reaction. After performing this reaction for a predetermined time, the sensor 12a is immersed in the buffer agent to which the intercalator agent has been added to perform electrolysis. To perform the electrolysis, an analog voltage is input into a predetermined electrode (counter electrode) immersed in the cell from the D/A converter 134. Separately from the electrodes (counter electrode, reference electrode) to apply a voltage to the solution, the detection circuit is connected to an electrode for the sensor (working electrode) on which the probe nucleic acid is immobilized. While a predetermined sweeping voltage is input, the detection circuit detects the current caused by the electrolysis of the intercalating agent. The detection circuit performs the current-to-voltage conversion, and a detection result is output as a detection signal to the selector 136 at any time . The selector 136 scans the array of a plurality of modules 135 based on the control of the chip control circuit 132. The time-division multiplexed detection signal obtained by this scanning is output to the A/D converter 137. The A/D converter 137 converts the analog signal to the digital signal which is output to the outside of the chip via the interface 131.

In this manner, when the sensor surface is immersed in the specimen solution that is a measurement object, an operation of performing electrolysis measurement to send the signal to the outside of the chip is performed in hardware in the nucleic acid detection chip 12. Moreover, an operation including extraction of a peak from data taken out of the chip, comparison with a threshold value, acquisition of a bit pattern, and output of nucleic acid concentration included in the specimen by collation with a numerical table is performed in a software manner in the processing unit 113 in the analysis apparatus housing 11 of FIG. 1.

FIG. 4 is a diagram showing one example of a detailed configuration of the module 135. The module 135 is three-electrode type Potentiostat in which resistances $R_s$ and $R_f$ connected to an inverting input terminal of an operational amplifier 152 are used to feed the voltage of a reference electrode 143 negatively back to a swept voltage input into a terminal I, and a desired voltage is applied to a solution regardless of fluctuations of various conditions of the electrode and solution in the cell.

This Potentiostat changes the voltage of an counter electrode 142 so as to set the voltage of the reference electrode 143 with respect to a working electrode 141 to an predetermined voltage, and accordingly an oxidation current of the intercalating agent is measured electrochemically. A set of the electrodes including the working electrode 141, counter electrode 142, and reference electrode 143 will hereinafter be referred to as a three-electrode system 140.

The working electrode 141 is the electrode for the sensor on which a probe nucleic acid 100 including a target-complementary nucleic acid complementary to a target nucleic acid can be immobilized and which detects a reaction current in the cell. The counter electrode 142 is an electrode which applies the voltage between the working electrode 141 and the counter electrode to supply the current to the sensor. The reference electrode 143 is an electrode which negatively feeds an electrode potential back to the input of the swept voltage so as to control the voltage between the reference electrode 143 and working electrode 141 to a predetermined voltage. This reference electrode 143 is capable of detecting the oxidation current with a high precision without being influenced by various detection conditions in the cell.

The voltage sweep signal from the D/A converter 134 is input into the inverting input terminal of the operational amplifier 152 for reference voltage control of the reference electrode 143 via a wiring 152b.

The wiring 152b is connected to the resistance $R_s$. A noninverting input terminal of the operational amplifier 152 is grounded, and an output terminal is connected to a wiring 142a.

The wiring 142a is connected to the counter electrode 142 on the nucleic acid detection chip 12. When a plurality of counter electrodes 142 are arranged, the wiring 142a is connected in parallel with respect to each counter electrode 142. Accordingly, the voltages can simultaneously be applied to the plurality of counter electrodes 142 by one voltage pattern. When the voltage between the electrodes is exactly controlled, one set of feedback circuits comprised of the operational amplifiers 152 and 153 is disposed with respect to one working electrode 141. In this case, a plurality of resistances $R_s$ are connected in parallel with the outputs of the D/A converter 134.

The reference electrode 143 is connected to the noninverting input terminal of the operational amplifier 153 via a wiring 143a. The inverting input terminal of the operational amplifier 153 is short-circuited by wirings 153b and 153a connected to the output terminal of the amplifier. The wiring 153b includes the resistance $R_f$. The wiring 153b is connected between the resistance $R_s$ of the wiring 152b and the inverting input terminal of the operational amplifier 152. Accordingly, a voltage obtained by feeding the voltage of the reference electrode 143 back to a voltage sweep signal $V_{in}$ is input into the operational amplifier 152. The voltage between the reference electrode 143 and the working electrode 141 is controlled by the output voltage obtained by inverting and amplifying the input voltage.

The working electrode 141 is connected to the inverting input terminal of an operational amplifier 151 via a wiring 141a. The noninverting input terminal of the operational amplifier 151 is grounded. A wiring 151c connected to the output terminal of the operational amplifier 151 is connected to the wiring 141a via a wiring 151a. A resistance $R_w$ is disposed in the wiring 151c. Assuming that a voltage of a terminal O on the output of the operational amplifier 151 is $V_w$, and a current is $I_w$, $V_w = I_w \cdot R_w$ is satisfied. An electrochemical signal obtained from the terminal O is output to the selector 136.

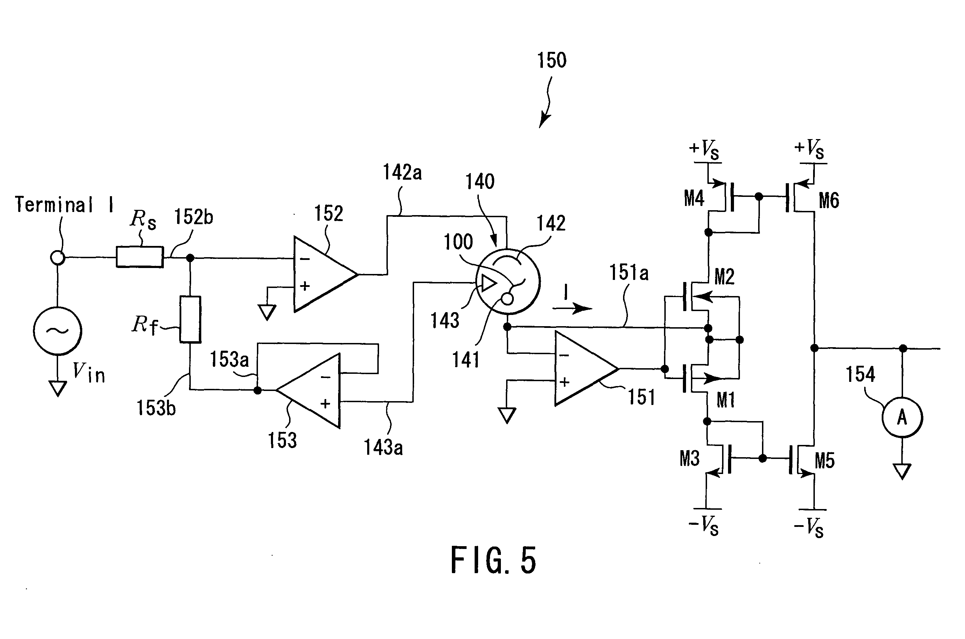

FIG. 5 is a diagram showing a detailed configuration of an improved module 150 obtained by improving the module 135 shown in FIG. 4. A configuration common to that of FIG. 4 is denoted with the same reference numerals, and detailed description thereof is omitted. The voltage applying circuit configuration including the counter electrode 142 and reference electrode 143 is common to that of FIG. 4. In order to expand the device integration, a resistor is not used in the circuit connected to a working electrode 141, and a current detection circuit is used including a circuit of the operational amplifier 151 and six transistors M1 to M6 instead of the resistance $R_w$ disposed in the circuit with the working electrode 141. M1 denotes a PMOS transistor, and M2 is an NMOS transistor.

The module 135 shown in FIG. 4 includes an element disadvantage for efficiency integrating the circuit. The disadvantageous element is the resistance $R_w$. A transimpedance amplification circuit constituted of the resistance $R_w$ and operational amplifier 151 has a general configuration. That is, this transimpedance amplification circuit is capable of realizing an operation to keep the potential of the working electrode 141 to be constant regardless of surrounding conditions of the solution, circuit and the like, and freely taking the current from the working electrode 141 without changing the potential of the working electrode 141, and this circuit is generally used in electrolysis measurement. The current taken out of the working electrode 141 is faint. When the current is measured with high precision, a resistance with a low noise generated by a device itself has to be selected, and a resistance value of the resistance has to be large. To effectuate the resistance satisfying the requirement on an integrated circuit, a device area increases, and it is therefore difficult to use characteristics of the integrated circuit. Therefore, the resistor is mounted as a single device outside the chip in many cases. In these cases, a whole apparatus is enlarged, and further disadvantage occurs that a simultaneousness of the measurement is impaired.

To solve the problem, in the present embodiment, as shown in FIG. 5, there is provided a current detection circuit in which any resistor is not used. In FIG. 5, the output terminals of the operational amplifier 151 are connected to gates of the transistors M1 and M2. The wiring 151a connected to the working electrode 141 is connected to sources of the transistors M1 and M2. Both bodies of the transistors M1 and M2 are short-circuited by the wiring 151a. A drain of the transistor M1 is connected to that of the transistor M3. The source of the transistor M3 is connected to a negative voltage source of - Vs, and the gate is connected to the gate of the transistor M5 and the drain of the transistor M3. Accordingly, the transistors M3 and M5 form a current mirror topology.

The source of the transistor M5 is connected to the negative voltage source of -Vs, and the drain is connected to that of the transistor M6. The gate of the transistor M6 is connected with respect to the gate and drain of the transistor M4. The sources of the transistors M4 and M6 are connected to positive voltage source of +Vs. Accordingly, the transistors M4 and M6 form the current mirror topology.

When the current $I$ flows in a direction of an arrow in FIG. 5, that is, toward the current detection circuit from the working electrode 141, the current flowing in the transistor M5 is taken out at the output node of the current mirror. Conversely, when the current flows in a direction opposite to the arrow of the figure, that is, toward the working electrode 141 from the current detection circuit, the current flowing in the transistor M6 is taken out. The current is measured by an ammeter 154.

In this configuration, assuming that transconductances of the transistors M1 to M6 are $\beta_1$, $\beta_2$, $\beta_3$, $\beta_4$, $\beta_5$, $\beta_6$, it is necessary to satisfy $\beta_1=\beta_2$, $\beta_3=\beta_4$, $\beta_5=\beta_6$ in order to establish a satisfactory linearity. It is to be noted that when $\beta_3=\beta_5$, $\beta_4=\beta_6$ are satisfied, a ratio of an original current to be measured with respect to an output current is 1:1. To amplify the measurement current, $\beta_3:\beta_5=1:B$, $\beta_4:\beta_6=1:B$. Accordingly, an amplification factor of 1:B is represented. That is, defining that a gate length and gate width of MOSFET are L, w, the gate lengths of the transistors M1 to M6 (MOSFET) are $L_1$ to $L_6$, and the gate widths are $w_1$ to $w_6$, $(W_3/L_3):(W_5/L_5) = 1:B$, $(w_4/L_4):(w_6/L_6) = 1:B$ may be designed. It is to be noted that in the specification, the amplification includes not only amplification with a gain exceeding one but also amplification with a gain of one .

The circuit operation shown in FIG. 5 will be described in accordance with the example of observation of the oxidation current.

When a reduction occurs in the working electrode 141, the reduction current flows into the current detection circuit from the working electrode 141. The potential on the wiring 151a rises by a voltage drop generated at this time. Moreover, conversely the potential of the output terminal of the operational amplifier 151 is largely lowered by the operational amplifier, and the transistor M1 is brought into an on-state. Accordingly, the current flows into the transistor M3, and the potential of the wiring 151a is negatively fed back and fixed at a ground potential. On the other hand, the current flowing in the transistor M3 is copied by the transistor M5. The current flowing in the transistor M5 can be measured by the ammeter 154.

When an oxidation current is observed, characteristics reverse to those in the reduction current in positive/negative characteristics are generated in the current detection circuit connected to the working electrode 141. That is, the potential on the wiring 151a is lowered by a voltage drop generated by the current with respect to the potential of a noninverting terminal of the operational amplifier 151, and the transistor M2 is brought into the on-state. Accordingly, the current flows in the transistor M4. The current flowing in the transistor M4 is copied by the transistor M6. The current flowing in the transistor M6 can freely be taken out by the ammeter 154.

In this manner, in case of oxidation current observation, the same operation as that of the transistors M1, M3, and M5 in reduction current observation is performed in the transistors M2, M4, and M6 with reverse characteristics. Accordingly, both the oxidation current and the reduction current can be measured.

It is to be noted that to short-circuit the bodies of the transistors M1 and M2, it is necessary to completely separate the device PMOS in the process of an N-type substrate, and NMOS in the process of a P-type substrate. This can be realized depending on the process, but the device does not necessarily have to be separated. For example, in an N-type substrate P well process, it is difficult to completely separate the bulk of PMOS. In this case, the bulk of the transistor M1 is directly connected to the positive voltage source. Even this circuit effectuates the equal circuit function. Furthermore, even when the bulk of the transistor M2 is directly connected to the negative voltage source, the equal circuit function is realized. Additionally, in this case, it is preferable to exactly realize $\beta_3=\beta_4$, $\beta_5=\beta_6$ as correctly as possible.

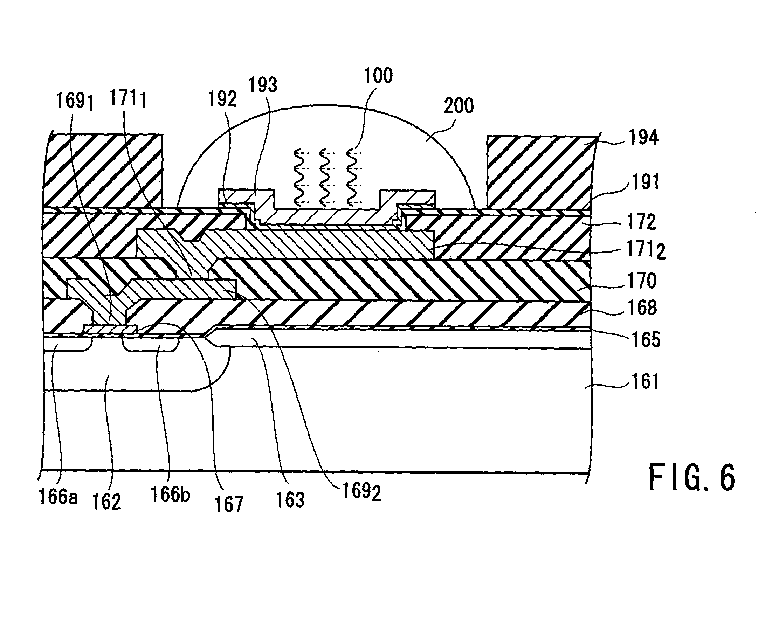

FIG. 6 is a diagram showing one example of a device sectional view of the nucleic acid detection chip 12 including the working electrode 141. As shown in FIG. 6, a circuit formed in LSI is prepared on an Si substrate 161 which is a substrate in a standard CMOS process.

A circuit including an insulating film, semiconductor film, metal film and the like is formed on the Si substrate 161. A well 162 is formed in the Si substrate 161. A field oxide film 163 is formed on the surface of the Si substrate 161 to separate the individual devices. Diffusion layers 166a and 166b shallower than the well 162 are formed in the well 162. A gate oxide film 165 is formed over the whole surface of the Si substrate 161 including the upper surface of the field oxide film 163. A gate electrode 167 is formed on the gate oxide film 165 between the diffusion layers 166a and 166b.

Furthermore, an interlayer insulating film 168 is formed so as to cover the upper surface of the gate oxide film 165 and the upper and side surfaces of the gate electrode 167. In the interlayer insulating film 168, first contact plugs $169_1$ and first-layer interconnections $169_2$ composed of metals such as Al or Cu are formed to be extended onto the interlayer insulating film 168 so the plugs are electrically connected to the gate electrode 167. An interlayer insulating film 170 such as TEOS or the like is formed on the interlayer insulating film 168 including the upper and side surfaces of the first contact plug $169_1$ and first layer interconnection $169_2$.

In the interlayer insulating film 170, a second contact plug $171_1$ and a second layer interconnection $171_2$ formed of the metals such as Al and Cu are formed to extend onto the interlayer insulating film 170 so the plugs are electrically connected to the first layer interconnection $169_2$. An interlayer insulating film 172 is formed on the interlayer insulating film 170 including the upper and side surfaces of the second contact plug $171_1$ and second layer interconnection $171_2$.

A trench portion (hereinafter referred to as a small trench portion) is formed in the interlayer insulating film 172 so as to be electrically connected to the second layer interconnection $171_2$. In FIG. 6, only one small trench portion is disposed, but in actual a plurality of small trench portions are disposed in accordance with the number of electrodes or that of electrode groups. A passivation film 191 is formed on the surface of the interlayer insulating film 172 and the side surface of the small trench portion other than the small trench portion bottom surface so as to cover the interlayer insulating film 172. An insulating film 194 including an oxide film, photoresist film, and the like for separation from another small trench portion is formed at a predetermined distance from the small trench portion on the passivation film 191 other than the small trench portion. A Ti electrode 192 and Au electrode 193 are sequentially stacked and buried/formed so as to extend to the side surface of the small trench portion and the passivation film 191 surface other than the small trench portion in the trench portion (hereinafter referred to as the large trench portion) partitioned by the insulating film 194. The probe nucleic acid 100 is immobilized on the Au electrode 193.

Next, a method of manufacturing the above-described nucleic acid detection chip 12 will be described.

First, the field oxide film 163 having a film thickness, for example, of 800 nm is formed on a part of the Si substrate 161 using a LOCOS process. Subsequently, the field oxide film 163 is used as a mask to form the well 162 on the surface of the Si substrate 161 through a process of impurity ion injection and diffusion or the like. Subsequently, the surfaces of the Si substrate 161 and field oxide film 163 are oxidized to form the gate oxide film 165 having a film thickness, for example, of 50 nm. Thereafter, a polysilicon film having a film thickness, for example, of 500 nm is formed on the gate oxide film 165. Next, the polysilicon film on a device forming region is selectively removed to selectively leave the polysilicon film on the device forming region. The selectively remaining polysilicon film functions as the gate electrode 167. Next, the gate electrode 167 selectively remaining on the device forming region is used as the mask to form the diffusion layers 166a and 166b in the well 162 through the process of impurity ion injection and diffusion. The diffusion layers 166a and 166b and the gate electrode 167 form a transistor in which the diffusion layers 166a and 166b are the source and drain.

Next, the interlayer insulating film 168 such as BPSG having a film thickness, for example, of 1550 nm is formed on the whole surface of the apparatus. Moreover, a contact is formed in the interlayer insulating film 168 so as to extend through the diffusion layer 166a.

Next, a metal film having a film thickness, for example, of 800 nm and formed of Al-Si-Cu is formed on the interlayer insulating film 168 in such a manner that the contact is charged. The metal film is selectively removed to form the first contact plug $169_1$ and first layer interconnection $169_2$ electrically connected to the diffusion layer 166a.

Next, the interlayer insulating film 170 such as TEOS having a film thickness, for example, of 1050 nm is formed on the interlayer insulating film 168 including the upper and side surfaces of the first contact plug $169_1$ and the first layer interconnection $169_2$. Moreover, a contact is formed in the interlayer insulating film 170 so as to extend through the first layer interconnection $169_2$. Furthermore, a metal film formed of Al-Si-Cu having a film thickness, for example, of 1000 nm is formed on the interlayer insulating film 170 in such a manner that the contact is charged. The metal film is selectively removed to form the second contact plug $171_1$ and second layer interconnection $171_2$ electrically connected to the first layer interconnection $169_2$.

Next, the interlayer insulating film 172, for example, formed of On-Al-PSG having a film thickness of 1050 nm is formed on the interlayer insulating film 170 including the upper and side surfaces of the second contact plug $171_1$ and second layer interconnection $171_2$. Moreover, a contact is formed in the interlayer insulating film 172 so as to extend through the second layer interconnection $171_2$. Furthermore, the passivation film 191 formed of OPSiN having a film thickness, for example, of 100 nm is formed so as to cover the bottom and side surfaces of the contact and to extend to the surface of the interlayer insulating film 172. Subsequently, the passivation film 191 formed on the small trench portion bottom surface is selectively removed. Accordingly, the second layer interconnection $171_2$ surface is exposed.

Subsequently, the second layer interconnection $171_2$ is coated with, for example, a Ti film having a film thickness of 100 nm and an Au film having a film thickness of 200 nm sequentially stacked/formed on the bottom and side surfaces of the small trench portion and the passivation film 191 surface outside the small trench portion. Moreover, the portion formed on the passivation film 191 surface outside the small trench portion is patterned. As a result, the Ti electrode 192 and Au electrode 193 are formed extending to the bottom and side surfaces of the small trench portion and a part of the passivation film 191 surface.

Furthermore, the insulating film 194 is formed on the passivation film 191 including the upper and side surfaces of the Ti electrode 192 and Au electrode 193. Moreover, the insulating film 194 is patterned and selectively removed so as to expose the Ti electrode 192 and Au electrode 193. Accordingly, the large trench portion is formed.

It is to be noted that FIG. 6 shows that the film is patterned so as to prevent the insulating film 194 from overlapping with the Au/Ti film outside the small trench portion, but the present invention is not limited to the example. The insulating film 194 may also be patterned over the Au/Ti film, and a remaining portion may determine an area of the electrode for the sensor.

Moreover, after forming and patterning the insulating film 194, the Ti electrode 192 and Au electrode 193 may also be formed in the large trench portion.

The large trench portion partitioned by the insulating film 194 functions as the cell. That is, a specimen solution 200 is dropped in the large trench portion, further the buffer agent, air, intercalating agent and the like are introduced, and accordingly the electrochemical reaction is caused on the Au electrode 193.

Moreover, a packing, O ring and the like may be used in addition to the insulating film 194 to secure a region in which the specimen solution 200, buffer agent, air, and intercalating agent are introduced.

In this manner, FIG. 6 shows the sectional structure of the nucleic acid detection chip 12 including the working electrode 141, but the similar section structure is also formed with respect to the counter electrode 142 and reference electrode 143. In this case, the counter electrode 142 is disposed apart from the reference electrode 143 in the same large trench portion as that of the working electrode 141. With the counter electrode 142 and reference electrode 143, it is not necessary to immobilize the probe nucleic acid 100 on the Au electrode 193. Needless to say, even with the use as the working electrode 141, the probe nucleic acid 100 does not have to be immobilized depending on a use purpose. Moreover, the area of each electrode may variously be changed in accordance with a measurement purpose.

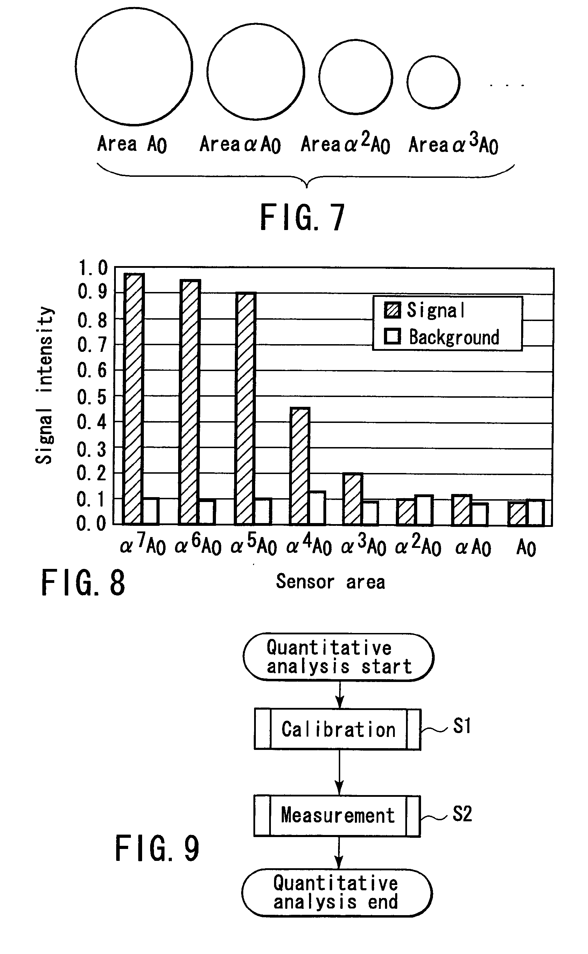

FIG. 7 is a schematic diagram of an electrode area of the working electrode 141 for performing the nucleic acid quantitative analysis of the present embodiment. As shown in FIG. 7, the areas of the working electrodes 141 for measuring the current from the same nucleic acid or the background current make a geometric progression as $A_0$, $\alpha A_0$, $\alpha^2 A_0$, $\alpha^3 A_0$ ... ($\alpha < 1$), assuming that a largest area is A0.

In the current detection type of nucleic acid detection chip, the electrode area is reduced, and time required for hybridization is lengthened to increase an absolute amount of a specific signal. This is because an amount of a labeled material electrochemically active for use as the intercalating agent that is non-specifically bonded to the surface of the electrode, especially a region on which the nucleic acid is immobilized is reduced. Accordingly, a ratio of the signal obtained from the intercalating agent specifically bonded to a double-stranded nucleic acid can be enhanced. That is, the signal level can be raised with respect to the background level. In this case, a minimum concentration $C_{min}$ (copy/ml) of a detection sensitivity has a following relation with respect to an electrode area A ($cm^2$).

$$\ln C_{min} = 0.72 \ln A + 8.$$

Based on this equation, a set of working electrodes 141, whose areas make a geometrical progression and on each surface of which an identical type of nucleic acid is immobilized, is used for measurement in a case where a surface density of probe molecules is constant. Accordingly, a nucleic acid analysis apparatus capable of realizing a broad dynamic range of the detection sensitivity is realized. It is to be noted that electrode areas A may have a relation substantially forming a geometric progression. That is, each of the electrode areas A may indicate a value in a range of ±10% from the geometric progression. In the quantitative analysis of the nucleic acid, one set of series of the electrodes is prepared as shown in FIG. 7, a single probe prepared in the equal concentration is immobilized, and a specimen having a certain concentration may be hybridized for an appropriate time. The surface density of the molecule of the probe immobilized with respect to the same electrode series is set to be common.

FIG. 8 shows a detection result using the probe series shown in FIG. 7. FIG. 8 shows a signal intensity in a case where the probe nucleic acid to be hybridized with the specimen solution is immobilized and hybridized for a predetermined time, and a signal intensity measured on similar conditions in a case where the probe nucleic acid is not immobilized. The signal intensity of the ordinate is normalized with the signal intensity obtained at the time when the hybridization takes place with respect to all the probes immobilized on the sensor (electrode) surface. The signal intensity normalized in this manner will be hereinafter referred to as the normalized signal intensity.

When the specimen having a certain nucleic acid concentration is hybridized for a certain time, the hybridization takes place in most of the probe nucleic acids on a small sensor surface for a reaction time, and therefore the normalized signal intensity is close to 1 without any limit. Conversely, the absolute number of probe nucleic acids causing the hybridization on a large sensor surface is small, and only a signal intensity comparable to a background level is obtained. On the electrode of which the area is adequately intermediate , the normalized signal intensity having a magnitude between the background level and signal intensity 1 is obtained. For example, the adequate electrode is an electrode having an electrode area of $\alpha^4 A_0$ in FIG. 8. Here, a plurality of nucleic acids having known concentrations are measured beforehand to calculate a relation between the electrode area and the nucleic acid concentration in which the signal intensity is obtained between the background level and a saturated level, that is, normalized signal intensity 1. If this relation is known beforehand, the nucleic acid concentration can be identified. To identify the nucleic acid concentration, even for the nucleic acid having an unknown concentration, the area of the sensor surface in which the normalized signal intensity appears between the saturated level and the background level may be known. Furthermore, it is possible to know the concentration more correctly depending on the magnitude of the signal intensity.

The absolute value of the signal which is obtained from the sensor surface and which is not normalized yet with the electrode area is supposed to be proportional to the electrode area. Therefore, when the electrode area of the sensor surface is reduced with a certain factor $\alpha$ ($\alpha < 1$), the absolute value of the signal accordingly drops. To perform this with the current detection type of the nucleic acid detection chip, Potentiostat having a higher sensitivity needs to be used. Therefore, the measurement circuit is preferably integrated and disposed in a portion closer to the sensor on the same substrate as that of the sensor.

Next, an operation of the above-described nucleic acid concentration quantitative analysis apparatus 1 will be described with reference to flowcharts of FIGS. 9 to 12.

As shown in FIG. 9, the quantitative analysis is carried out by performing calibration (s1) followed by measurement (s2). The calibration is a process for obtaining a bit pattern before analysis of the nucleic acid concentration contained in the specimen solution which is a measurement object. The bit pattern is data indicating judgment conditions of the concentration of the measurement object.

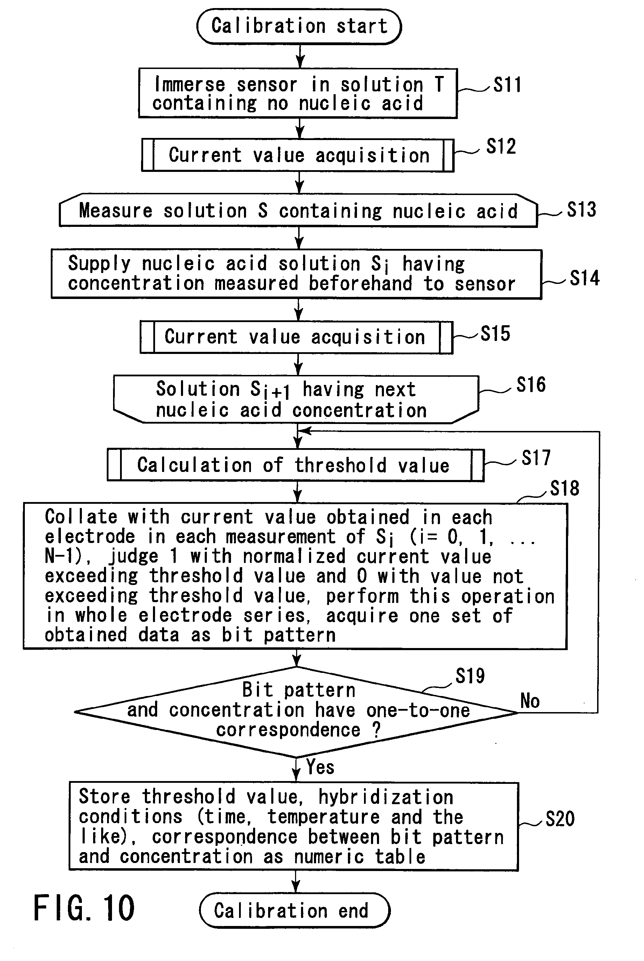

FIG. 10 is a flowchart of one example of a concrete process of the calibration (s1). As shown in FIG. 10, first the sensor 12a on which the probe nucleic acid is not immobilized or the sensor 12a on which the probe nucleic acid is immobilized but the probe nucleic acid not hybridized with a solution T is immobilized, are immersed in the solution T which does not contain the nucleic acid (s11). Subsequently, a current value acquisition process is executed (s12).

The background level (current value) is determined based on the obtained current value.

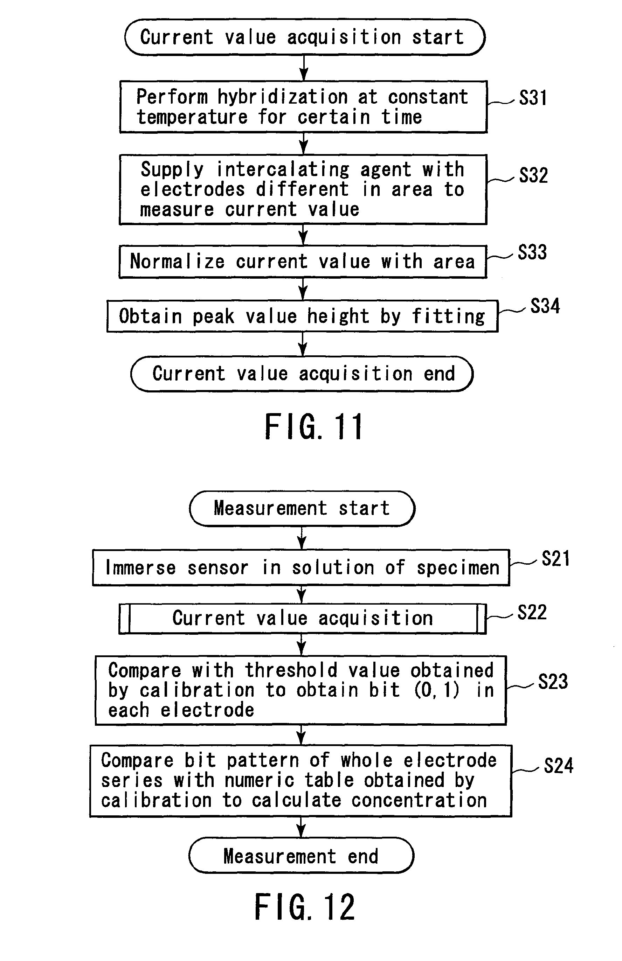

A concrete process flow of the current value acquisition process shown in (s12) is shown in the flowchart of FIG. 11 described later.

Next, a solution S containing the nucleic acid is measured (s13). Concretely, nucleic acid solutions $S_0$, $S_1$, ..., $S_{N-1}$ having N types of concentrations $C_0$, $C_1$, ..., $C_{N-1}$ (i = 0, 1, ..., *N*-1) which cover the dynamic range are prepared with respect to M types of electrode areas $A_j$ (*j* = 0, 1, ..., *M*-1). Moreover, a nucleic acid solution $S_i$ having concentration $C_i$ is dropped into the sensors 12a on which the same nucleic acid probe is immobilized and which have M types of different electrode areas $A_j$ (s14). Accordingly, the probe nucleic acid immobilized on the sensor 12a and the nucleic acid in the nucleic acid solution $S_i$ are hybridized. Moreover, the current value acquisition process is executed in the same manner as in (s12) (s15). The measurement conditions of the current value acquisition process of (s15) are determined in the same manner as in (s12). When the measurement ends with respect to the nucleic acid solution $S_i$ having the concentration $C_i$, i = i+1 is set, and the measurement is performed with respect to the nucleic acid solution $S_{i+1}$ having another concentration (s16). When M×N current values $I_p(i, j)$ are obtained with all the concentrations and all the electrode areas $A_j$ with respect to all the nucleic acid solutions $S_i$, a threshold value $I_{th}$ is set (s17).

The threshold value is set to a current value between a saturated current value $I_{st}$ and background current value $I_{bg}$. That is, $I_{st} > I_{th} > I_{bg}$.

To determine the threshold value, the saturated current value needs to be known. An ideal saturated current value is obtained in a case all the probe nucleic acids on the substrate form a double-stranded structure. To obtain the saturated current value, an experiment may be carried out so as to acquire the signal from the electrode on which the nucleic acid forming a double-stranded structure is immobilized. In this case, the nucleic acid is preferably immobilized at a density equal to the surface density on the electrode on which the probe nucleic acid not forming a double-stranded structure is immobilized.

When it is difficult to prepare the hybridized electrode beforehand, a sample having a sufficiently high labeled nucleic acid concentration is hybridized for a sufficient time. Accordingly, it is possible to determine an actual saturated level.

Alternatively, a plurality of nucleic acid solutions $S_i$ are measured with sensors having different areas $A_j$, and obtained data is analyzed. Accordingly, it is possible to define the actual saturated level. In this case, the saturated current value is defined as follows using a property that the value is proportional to the sensor area. First, the current value obtained from each electrode is normalized by the area. When the normalized current values are compared among the different areas, the normalized current value obtained from the electrode having an opening area smaller than a certain area is sufficiently larger than that of the background, and indicates a substantially constant value regardless of the area. Then, it is possible to define the normalized current value as the saturated level in all the electrodes, that is, the normalized signal intensity 1.

Moreover, this can be also applied to the measurement of the background level. That is, when data obtained with respect to a plurality of solutions having different nucleic acid concentrations, and data obtained from the electrodes having different areas are analyzed, the background level can be estimated. In this estimation, first the obtained current value is normalized with respect to the area. Moreover, when the obtained normalized current values are compared among the different areas, a plurality of normalized current values obtained from the electrode having an opening area larger than the certain area are sufficiently smaller than the saturated level, and indicate a substantially constant value regardless of the area. Then, it is possible to define the normalized current value as the background level in all the electrodes.

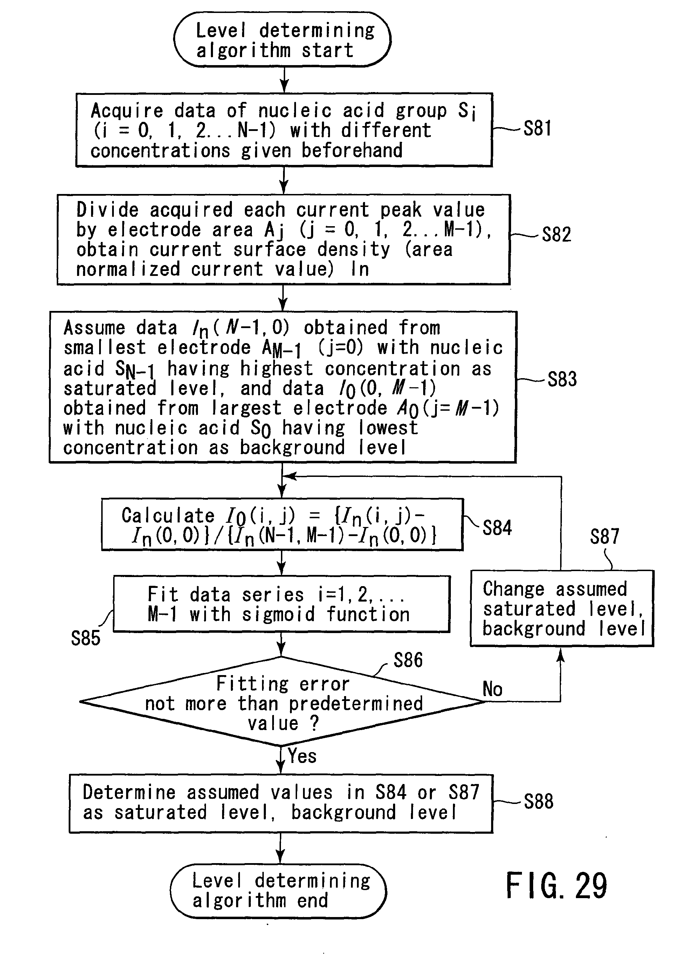

One example of a determining algorithm of the saturated level, background level for concrete threshold value calculation will be described with reference to a flowchart of FIG. 29.

With respect to different N types of nucleic acids $S_i$ (i = 0, 1, ..., N-1) having concentrations given beforehand, or all the sensors 12a on which a single-stranded nucleic acid molecule is immobilized and which have different areas $A_j$ (j = 0, 1, ..., M-1), the current peak value $I_p(i, j)$ is acquired in (s81) by the operation shown in (s13) to (s16). The peak value may be acquired by a subsequent-stage circuit or software. When the peak value is acquired in the subsequent stage, the current values I in a plurality of times may be acquired in this (s81).

The current peak values obtained in (s81) are subjected to a first normalization (s82) by the electrode areas $A_j$ (j = 0, 1, ..., M-1) to obtain a first normalized current value $I_n(i,j)$. Accordingly, the current value per unit area is obtained. Next, a current $I_n(N-1, M-1)$ obtained from a combination of a nucleic acid solution $S_{N-1}$ having a highest concentration and the sensor 12a having a smallest opening area $A_{M-1}$ is assumed as a saturated current, and a current $I_n(0, 0)$ obtained from a combination of a nucleic acid solution $S_0$ having a lowest concentration and the sensor having a largest open area $A_0$ is assumed as a background current (s83).

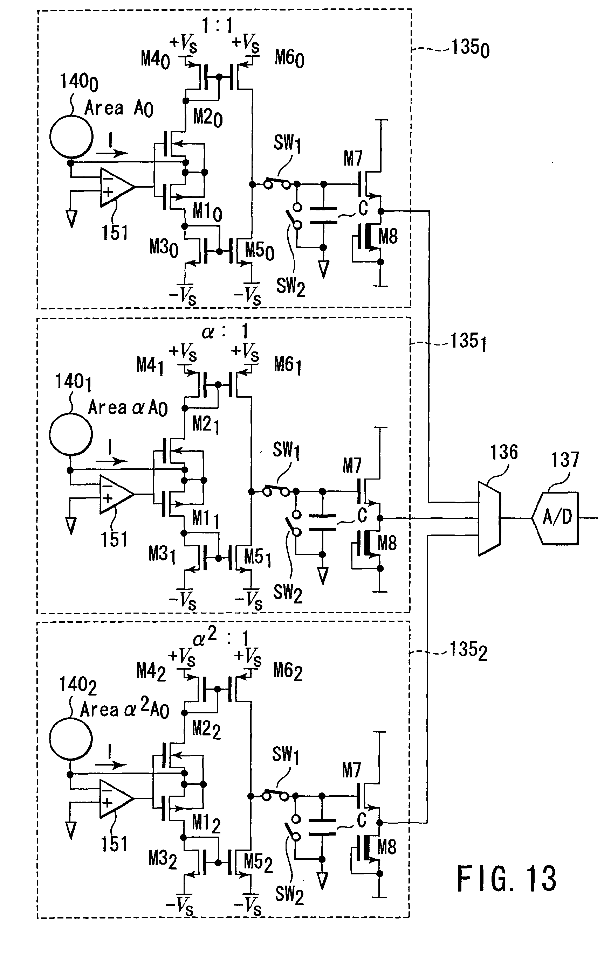

The first normalization of (s82) is realized by adjustment of a current amplification factor of a current mirror circuit in FIG. 13 described later.

Next, with each measured current value $I_n(i, j)$, a second normalization process is performed by subtracting the background current value from the saturated current value for evaluation with a sigmoid function. Concretely, a second normalized current value $I_0(i,j)$ is obtained by the following equation (s84).

$$I_0(i,j) = \{I_n(i,j)-I_n(0,0)\}/\{I_n(N\text{-}1,M\text{-}1)-I_n(0,0)\}$$

Next, a data series with respect to one set of electrodes obtained from the measurement of the respective concentrations is fitted with the sigmoid function (s85). Next, it is determined whether a fitting result is not more than a predetermined value (s86). When the fitting result is within a predetermined error, the assumed values are determined as the saturated level and background level (s88). In a case where the results exceed the predetermined error, the current values with respect to the different nucleic acids in which either i or j is changed are assumed as a saturated level $I_{st}$ and background level $I_{bg}$ (s87).

In this manner, with respect to the saturated level and background level obtained in (s88), a threshold value $I_{th}$ may be set so as to satisfy $I_{st} > I_{th} > I_{bg}$. It is to be noted that the threshold value setting process mentioned herein is merely one example. For example, for the first normalization process with respect to the current value per area in (s84), with the evaluation that is performed using the fitting into the functions other than the sigmoid function, the normalization process may also be performed based on the saturated current value $I_{st}$. In this case, the normalized current value $I_0(i,j)$ is as follows.

$$I_0(i,j) = I_n(i,j)/I_n(N\text{-}1,M\text{-}1)$$

When the threshold value $I_{th}$ is set, and a relation between the threshold value $I_{th}$ and the hybridization time is set in this manner, a one-to-one correspondence can be found between the number of electrodes exceeding the threshold value $I_{th}$ and the sample for calibration having the concentration measured beforehand. For example, a case where the threshold value $I_{th}$ is exceeded is represented by bit "1", and a case where the threshold value $I_{th}$ is not exceeded is represented by "0". Then, in order from a low concentration of the solution, bit data B is represented as $\{B(A_0), B(A_0\alpha^1), B(A_0\alpha^2), B(A_0\alpha^3), ..., B(A_0\alpha^{N\text{-}1}),\} = \{B(A_0), B(A_1), ..., B(A_{N\text{-}3}), B(A_{N\text{-}2}), B(A_{N\text{-}1})\} = \{0, 0, ..., 0, 0, 0\}, \{0, 0, ..., 0, 0, 1\}, \{0, 0, ..., 0, 1, 1\}, ..., \{1, 1, 1, 1, ..., 1\}$. This set of bit data B (hereinafter referred to as the bit pattern) is acquired with respect to each nucleic acid concentration $C_i$.

Next, it is determined whether or not the obtained bit pattern has a one-to-one correspondence to the concentration (s19).

The threshold value is preferably set in such a manner that the bit pattern of the certain concentration is not identical with that of another concentration. For this, concretely, after obtaining the bit pattern with respect to each concentration $C_i$, the bit patterns concerning the concentrations closest to each other are compared to be determined whether or not the patterns are identical with each other. When both the patterns are identical with each other, the threshold value $I_{th}$ is changed to obtain the bit pattern again. The comparison and bit pattern calculation may be repeated until both the patterns are not identical. This process can be executed by the processing unit 113. The comparison of the bit patterns having adjacent concentrations, the re-setting of the threshold value $I_{th}$ in a case where the comparison result is disagreement and the process of repeating the comparison and re-setting until the bit patterns do not agree are stored as the program in the storage unit 116. The processing unit 113 may read and execute the program. When the threshold value $I_{th}$ is changed in this manner, the bit patterns different with the concentrations are obtained, and the analysis precision of the nucleic acid concentration is enhanced.

It is to be noted that the threshold value $I_{th}$ may be any value between the normalized saturated current value $I_{st}$ and the normalized background current value $I_{bg}$. Since the threshold value $I_{th}$ is used in comparing the magnitude with that of the current value normalized by the electrode area by the first normalization, one threshold value $I_{th}$ may be set with respect to the electrodes having a plurality of electrode areas.

When a magnification $\alpha$ of the electrode area is reduced, the analysis precision is enhanced, but the bit patterns having the close concentrations simply agree with each other in some case. In this case, the process does not have to return to the case where the bit patterns having closest concentrations agree with each other (s18), and may advance to (s20).

By this judgment process of (s19), the bit patterns different with the respective concentrations are obtained. The obtained bit pattern is stored, for example, as the following judgment table in the storage unit 116.

Table 1

| Hybridization time $t_0$ | | |
|---|---|---|
| Nucleic acid | Concentration | Bit pattern $\{A_0,...A_j,...,A_{M-1}\}$ |
| $S_0$ | $C'_0$ | $\{00...000\}$ |
| $S_1$ | $C'_1$ | $\{00...001\}$ |
| $S_2$ | $C'_2$ | $\{00...011\}$ |
| ... | ... | ... |
| $S_{N-1}$ | $C'_{N-1}$ | $\{11...111\}$ |
| Threshold value | | |
| $I_{th1}$ | | |
| Hybridization time $t_0$ | | |
| Nucleic acid | Concentration | Bit pattern $\{A_0,...A_j,...,A_{M-1}\}$ |
| $S'_0$ | $C'_0$ | $\{00...000\}$ |
| $S'_1$ | $C'_1$ | $\{00...001\}$ |
| $S'_2$ | $C'_2$ | $\{00...011\}$ |
| ... | ... | ... |
| $S'_{N-1}$ | $C'_{N-1}$ | $\{11...111\}$ |
| Threshold value | | |
| $I_{th2}$ | | |

As shown in Table 1, the bit pattern is associated with a hybridization time t and the nucleic acid concentration $C_i$ of the solution and stored (s20). Threshold values $I_{th1}$, $I_{th2}$, ... which are bases of the judgment are associated and stored together with the judgment table. When M×N bit data are obtained for each electrode area $A_j$, nucleic acid concentration $C_i$, and hybridization time, the calibration process ends.

To change the dynamic range of the concentration measurement, the hybridization time may be changed. Accordingly, the measurement is possible with the same sensor series.

Details of the current value acquisition processes of (s12) and (s15) are shown in the flowchart of FIG. 11. First, the hybridization is performed at a constant temperature for a constant time (s31), and the intercalating agent is introduced with the electrode having a different area to measure the current value I (s32). The obtained current value I is normalized with the electrode area (s33). The measurement and normalization of the current value are executed in the module 135 of FIG. 3. A detailed configuration of the circuit which performs the normalization will be described later. Moreover, the normalized current value $I_n$ is output to the processing unit 113 from the module 135 via the selector 136 and A/D converter 137. The processing unit 113 calculates a peak value $I_{np}$ of the obtained normalized current value $I_n$ by the fitting process (s34).

It is to be noted that each process of the measurement of these current values is not necessarily limited to the description herein. For example, the process including the normalization of the electrode area may also be performed in a processing unit 113. The peak value calculation process may also be performed in the module 135. Moreover, as shown in the example of FIG. 29, the peak value $I_p$ may also be calculated before the first normalization.

Next, the details of the measurement process (s2) of FIG. 9 will be described with reference to FIG. 12. This measurement process is executed after obtaining a judgment table shown in the calibration process (s20) in FIG. 10. It is to be noted that when the judgment table is obtained beforehand, the calibration process (s1) does not have to be performed before the measurement process (s2).

First, the solution of the specimen which is an object of measurement is introduced into the cell in which the sensors 12a are arranged, and the sensors 12a are immersed in the specimen solution (s21). Next, the current value is acquired (s22) through the process of (s31) to (s34) along the flow shown in FIG. 11. Next, the threshold value $I_{th}$ obtained in (s20) and stored in the storage unit 116 is read out. The processing unit 113 compares the threshold value $I_{th}$ with the measured current value of (s22) to acquire the bit data B (s23). The bit data B is obtained by representation of the case where the threshold value $I_{th}$ is exceeded as "1" and the case where the threshold value $I_{th}$ is not exceeded as "0" in the same manner as in (s18). The bit data B is obtained with respect to each electrode area $A_j$ to acquire the

bit pattern. The processing unit 113 searches Table 1 for the bit pattern which is identical with this bit pattern to determine the nucleic acid concentration $C_i$ associated with the bit pattern as the concentration of the specimen (s24). The measurement process ends as described above.

FIG. 13 is a diagram showing a detailed configuration of the circuit for performing the normalization in (s33) or (s82). In FIG. 13, the configuration common to that in the other figures such as FIGS. 3 and 5 is denoted with the same reference symbols and the detailed description is omitted.

As shown in FIG. 13, signal outputs of modules $135_0$, $135_1$, $135_2$ including three-electrode systems $140_0$, $140_1$, $140_2$ which have working electrodes 141 each having different electrode areas $A_0$, $\alpha A_0$, $\alpha^2 A_0$ ($\alpha < 1$) are connected to the selector 136. The configuration of the current detection circuit including a current mirror for positive/negative current including six transistors $M1_0$ to $M6_0$, $M1_1$ to $M6_1$, $M1_2$ to $M6_2$ is common to that of FIG. 5. In FIG. 13, the configuration of the counter electrode 142 and reference electrode 143 in the three-electrode systems $140_0$, $140_1$, $140_2$ is omitted. In the example of FIG. 13, these modules $135_0$, $135_1$, $135_2$, especially the current mirrors for positive/negative current function as a circuit to normalize the detected current obtained by the sensor with respect to the sensor area.

The gate of a transistor M7 is connected to output node of the current mirror for positive/negative current on a current via a switch $SW_1$. The source of the transistor M7 is connected to the drain of a depletion mode N-type MOSFET M8 and the selector 136. The source of a transistor M8 is connected to the gate. This is one of circuit configurations called a source follower. Needless to say, buffers may also be used such as the source follower constituted in another method and a voltage follower. A switch capacitor including a switch $SW_2$ and capacitor C is disposed between the output node of the current mirror for positive/negative current and the transistor M7. A charge flowing via a current mirror is accumulated in the capacitor C in an open state of the switch $SW_2$ by the function of this switched capacitor, and can be allowed to be discharged, when the switch $SW_2$ is closed.

When the switches $SW_1$ and $SW_2$ are open/close controlled in the following order, the currents of the respective modules $135_0$ to $135_2$ are selectively output to the selector 136.

Concretely, first the switch $SW_1$ is turned on, the switch $SW_2$ is turned off, a current i flowing for a time $\Delta t$ is integrated, and the opposite ends of the capacitor C are charged. Accordingly, voltages $\Delta t i/C$ proportional to time integral values of the currents are generated on the opposite ends of the capacitor C. Moreover, both the switches $SW_1$ and $SW_2$ are turned off. This voltage $\Delta t i/C$ is output to the selector 136 in the transistor M7 and M8. The switch $SW_1$ is turned off, and the switch $SW_2$ is turned on so that reset is possible. Here, when $\Delta t$ is determined as a micro value having sufficiently little change of the current, a proportional relation is established between the output voltage and current. As a result, current-to-voltage conversion is performed.

A ratio of W/L of transistor $M6_i$ to $M4_i$, where W and L respectively denote the gate with and length of a MOSFET, namely, a current amplification factor of the current mirror is set to be inversely proportional to the ratio of the electrode area to the largest electrode area $A_0$. This also applies to the other transistors $M3_i$ and $M5_i$. In the example of FIG. 13, the electrode area of $A_0$ is largest. Therefore, W/L of $M4_0$ and $M6_0$, and $M3_0$ and $M5_0$ is 1:1, W/L of $M4_1$ and $M6_1$, and $M3_1$ and $M5_1$ is $\alpha$:1, and W/L of $M4_2$ and $M6_2$, and $M3_2$ and $M5_2$ is $\alpha^2$:1. Here, $\alpha < 1$.

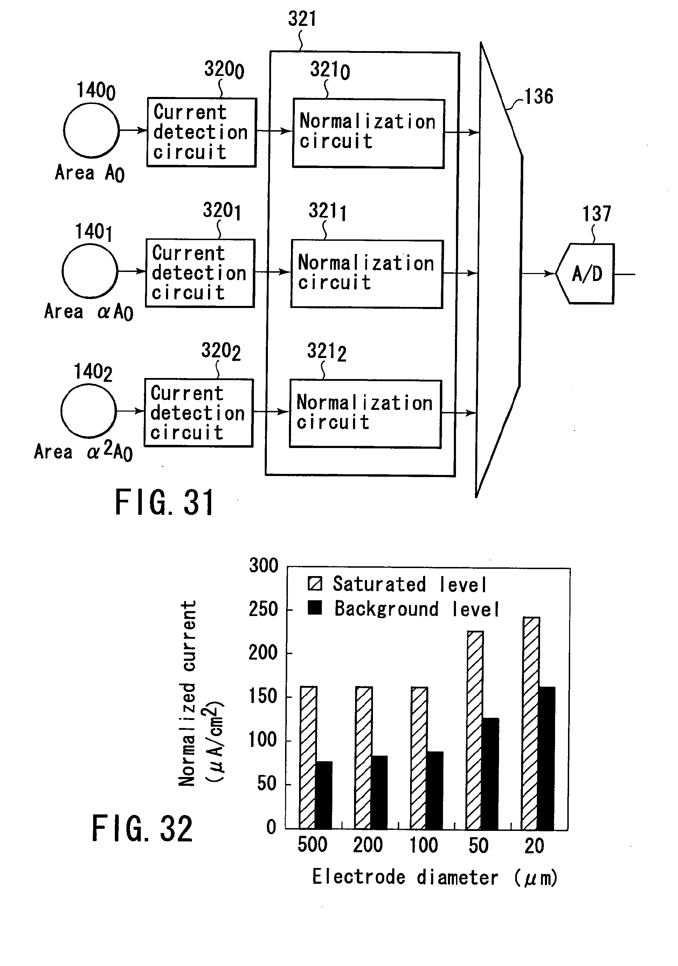

That is, a configuration is formed in which a normalization circuit is added to the current detection circuit including six transistors on the output side. FIG. 31 is a schematic diagram of the circuit configuration shown in FIG. 13. The currents of the respective three-electrode systems $140_0$, $140_1$, $140_2$ are detected by current detection circuits $320_0$, $320_1$, $320_2$. The detection currents are respectively output to normalization circuits $321_0$, $321_1$, $321_2$ in each normalization circuit 321, normalized with respect to the electrode area $A_i$, and output to the selector 136.

Accordingly, the detection current in the module $135_1$ is amplified by $1/\alpha$ times, and that in the module $135_2$ is amplified by $1/\alpha^2$ times. Therefore, the size can be normalized to that of the module $135_0$ having a largest current, and a conversion ratio of the current-to-voltage conversion circuit, A/D converter and the like can be common.

It is to be noted that the example of three modules has been described for the sake of convenience of the description with reference to FIG. 13, but the present invention is not limited to this. Needless to say, the module including other electrode areas $\alpha^3 A_0$, $\alpha^4 A_0$ ... includes the above-described configuration. Needless to say, the electrode area can variously be set in accordance with the precision of the concentration measurement. In general, assuming that a maximum electrode area of the system is $A_{max}$, an amplification factor in the current mirror having the electrode area of $A_1$ is represented by $a = A_{max}/A_1$.

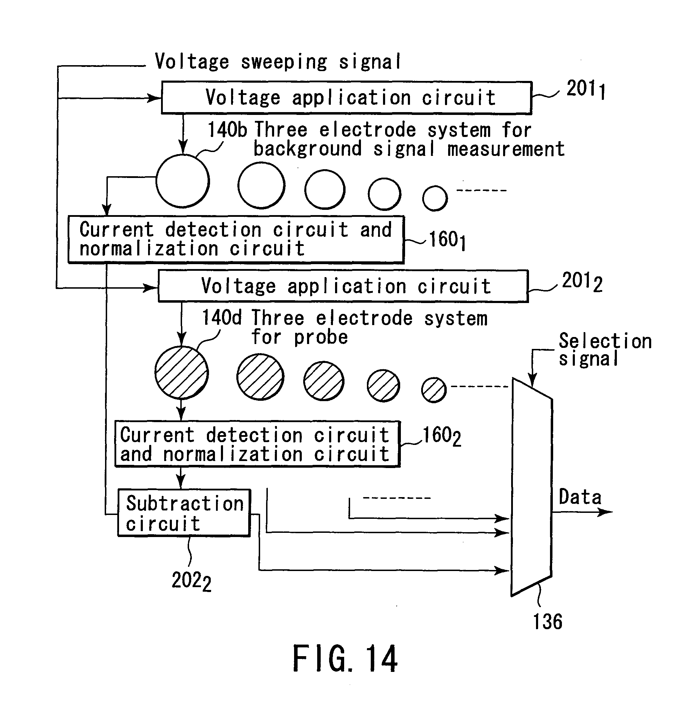

FIGS. 14 to 18 relate to modifications of the nucleic acid concentration quantitative analysis apparatus 1 shown in FIGS. 1 to 13.

FIG. 14 is a schematic diagram of the nucleic acid detection chip 12 in the modification. Reference numerals $201_1$ and $201_2$ of FIG. 14 denote voltage applying circuits disposed between the counter electrode 142 and reference electrode 143 and the voltage sweep signal generation means of FIGS. 4 and 5, and $160_1$ and $160_2$ denote the current detection circuit and normalization circuit connected to the working electrode 141 side of FIGS. 4 and 5. A current detection portion corresponds to a circuit including the operational amplifier 151 and resistance $R_w$ in the example of FIG. 4, and corresponds to a circuit including the transistors M1 to M6 and operational amplifier 151 in the example of FIG. 5.

Reference numeral 140b denotes a three-electrode system for background signal measurement (for negative control), and 140d denotes a three-electrode system for probe (for specimen measurement). Each of these three-electrode systems 140b and 140d includes the working electrode 141, counter electrode 142, and reference electrode 143 shown in FIGS. 4 and 5. The probe is not immobilized on the working electrode 141 belonging to the three-electrode system for background signal measurement 140b. A single-stranded probe is immobilized on the working electrode 141 belonging to the three-electrode system for probe 140d in the same manner as in FIGS. 4 and 5. Alternatively, the nucleic acid having a similarity , for example, of 50% or less with respect to the nucleic acid immobilized on the working electrode 141 belonging to the three-electrode system for probe 140d may also be immobilized as the probe on the working electrode 141 belonging to the three-electrode system for background signal measurement 140b. Here, the similarity is a ratio of the number of bases with respect to the total number of bases with respect to two nucleic acid pieces to be compared, in which the base of the corresponding portion is the same. Since a ratio of the specimen nucleic acid bonded to the probe for negative control is sufficiently small as compared with that of the probe immobilized on the three-electrode system for probe 140d, it is possible to simultaneously monitor the background level.

The measurement signals of the current detection circuit and the normalization circuits $160_1$ and $160_2$ are output to a subtraction circuit 202. The subtraction circuit 202 subtracts the measurement signal from the three-electrode system for background signal measurement 140b from that from the three-electrode system for probe 140d to output the signal to the selector 136.

Since the background level also differs with the area of the electrode, a set of electrodes for background monitor are disposed so that the counterpart of an electrode in the signal measurement set exists and the counterparts have the same area size with each other.

By this configuration, the detection signal from the three-electrode system for background signal measurement 140b can be subtracted from the detection signal from the three-electrode system for probe 140d, and a intrinsic signal can be obtained by subtracting the background level from the signal caused by the probe. As a result, changes of the background level by fluctuations of experiment conditions are constantly monitored, and the precision of the measurement is improved.

It is to be noted that although not described with reference to FIG. 14, the current-to-voltage conversion circuit may be appropriately disposed. For example, when the current-to-voltage conversion circuit is disposed in the subsequent stage of the subtraction circuit $202_2$, an output signal current of the subtraction circuit $202_2$ is converted to a voltage by the current-to-voltage conversion circuit and output to the selector 136. Alternatively, the current-to-voltage conversion circuit may also be disposed in the previous stage of the subtraction circuit $202_2$ and in the subsequent stage of the current detection circuits $160_1$, $160_2$. In this case, the output signal currents of the current detection circuits $160_1$, $160_2$ are converted to the voltages by the current-to-voltage conversion circuit and output to the subtraction circuit $202_2$.

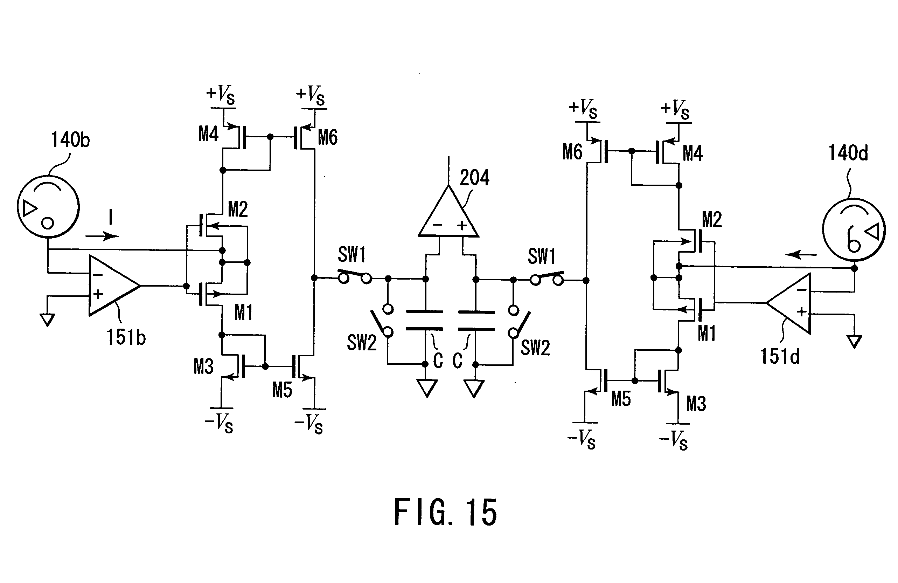

FIG. 15 shows a detailed configuration example of a circuit including the subtraction circuit 202. The example of the circuit of FIG. 15 is a circuit which performs current detection, current normalization, current-to-voltage conversion, and subtractionin order. As shown in FIG. 15, the operational amplifier 151, transistors M1 to M6, and switched capacitor shown in FIG. 13 form the same configuration on the background side as that on the probe detection side. The output of the switched capacitor is connected to a differential amplifier 204. The differential amplifier 204 corresponds to the subtraction circuit 202 of FIG. 14.

In the same manner as in FIG. 13, the transistors M1 to M6 normalize the current. Then, the switched capacitor including the capacitor C and switches $SW_1$ and $SW_2$ is operated, and the voltage value proportional to the current obtained from the three-electrode system for background signal measurement 140b, and the voltage value proportional to the current obtained from the three-electrode system for probe 140d are respectively output to the differential amplifier 204. The differential amplifier 204 outputs a difference between these voltage values to the selector 136.

In accordance with the circuit configuration of FIG. 15, since the current is used in the calculation on a secondary side of the current mirror, the operation of the three-electrode system electrochemical reaction or the like is not influenced. As characteristics of the circuit shown in FIG. 15, since the subtraction is performed before performing the data conversion by the A/D converter 137, the dynamic range of the output signal determined by the positive/negative voltage source of the circuit, and the dynamic range determined by the precision of the A/D converter 137 can effectively be used.

It is to be noted that a circuit topology shown in FIG. 15 is merely one example, and the above-described process can similarly be realized by various circuits and methods.

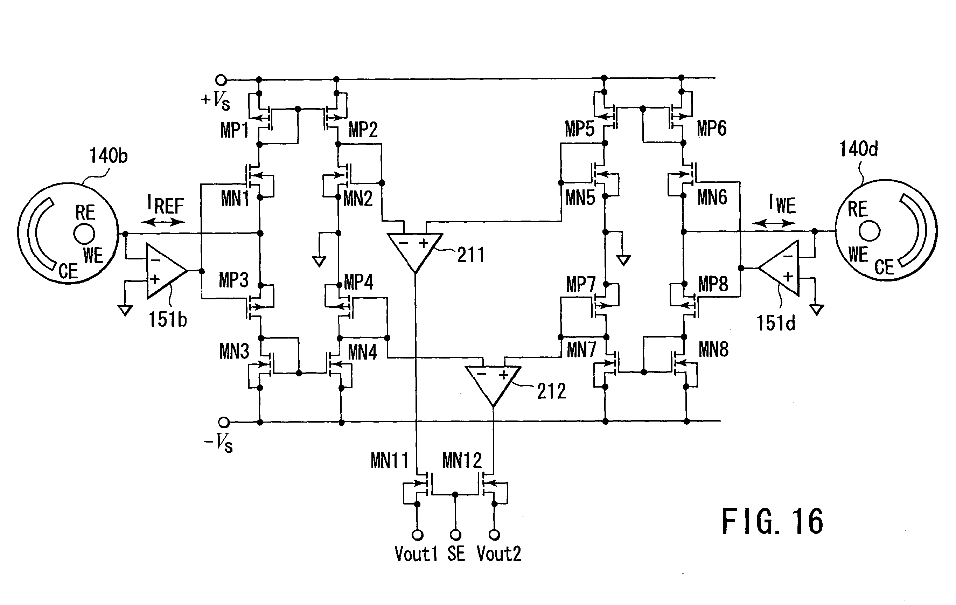

FIG. 16 is a diagram showing a modification of the subtraction circuit shown in FIG. 15. Also in FIG. 16, the configuration of the measurement circuit on the background side is common to that of the measurement circuit on the probe side in the same manner as in FIG. 15. That is, this example of the circuit of FIG. 16 is a circuit which performs the current detection, current normalization, current-to-voltage conversion, and subtraction in order.

As shown in FIG. 16, a connection relation between the three-electrode system for background signal measurement 140b and an operational amplifier 151b is common to that of FIG. 15. The output of the operational amplifier 151b is connected to the gates of a PMOS transistor MP3 and NMOS transistor MN1. A working electrode 141b of the three-electrode system for background signal measurement 140b is connected to the sources of the NMOS transistor MN1 and PMOS transistor MP3. The bulk and source of the NMOS transistor MN1 are short-circuited, and the drain is connected to the drain and gate of a PMOS transistor MP1. The bulk and source of the PMOS transistor MP1 are short-circuited, and the voltage is held at the positive voltage +Vs. The gate of the PMOS transistor MP1 is connected to that of a PMOS transistor MP2. The source and bulk of the PMOS transistor MP2 are short-circuited, and the voltage is held at the positive voltage +Vs. The PMOS transistors MP1 and MP2 form a current mirror topology. The drain of the PMOS transistor MP2 is connected to the drain and gate of an NMOS transistor MN2. The bulk and source of the NMOS transistor MN2 are grounded, and the gate of the transistor is connected to the inverting input terminal of a differential amplifier 211.

The drain of the PMOS transistor MP3 is connected to the gate and drain of an NMOS transistor MN3, and the gate of an NMOS transistor MN4. The bulk and source of the NMOS transistor MN3 are short-circuited, and the voltage is held at the negative voltage -Vs. The source and bulk of the NMOS transistor MN4 are short-circuited, and the voltage is held at the negative voltage - Vs. The NMOS transistors MN3 and MN4 form the current mirror topology.

The drain of the NMOS transistor MN4 is connected to the gate and drain of a PMOS transistor MP4 and further the inverting input terminal of a differential amplifier 212. The bulk and source of the PMOS transistor MP4 are grounded.

In the above, the transistors MP1, MP2, MN1, and MN2 operate during the measurement of the oxidation current, and the transistors MP3, MP4, MN3, and MN4 operate during the measurement of the reduction current.

The measurement circuit for background signal measurement and that for the probe signal measurement described above form a common circuit configuration. The constituting elements for the background signal measurement denoted with symbols 140b, 151b, MP1, MP2, MN1, MN2, MP3, MP4, MN3, and MN4 correspond to those for probe signal measurement denoted with 140d, 151d, MP6, MP5, MN6, MN5, MP8, MP7, MN8, and MN7.

Moreover, the gate of the NMOS transistor MN5 and the drain of the PMOS transistor MP5 are connected to the noninverting input terminal of the differential amplifier 211. The gate of the PMOS transistor MP7 and the drain of the NMOS transistor MN7 are connected to the noninverting input terminal of the differential amplifier 212.

The output of the differential amplifier 211 is connected to the drain of an NMOS transistor MN11. The gate of the NMOS transistor MN11 is connected to that of an NMOS transistor MN12, and the output is taken via a terminal SE. The voltage of the bulk and source of the NMOS transistor MN11 is taken out as a voltage $V_{out1}$.

The output of the differential amplifier 212 is connected to the drain of the NMOS transistor MN12. The voltage of the bulk and source of the NMOS transistor MN12 is taken out as a voltage $V_{out2}$.

When the reduction current is detected, the differential amplifier 212 subtracts the current detected on the working electrode 141b of the three-electrode system for background signal measurement 140b from the current detected on a working electrode 141d on the three-electrode system for probe 140d to send the output to the NMOS transistor MN11. The voltage $V_{out1}$ applied to the NMOS transistor MN11 by the current is a subtracted value.

In the oxidation current measurement, the differential amplifier 211 outputs the current value obtained by the subtraction in the same manner as in the differential amplifier 212 to the NMOS transistor MN12. The voltage $V_{out2}$ applied to the NMOS transistor MN12 by the current is the subtracted value.

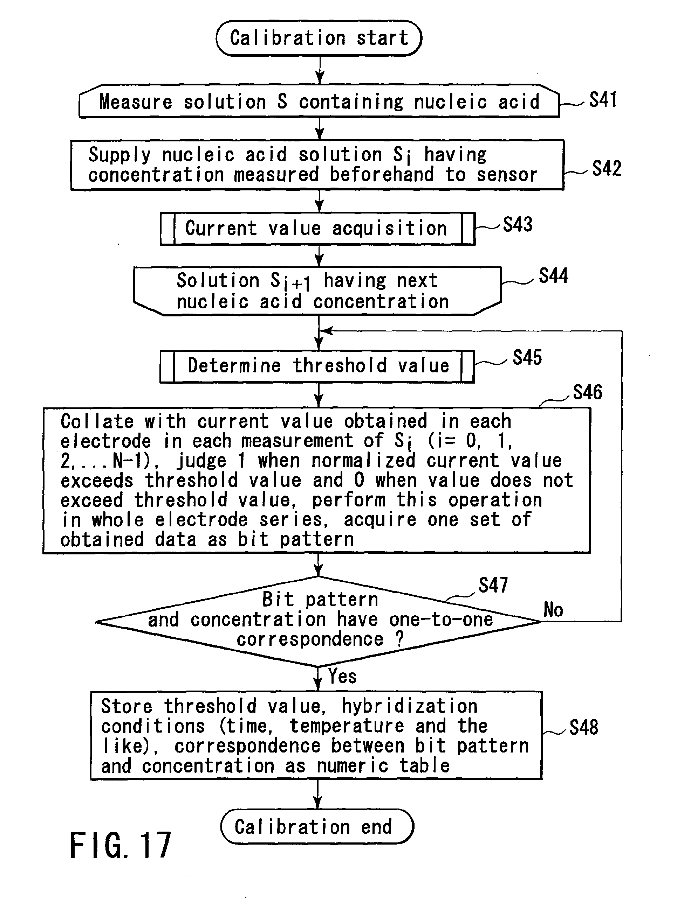

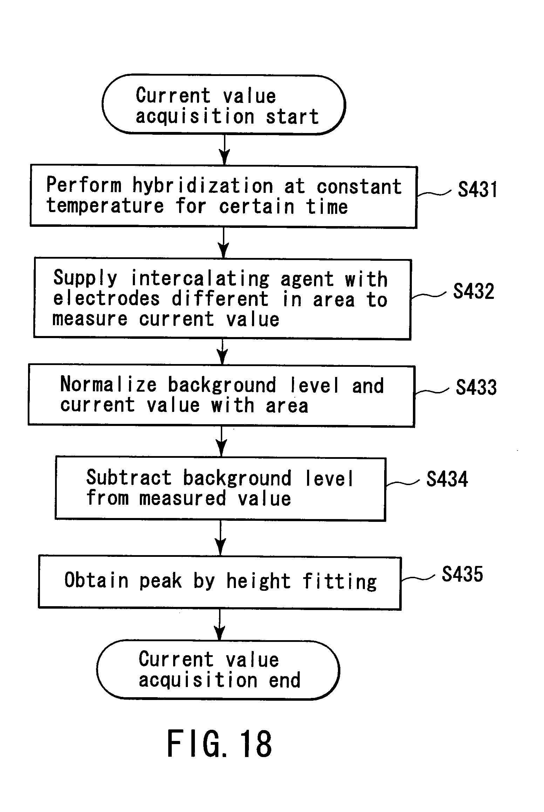

Next, an analysis process flow using a chip with an electrode for background measurement will be described with reference to FIGS. 17 and 18.

The flow is common to that of FIG. 9 in that the analysis process includes the calibration (s1) and measurement (s2).

The calibration process (s1) includes a process of (s41) to (s48) shown in FIG. 17. First, as the measurement of the solution $S_i$ containing the nucleic acid (s41), the nucleic acid solution $S_i$ having the known concentration $C_i$ is introduced into the cell including the sensors 12a having different electrode areas $A_j$ (j = 0, 1, ..., N-1) (s42). Moreover, the current value acquisition operation described later is performed (s43). Moreover, the current value of the nucleic acid solution $S_{i+1}$ having the concentration $C_{i+1}$ is acquired (s44). In this manner, the current values are acquired with respect to all the N types of nucleic acid solution $S_i$ (i = 0, 1, 2, ..., N-1) and all the sensors 12a having the electrode areas $A_j$. Next, the threshold value is calculated in the same manner as in (s17) (s45). It is to be noted that during the threshold value calculation, for the current values $I_p$, $I_n$, $I_o$ which are the bases of the calculation, as shown in the flowchart of FIG. 18 described later, the current value obtained by subtracting the background signal from the probe signal is calculated and used.

Next, the processing unit 113 compares the threshold value $I_{th}$ obtained in (s45) with the current value $I_n$ acquired and normalized in each measurement of each nucleic acid solution $S_i$. When the normalized current value $I_n$ exceeds the threshold value $I_{th}$, "1" is determined. When the value does not exceed the threshold value, "0" is determined. The set of judgment results obtained by the judgment process with respect to all the electrode series is acquired as the bit pattern (s46). Next, in (s47), the processing unit 113 determines whether or not the obtained bit pattern has the one-to-one correspondence with respect to the concentration in the same manner as in (s19). With the correspondence, the flow advances to (s48). In (s48), the processing unit 113 associates the bit pattern with the hybridization time t and the nucleic acid concentration $C_i$ of the solution to store the pattern as the judgment table together with the threshold value $I_{th}$ in the same manner as in (s20). With non-correspondence, the flow returns to the setting process of the threshold value $I_{th}$ again.

FIG. 18 is a detailed flowchart of the current value acquisition operation shown in (s43) of FIG. 17. As shown in FIG. 18, first the hybridization is performed at the constant temperature for the certain time (s431) in the procedure of (s11) and (s12) of FIG. 11. Moreover, the intercalating agent is supplied to the electrodes having different areas to measure the background level, probe current, and current value (s432). The obtained current value is normalized with the electrode areas $A_j$, for example, by the current mirror circuit represented by transistors $M1_i$ to $M6_i$ of FIG. 13 (s433). Furthermore, for example, the subtraction circuit 202 of FIG. 14 subtracts the background current value from the probe current value (s434). Moreover, in (s435), the processing unit obtains the peak value of the obtained subtracted value by the fitting process in the same manner as in (s34).

As described above, the intrinsic probe signal from which the background level is subtracted can be obtained through the processing flows shown in FIGS. 9, 11, 17, 18.

In the detection of the nucleic acid having the low concentration, it is very important to remove various noise components from the intrinsic signal components obtained in the measurement. In accordance with the modification shown in FIGS. 14 to 18, a current component caused by the intercalating agent bound on any place other than double-stranded nucleic acids and mixed in the signal as a noise, can be removed.

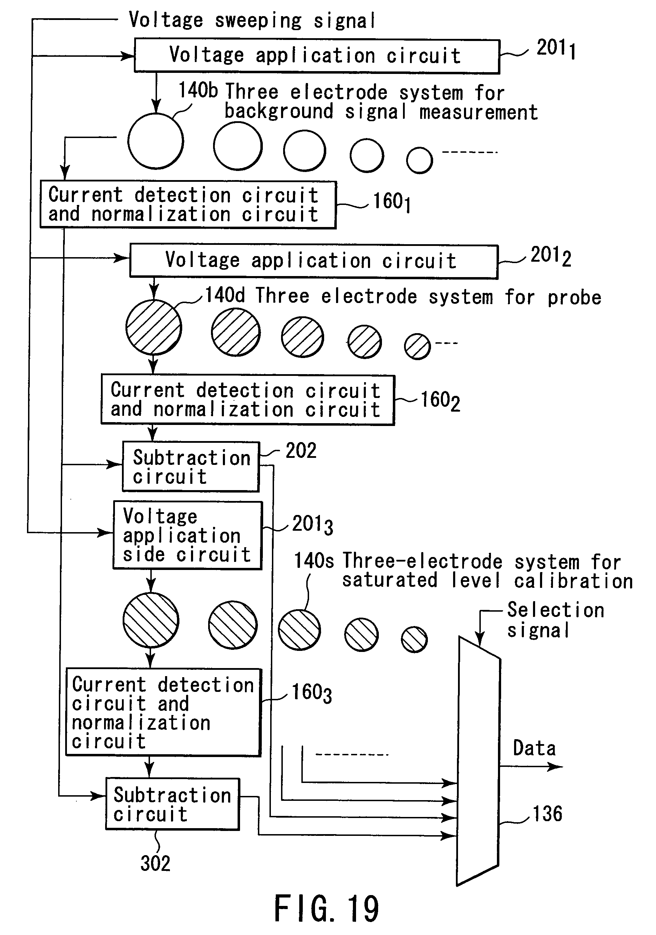

FIGS. 19 to 22 relate to further modifications of the nucleic acid concentration quantitative analysis apparatus 1 shown in FIGS. 1 to 13 and the analysis apparatus 1 using the chip provided with the electrode for background measurement described with reference to FIGS. 14 to 18. FIG. 19 is a schematic diagram of the nucleic acid detection chip 12 of the modification.

As shown in FIG. 19, in addition to the three-electrode system for background signal measurement 140b and three-electrode system for probe 140d shown in FIG. 14, a three-electrode system for saturated level calibration 140s is disposed. Also for the three-electrode system for saturated level calibration 140s, in the same manner as in the three-electrode systems 140b and 140d, a set of electrodes for saturated level calibration are disposed so that the counterpart of an electrode in the signal measurement set or background monitoring set exist and the counterparts have the same area size among them. This is because the saturated level changes with the area of the electrode.

These three-electrode systems 140b, 140d, and 140s have a common basic configuration, but the double-stranded probe in which the hybridization has already taken place is immobilized on a working electrode 141s of the three-electrode system for saturated level calibration 140s. The configuration common to that of FIG. 14 is denoted with the same reference numerals, and the detailed description is omitted.

The voltage sweep signal is input into the three-electrode system for saturated level calibration 140s via a voltage applying circuit $201_3$. The output of the three-electrode system for saturated level calibration 140s is connected to a subtraction circuit 302 via a current detection circuit and normalization circuit $160_3$. In the subtraction circuit 202, the background current signal is subtracted from the probe current signal to output the signal to the selector 136 in the same manner as in FIG. 14. On the other hand, the subtraction circuit 302 subtracts the background current signal from a saturated level current signal to output the signal to the selector 136.

In this manner, since both the background level and the saturated level can be measured, the measurement data can be normalized by both the electrode area and the value obtained by subtracting the background level from the saturated level. Therefore, the threshold value $I_{th}$ can constantly be adjusted to the adequate value regardless of the fluctuations of the experiment conditions. The adequate value is, for example, an intermediate value between the saturated level and the background level, that is, $I_{th} = (I_{st} - I_{bg})/2$. Accordingly, the measurement precision is further improved. Therefore, it is not necessary to measure the threshold value $I_{th}$ every measurement.

It is to be noted that although not described with reference to FIG. 19, the current-to-voltage conversion circuit may be appropriately disposed. For example, when the current-to-voltage conversion circuit is disposed in the subsequent stage of the subtraction circuit $202_2$, the output signal current of the subtraction circuit $202_2$ is converted to the voltage by the current-to-voltage conversion circuit and output to the selector 136. Alternatively, the current-to-voltage conversion circuit may also be disposed in the previous stage of the subtraction circuit $202_2$ and in the subsequent stage of the current detection circuits $160_1$, $160_2$. In this case, the output signal currents of the current detection circuits $160_1$, $160_2$ are converted to the voltages by the current-to-voltage conversion circuit and output to the subtraction circuit $202_2$.

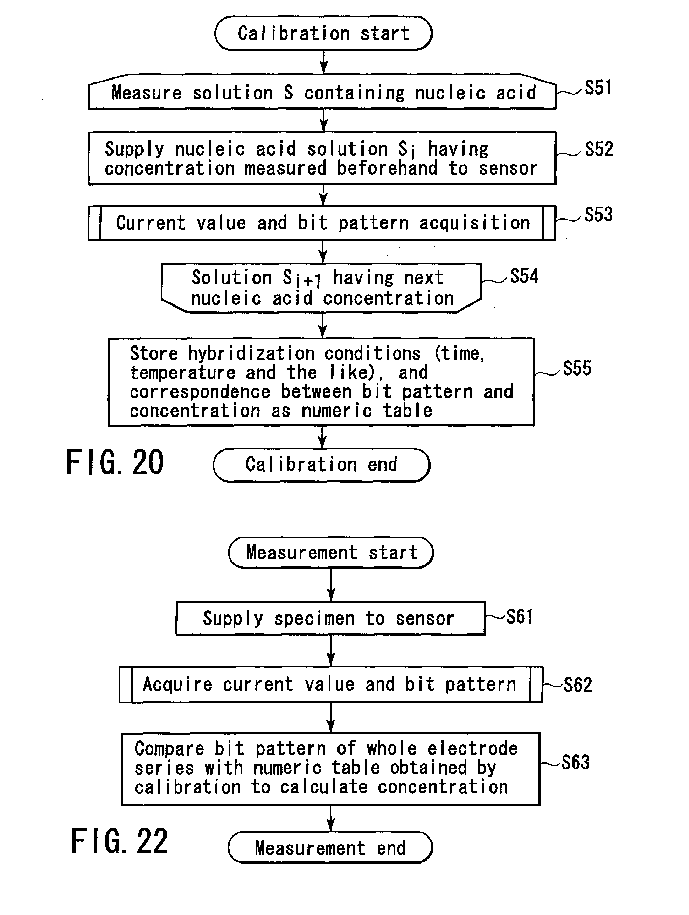

Next, the analysis process flow using the chip provided with the electrode for saturated level calibration will be described with reference to FIGS. 20 to 22.

The flow is common to that of FIG. 9 in that the analysis process includes the calibration (s1) and measurement (s2).

The calibration process (s1) includes a process of (s51) to (s55) shown in FIG. 20. First, as the measurement

of the solution $S_i$ containing the nucleic acid (s51), the nucleic acid solution $S_i$ having the known concentration $C_i$ is introduced into the cell including the sensors 12a having the different electrode areas $A_j$ (j = 0, 1, ..., N-1) (s52). Moreover, the current value and bit pattern acquisition operation described later is performed (s53). Moreover, the current value of the nucleic acid solution $S_{i+1}$ having the concentration $C_{i+1}$ and the bit pattern are acquired (s54). In this manner, the current values are acquired with respect to all the N types of nucleic acid solutions $S_i$ (i = 0, 1, 2, ..., N-1) and all the sensors 12a having the electrode areas $A_j$. Next, in (s55), the processing unit 113 associates the bit pattern with the hybridization time t and the nucleic acid concentration $C_i$ of the solution to store the judgment table in the same manner as in (s20).

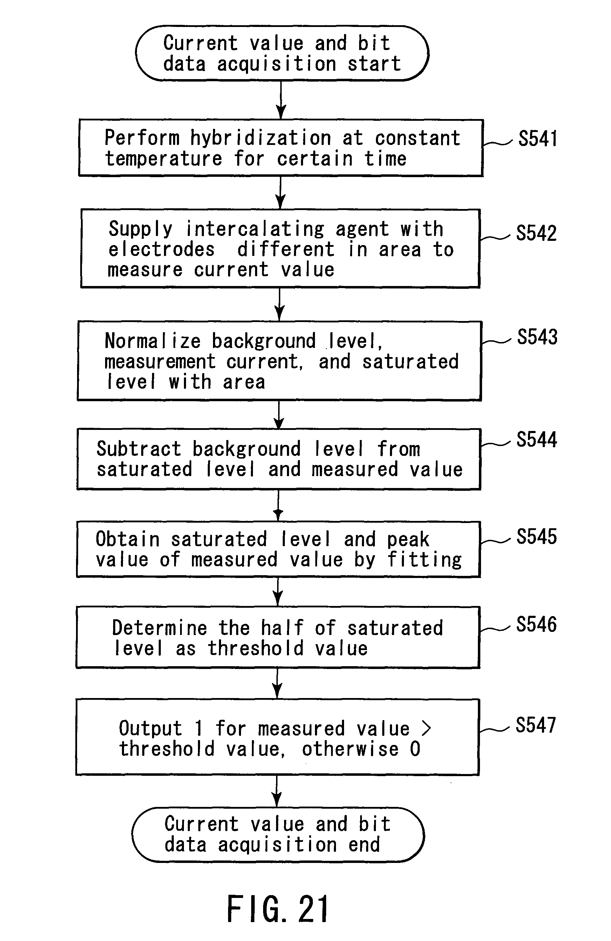

FIG. 21 is a detailed flowchart of the current value and bit pattern acquisition operation shown in (s54). As shown in FIG. 21, first the hybridization is performed at the constant temperature for the certain time (s541) in the procedure of (s11) and (s12) of FIG. 11. Moreover, the intercalating agent is supplied to the electrodes having different areas $A_j$ to measure the background level, probe current, and current values $I_{bg}$, $I$, $I_{st}$ of saturated levels (s542). The obtained current values $I_{bg}$, $I$, $I_{st}$ are normalized, for example, by the current mirror circuit represented by the transistors $M1_i$ to $M6_i$ of FIG. 13 (s543). Furthermore, for example, the subtraction circuit 202 shown in FIG. 19 subtracts the background level $I_{bg}$ from the measured value I, and the subtraction circuit 302 subtracts the background level $I_{bg}$ from the saturated level $I_{st}$ (s544). Moreover, in (s545), the processing unit 113 obtains the peak values of both the subtracted value of $I - I_{bg}$ and the peak value of $I_{st}-I_{bg}$ by the fitting process in the same manner as in (s34). A value of $(I_{st} - I_{bg})/2$ is set to the threshold value $I_{th}$ (s546). Next, the processing unit 113 compares the obtained threshold value $I_{th}$ with the measured value I. As a result of the comparison, in the case of $I > I_{th}$, the processing unit 113 determines "1". In the case of $I \leq I_{th}$, "0" is determined, and the bit data is acquired (s547).

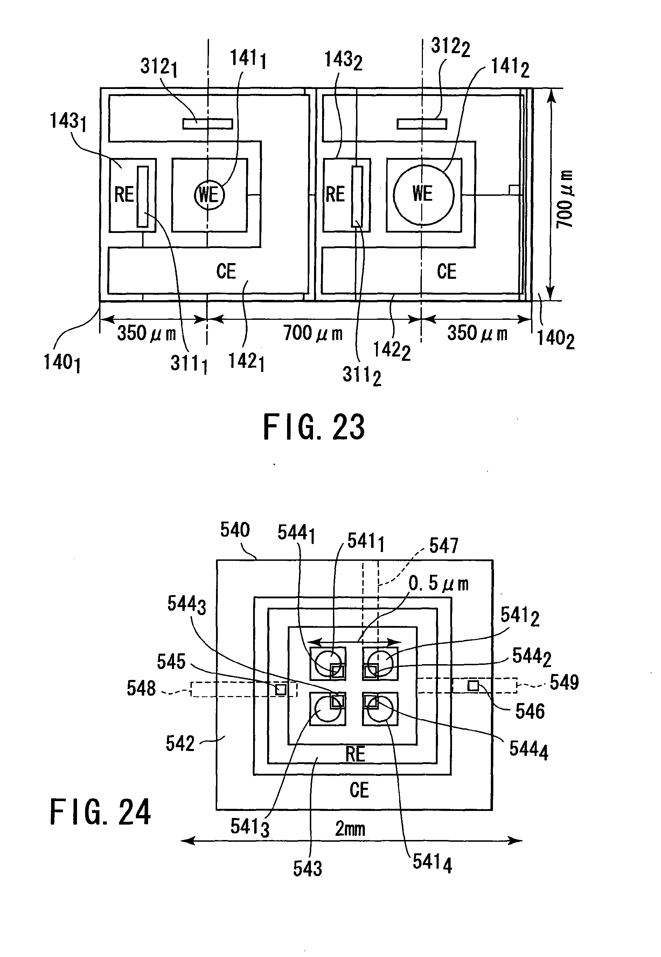

FIG. 22 is a detailed process flowchart of the measurement process (s2) using the chip provided with the electrode for saturated level calibration. As shown in FIG. 22, first the solution of the specimen which is the object of measurement is introduced into the cell in which the sensors 12a are arranged, and the sensors 12a are immersed in the specimen solution (s61). Next, the current value and bit pattern are acquired (s62) through the process of (s541) to (s547) of FIG. 21. Next, the processing unit 113 collates the bit pattern of the whole electrode series obtained with respect to the specimen solution with the judgment table obtained in (s55) of the calibration process (s1) of FIG. 20 to determine the identical bit pattern as the solution concentration C (s63). The measurement ends as described above.

It is to be noted that in the embodiment shown in FIGS. 19 to 22, the example in which the three-electrode system is disposed to detect both the saturated level and the background level has been described, but the three-electrode system to detect the background level is not disposed, and only a set of the three-electrode system for saturated level calibration 140s and three-electrode system for probe 140d may also be disposed. In this case, the configuration for the background level shown in FIG. 14 is replaced with that for the saturated level calibration and the sign of the subtraction result is reversed. Alternatively, the ratio of the measurement signal from probe to the saturated level measurement signal is taken between the counterpart sensors, and the concentration of the target nucleic acid contained in the specimen may also be determined from the intensity of the signal obtained from the pair in which the ratio is not 100%.

FIG. 23 is a plan view of one example of the detailed configuration of an electrode arrangement of the three-electrode system 140 in the embodiments of FIGS. 1 to 13, 14 to 18, 19 to 22. In FIG. 23, for the convenience of the description, two adjacent three-electrode systems $141_1$ and $141_2$ will be described, but the similar configuration is formed also with respect to a plurality of three-electrode systems $140_i$ (i = 0, 1, ..., N-1). Both the three-electrode systems $140_1$ and $140_2$ are disposed in a square region, for example, with 700 $\mu m \times 700$ $\mu m$. The configurations of counter electrodes $142_1$ and $142_2$, and reference electrodes $143_1$ and $143_2$ are common, and the working electrodes $141_1$ and $141_2$ have different areas. The working electrodes $141_1$ and $141_2$ are disposed in central positions of regions where the three-electrode systems $140_1$ and $140_2$ are formed, and the counter electrodes $142_1$ and $142_2$ are disposed in U shapes so as to surround three directions of the working electrodes $141_1$ and $141_2$. Moreover, the reference electrodes $143_1$ and $143_2$ are disposed on a side on which the counter electrodes $142_1$ and $142_2$ are not disposed as seen from the working electrodes $141_1$ and $141_2$.

As described above, one of counter electrodes $142_1$ and $142_2$ and one of reference electrodes $143_1$ and $143_2$ are disposed for each of the working electrodes $141_1$ and $141_2$ so that the three electrodes with any distance in a substantially constant position. Since the counter electrodes $142_1$ and $142_2$, and reference electrodes $143_1$ and $143_2$ have the same configuration, the positional relation lies in the equal distance.

Furthermore, each of feedback circuits for voltage application of the three-electrode systems $140_1$ or $140_2$ are also connected to each of the counter and reference electrode set of $142_1$ and $143_1$ or $141_2$ and $143_2$. Reference numerals $312_1$, $312_2$, $311_1$ and $311_2$ denote contacts to be connected to an interconnection in the lower layer.

In order to use the precision of the A/D converter 137 sufficiently, as shown in FIG. 15 or 16, it is effective to subtract the detection current in an analog circuit. In this case, as shown in FIG. 18 or the like, after the subtraction, a peak height is analyzed. When a peak position deviates among the three-electrode system for background signal measurement 140b, three-electrode system for probe 140d, and three-electrode system for saturated level calibration 140s, there is a possibility that the measurement precision of the analysis result is adversely affected. The deviation of the peak position is caused by presence of solution resistance components in many cases.

When the counter electrode and reference electrode are disposed for each working electrode as shown in FIG. 23, the fluctuation of the solution resistance caused in the case where a single set of a counter electrode and a reference electrode are disposed for a plurality of working electrodes can be eliminated. As compared with a case where there is only one feedback loop for a plurality of working electrodes, the voltage between the reference electrode and a comparative pole can be controlled in accordance with a slight difference of measurement conditions.

FIG. 24 is a plan view of the electrode arrangement different from that of FIG. 23.

In FIG. 24, in a three-electrode system 540, four working electrodes $541_1$ to $541_4$ are arranged at a predetermined interval in a 0.5 mm square region. One reference electrode 543 is disposed so as to surround four working electrodes $541_1$ to $541_4$ at the predetermined interval. Furthermore, one counter electrode 542 is disposed so as to surround the reference electrode 543 at the predetermined interval. This three-electrode system 540 is disposed within a 2 mm square region. Distances to the counter electrode 542 and reference electrode 543 from the respective working electrodes $541_1$ to $541_4$ are substantially equal. Each electrode is disposed in a symmetric position as seen from the center of the working electrodes $541_1$ to $541_4$, and is formed, for example, of Au.

The example of FIG. 24 shows a case where the electrode areas of four working electrodes $541_1$ to $541_4$ are common, but different electrode areas may also be used. Reference numerals $544_1$ to $544_4$, 545, 546 denote contacts to be connected to an interconnection in a lower layer, and 547 to 549 denote Al interconnections formed under the electrodes and correspond to second layer interconnections $171_2$ in the sectional structure of FIG. 5.

In this manner, one reference electrode or one counter electrode may also be disposed with respect to a plurality of working electrode. This arrangement is effective in a case where there are restrictions to the size of the circuit or a droplet radius of the solution that can be dropped onto the substrate.

Moreover, a structure in which the working electrode is surrounded with the reference electrode and counter electrode also has an effect of avoiding electrostatic or electromagnetic disturbance of an outer field with respect to the working electrode, and is effective as a countermeasure against noises of measurement. Any concentration does not easily occur in distribution of an electric field, and the fluctuation of measurement is effectively reduced.

It is to be noted that FIGS. 23 and 24 show a planar electrode arrangement structure, but the present invention is not limited to this. For example, the respective electrodes 141 to 143 may also have a three-dimensional solid structure.

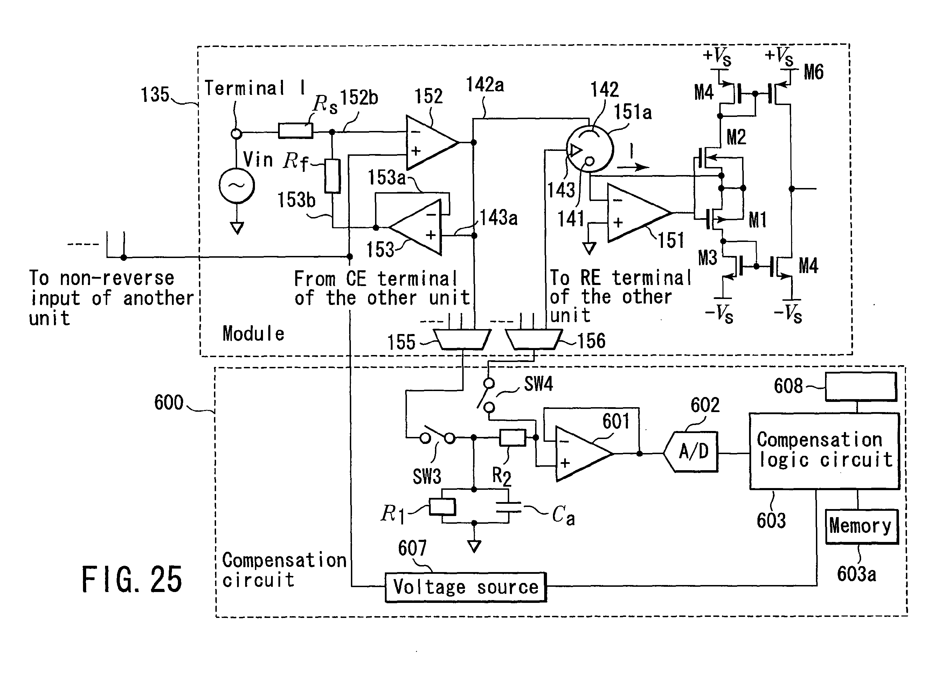

FIG. 25 is a diagram showing one example of a configuration of a compensation circuit 600 to which a function of compensating for offset of a sweeping voltage and linearity is added in the respective embodiments of FIGS. 1 to 13, FIGS. 14 to 18, FIGS. 19 to 22. The configuration common to that of FIG. 5 is denoted with the same reference symbols, and the detailed description is omitted. For example, when the modules 135 are arranged in an array, the compensation circuit 600 compensates for positional unevenness of a semiconductor manufacturing process, or the offset or linearity of the sweeping voltage caused by the deviation of the device dimension with respect to a designed value.

The compensation circuit 600 is disposed in the analysis apparatus housing 11 of FIG. 1, and the nucleic acid detection chip 12 is attached to the analysis apparatus housing 11 to physically connect the nucleic acid detection chip 12 to the reagent feed/temperature control apparatus 111. When the nucleic acid detection chip 12 is electrically connected to the chip/housing interface 112, the wirings 142a and 143a in each module 135 of the nucleic acid detection chip 12 are automatically connected to switches $SW_3$ and $SW_4$ via selectors 156 and 155. The signals from the wirings 142a and 143a of each module 135 are selected by the selectors 156 and 155 and output toward the switches $SW_3$ and $SW_4$.

The output of the operational amplifier 152 connected to the counter electrode 142 shown in FIG. 25 is connected to a circuit in which a resistance $R_1$ and capacitor $C_a$ are connected in parallel via the switch $SW_3$. These resistance $R_1$ and capacitor $C_a$ are connected to one end of a resistance $R_2$. The other end of the resistance $R_2$ is connected to the switch $SW_4$ and a noninverting input of an operational amplifier 601.

These resistance $R_1$, capacitor $C_a$, and resistance $R_2$ form a circuit simulating a solution system in the cell including three electrodes 141 to 143. The resistance and capacity values are set, for example, to $R_1 = 1$ MΩ, $C_a = 200$ nF, $R_2 = 1$ kΩ.

The inverting input and output of the operational amplifier 601 are short-circuited, and the amplifier functions as a voltage follower. The output of the operational amplifier 601 is connected to a compensation logic circuit 603 via an A/D converter 602.

The compensation logic circuit 603 has a function of compensating for the offset or linearity of the sweeping voltage generated by each module 135, and may also be realized by a combination of hardware and software or only by hardware. The compensation logic circuit 603 stores the measured value obtained from each module 135 in a memory 603a. Moreover, the compensation logic circuit 603 outputs signals for offset compensation and linearity compensation to a voltage source 607 based on the stored measured value. The voltage source 607 applies the voltage instructed from the compensation logic circuit 603 to the noninverting input terminal of the operational amplifier 152.

The operation of the compensation circuit 600 will hereinafter be described.

When the nucleic acid detection chip 12 is attached to the analysis apparatus housing 11, the output of the selector 155 is electrically connected to the switch $SW_3$, and the output of the selector 156 is electrically connected to the switch $SW_4$. Both the switches $SW_3$ and $SW_4$ are turned on before the solution measurement. The voltage source 607 applies a predetermined voltage to the noninverting input terminal of the operational amplifier 152 based on the command from the compensation logic circuit 603. Accordingly, a voltage $V_{tk}$ is applied to the resistance $R_1$, capacitor $C_a$, and resistance $R_2$ simulating the solution system. The voltage $V_{tk}$ (k = 1, 2, ..., K) is output to the compensation logic circuit 603 via the operational amplifier 601 and A/D converter 602. The compensation logic circuit 603 sequentially stores the voltage $V_{tk}$ in the memory 603a with respect to all K modules 135. It is to be noted that the module 135 is selected by the selectors 155 and 156, but the successive selection operation by the selectors 155 and 156 is controlled by the circuit on the analysis apparatus housing 11 side. Moreover, the compensation logic circuit 603 calculates an average value $V_{tav}$ of the output voltages $V_{tk}$, for example, with respect to all the modules 135. Furthermore, the compensation logic circuit 603 determines whether or not a difference ($V_{tk}$ - $V_{tav}$) between the average value $V_{tav}$ and each output voltage $V_{tk}$ is in a predetermined range. Within the predetermined range, the compensation logic circuit 603 displays "satisfactory product" in a display unit 608. Out of the predetermined range, "defect" is displayed in the display unit 608.

When the "satisfactory product" is determined, the difference between the output voltage $V_{tk}$ of each module 135 and the average value $V_{tav}$ is stored as an offset $V_{kof}$ together with the average value in the memory 603a. During the actual measurement, the measured value obtained from each module 135 is corrected in accordance with a correction value for the offset $V_{kof}$ of the memory 603a, and accordingly a measurement error of the obtained actual measured value can be corrected. Moreover, during the actual measurement, the sweeping voltage of each module 135 may also be corrected by the correction value in accordance with the average value $V_{tav}$ of the output voltages $V_{tk}$. Concretely, when an offset compensation voltage -$V_{tav}$ is applied from the voltage source 607 during the actual measurement, the offset can be compensated.

In this manner, a deviation from the predetermined voltage, that is, the offset of the feedback circuit or a deviation of the output voltage with respect to the input voltage can be known from an inverting output voltage which appears in the reference electrode 143. Especially the offset can be removed by the adjustment of the voltage applied to the noninverting input of the operational amplifier 152, and the precision of feedback can be improved. The semiconductor circuit including the modules 135 prepared in the array is disposed in the same chip so as to satisfy translation symmetry. This disposition scheme produces a uniformity of some influences appearing among elements in the array caused by unevenness in a semiconductor manufacturing process, like a process gradation. More concretely, all the operational amplifiers 152 existing in different modules are disposed in the same direction. This also applies to the operational amplifiers 151, 153. This reduces the difference caused between the modules. Therefore, when a common voltage is simply applied to the noninverting input terminal of the operational amplifier 152, a large part of offset can simultaneously be eliminated.

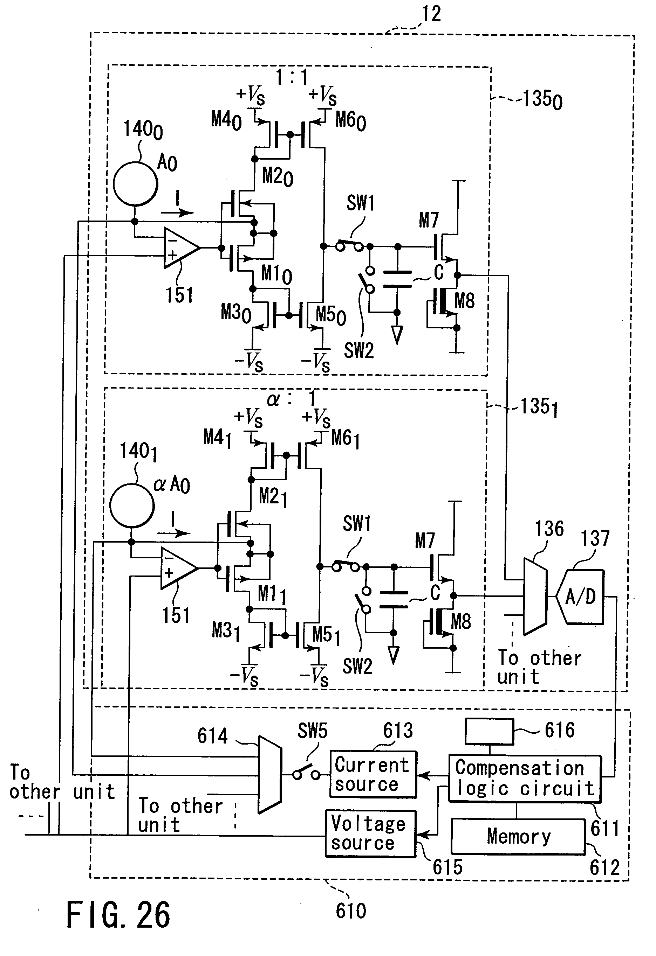

FIG. 26 is a diagram showing one example of a compensation circuit 610 which compensates for not only the offset but also a deviation of the linearity, that is a coefficient of proportionality between the input and the output, of the measurement circuit with respect to a designed value in the respective embodiments of FIGS. 1 to 13, FIGS. 14 to 18, FIGS. 19 to 22. The configuration common to that of FIG. 5 or 13 is denoted with the same reference symbols, and the detailed description is omitted.

The compensation circuit 610 is disposed in the analysis apparatus housing 11 of FIG. 1, and the nucleic acid detection chip 12 is attached to the analysis apparatus housing 11 to physically connect the nucleic acid detection chip 12 to the reagent feed/temperature control apparatus 111. When the nucleic acid detection chip 12 is electrically connected to the chip/housing interface 112, the A/D converter 137 of the nucleic acid detection chip 12 is automatically and electrically connected to a compensation logic circuit 611 of the compensation circuit 610 via the interface 131. As a result, the output of a selector 614 is connected to the working electrode 141 of each module $135_0$, $135_1$. The electrode areas of the modules $135_0$ and $135_1$ are $A_0$ and $\alpha A_0$ respectively, and current amplification factors are 1:1, $\alpha$:1. It is to be noted that here two modules $135_0$ and $135_1$ are described for the convenience of the description with reference to FIG. 26, but, needless to say, the present invention can also similarly be applied to three or more modules.

As shown in FIG. 26, the compensation circuit 610 includes a memory 612, the compensation logic circuit 617 including a display unit 616, a current source 613, a voltage source 615, and the selector 614. The output of the compensation logic circuit 617 is connected to the current source 613 and voltage source 615. The current source 613 is connected to the input of the selector 614 via a switch $SW_5$. The outputs of the voltage source 615 are connected to the noninverting inputs of the operational amplifiers 151 of the modules $135_0$ and $135_1$. When the currents are passed into the current detection circuits of the modules $135_0$ and $135_1$ connected to the working electrodes 141 from the current source 613, the current observed at an actual measurement time can be applied from the outside of the module in a simulating manner.

The switch $SW_5$ is turned on, and the current is selectively passed into each of the modules $135_0$ and $135_1$ through the working electrode 141 node from the current source 613 via the selector 614. In this stage, the voltage is not applied from the voltage source 615. This current is output to the compensation logic circuit 611 via the current detection circuit, normalization circuit, selector 136, and A/D converter 137. The compensation logic circuit 617 measures the linearityor offset by utilizing the signal from the A/D converter 137, and stores the measured value into the memory 612.

After the above-described advance measurement, as shown in FIG. 9, the calibration (s1) and measurement (s2) are performed, and the obtained measurement results are corrected based on the measured values.

Accordingly, the measurement results can be obtained by the correction of the deviations of the linearityoffset and the like of the measurement circuit including the current mirror circuit with respect to the designed values.

Moreover, a response of each module 135 to the input of the current source 613 is analyzed, and the appropriate voltage is input into the noninverting input terminal of the operational amplifier 151 from the voltage source 615 based on the result. Accordingly, it is possible to compensate for the offset in the same manner as in FIG. 25.

Concretely, positive and negative currents of $\pm\Delta I_0$ and $\pm\Delta I_1$, of which absolute values are identical respectively to the upper limit values of the offset current $\Delta I_0$ and $\Delta I_1$ defined in the specifications with respect to the sensors of the modules $135_0$ and $135_1$, are input respectively into the modules $135_0$ and $135_1$ through the selector 614 to observe the response. When $+\Delta I_0$ and $+\Delta I_1$ are input, output values are certain positive values. When $-\Delta I_0$ and $-\Delta I_1$ are input, the output values are certain negative values. In this case, the sensor of the noted module satisfies the specifications. Here, when the preparation process of the apparatus is appropriate with respect to the specifications, the appropriate voltage is input into the noninverting input terminal of the operational amplifier 151. Accordingly, in all the modules 135, it is possible to find such conditions that the output values are positive with the inputs of $+\Delta I_0$ and $+\Delta I_1$ and the output values are negative with the inputs of $-\Delta I_0$ and $-\Delta I_1$. When the conditions are satisfied, the offset is removed in an optimum manner.

Moreover, at this time, it is also possible to simultaneously determine the "satisfactory product" and "defect". That is, when there is not any voltage capable of removing the offset in the optimum manner, the offset of the measurement circuit does not satisfy the specifications, and therefore the processing unit 113 determines the "defect".

FIG. 27 is a diagram showing a modification of the nucleic acid detection chip. A nucleic acid detection chip 700 shown in FIG. 27 includes a chip on glass structure in which a plurality of Si chips 702 and arrayed three-electrode systems 140 are arranged on a glass substrate 701. Each three-electrode system 140 is connected to any of the Si chips 702 via the wiring, and the detection signal in the three-electrode system 140 is processed on an Si chip 702 side. The Si chip 702 is connected to the chip/housing interface 112, and the signal is output to the processing unit 113.

As described above, in accordance with the present embodiment, the concentration can quantitatively be analyzed in a broad dynamic range of the sensitivity by using the current detection type nucleic acid detection chip 12.

Moreover, when a circuit is integrated on the identical substrate with the probe array, it is possible to keep the simultaneity of the measurement time while reducing electric noises.

The simultaneity is important in the current measurement type chip, because the signal intensity fluctuates depending on the degradation of the intercalating reagent, for instance Hoechst 33258, or the accumulated amount of the intercalating reagent bound on the double-stranded nucleic acid, which is constituted of a probe and a target, by a time progress. Especially for the signal to be compared, the simultaneity is preferably ensured as much as possible. As shown in FIG. 1, this is realized by integration of the same number of circuits for measurement and probes in the nucleic acid detection chip 12 on which a large number of probes are mounted in the array. Since the reduction of noises by electric disturbance is also anticipated by the integration of the circuit, the electric noises generated in a peripheral circuit can also be removed.

Furthermore, in accordance with the present embodiment, the signal detected by the probe and the background current observed at the same time are directly subtracted from the current detected from the probe, and a intrinsic signal current is correctly obtained. Accordingly, when the signal level is relatively small with respect to the background level, for example, the dynamic range of the amplification circuit or the A/D converting circuit positioned in the subsequent stage of the subtraction circuit can effectively be used. This effect is advantageous especially in gene development analysis.

The present invention is not limited to the above-described embodiment.

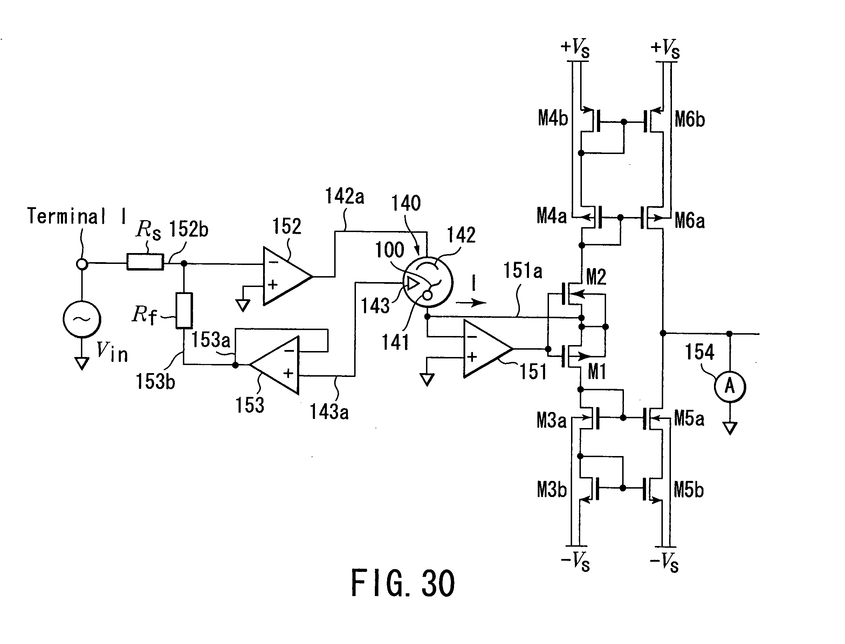

The configuration of the module 150 shown in FIG. 5 is merely one example. For example, as shown in FIG. 30, a cascade current mirror may also be used in which current mirrors are connected in a cascade topology.

In FIG. 30, the configuration common to that of FIG. 5 is denoted with the same reference symbols, and detailed description is omitted. As shown in FIG. 30, the source of a transistor M3a is connected to the drain and gate of a transistor M3b, and the gate of a transistor M5b. The bulk of the transistor M3a and the source of the transistor M3b are connected to the negative voltage source $-Vs$. The source of a transistor M5a is connected to the drain of the transistor M5b, and the bulk of the transistor M5a and the source of the transistor M5b are connected to the negative voltage source $-Vs$.

The current amplification factor of the current mirror in the first stage including the transistors M3a and M5a is set to be equal to that of the current mirror in the second stage including the transistors M3b and M5b.

Transistors M4a, M4b, M6a, M6b form the same topologies as those of transistors M3a, M3b, M5a, M5b except in the positive/negative reverse characteristics.

In the circuit shown in FIG. 5, the precision of the current mirror is not expected to be improved well because of a channel modulation effect of the transistor in some case. In this case, by the use of the cascade current mirror shown in FIG. 30, the precision of the current detection can be improved.

In the above-described embodiment, the nucleic acid quantitative analysis apparatus and analysis method in which the quantitative analysis of the nucleic acid is performed have been described, but the present invention is not limited to this. The object of the quantitative analysis is not limited to the nucleic acid, and the material having any base arrangement whose presence/absence can be measured by the hybridization reaction is an object. Therefore, the present invention may be established as a base arrangement quantitative analysis apparatus and analysis method in which the quantitative analysis of a predetermined base arrangement is performed.

Therefore, the nucleic acid detection chip 12 shown in FIG. 2 may be replaced with a chip for base arrangement detection, for detecting not only the nucleic acid but also a broad base arrangement, to which the present invention is applicable. The chip configuration shown in FIG. 2 is merely one example, and the electrodes are not linearly arranged, and the chip may be replaced with any nucleic acid detection chip such as the arrayed arranged chip, to which the present invention is applicable.

Moreover, the module 135 including the three-electrode system 140 shown in FIG. 5 may be used not only as the nucleic acid concentration quantitative analysis apparatus but also broadly as an electrolysis apparatus.

Furthermore, the case where the program for executing the function of the present invention is incorporated in the processing unit 113, and the function of the present invention is executed by the program has been described. However, for example, a computer readable recording medium in which the program is recorded is read from a recording medium reader (not shown) connected to the processing unit 113, and the processing unit 113 may also be allowed to execute the function.

(Second Embodiment)

A second embodiment relates to a modification of the first embodiment. In the present embodiment, the influence of a background current is reduced.

FIG. 32 is an explanatory view of a problem by the background current. For the currents having the saturated level and background level, normalized currents are compared and described with respect to five examples of electrode diameters of 20, 50, 100, 200, and 500 μm.

For the current components contained in the background current, it can be confirmed that the component proportional to the electrode area is relatively small as compared with the component proportional to a circumferential length of the electrode. In this case, when the area of the electrode is reduced, the signal current having a strong tendency to be proportional to the area of the electrode is relatively small, and the precision of measurement declines. This is because most of the dynamic range of the circuit to measure the signals is occupied by the background components.

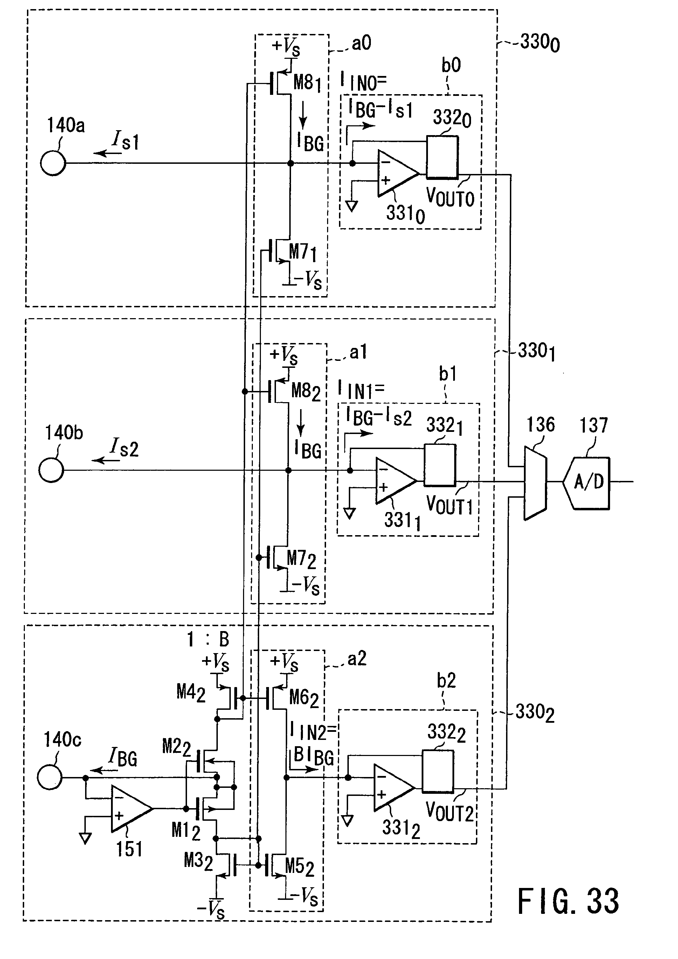

A circuit configuration of the module for solving the problem is shown in FIG. 33. The signal outputs of modules $330_0$, $330_1$, $330_2$ including three-electrode systems $140_0$, $140_1$, $140_2$ having the equal sensor area, that is, the equal area of the working electrode are connected to the selector 136. The module $330_2$ is a module for background current detection for the modules $330_0$, $330_1$. The configurations other than those of the modules $330_0$, $330_1$, $330_2$ are common to those described in the first embodiment, and therefore the detailed description is omitted.

The current detection circuit including the current mirror for positive/negative electrode including six transistors $M1_2$ to $M6_2$ in the module $330_2$ is common to that of FIG. 5 or 13, and the current amplification ratio is 1:B. In FIG. 33, the counter electrode 142 and reference electrode 143 in the three-electrode systems $140_0$, $140_1$, $140_2$ with the voltage application circuits, are omitted.

Moreover, in each of the module $330_0$, $330_1$, and $330_2$, the output node of current mirror for positive/negative current is connected to the inverting input terminal of an operational amplifier $331_0$, $331_1$, and $331_2$, and the noninverting input terminal of the amplifier $331_0$, $331_1$, and $331_2$ are grounded. Moreover, the inverting input terminal and the output of the operational amplifier $331_0$, $331_1$, and $331_2$ are connected to a circuit $332_0$, $332_1$, and $332_2$ for performing the current-to-voltage conversion or current amplification, and the output of the amplifier $331_0$, $331_1$, and $331_2$ are connected to the selector 136.

The gate of the transistor $M4_2$ is connected in parallel with not only the gate of the transistor $M6_2$ but also the gate of a transistor M81 of the module $330_0$, and the gate of a transistor M82 of the module $330_1$. The gate of the transistor $M3_2$ is connected in parallel with not only the gate of the transistor $M5_2$ but also the gate of a transistor M71 of the module $330_0$ and the gate of a transistor M72 of the module $330_1$. The ratio of gate width of each MOSFET $M3_2$,

$M4_2$, M72, M82, M71 to M81 $M5_2$, $M6_2$ are set to be 1:B.

At the same time a signal current $I_{S1}$ flows in a three-electrode system $140_a$ in the module $330_0$, a signal current $I_{S2}$ flows in a three-electrode system $140_b$ in the module $330_1$, and a background current $I_{BG}$ flows in a three-electrode system $140_c$ in the module $330_2$. At this time, in the module $330_0$, the background current $I_{BG}$ flows from a transistor M81, and a current $I_{BG}-I_{S1}$ flows in a current-to-voltage conversion circuit $b_0$ by an effect of a current mirror formed by transistors $M3_2$, $M4_2$, transistors M71, M81, and transistors M72, M82. Similarly, in the module $330_1$, the background current $I_{BG}$ flows from the transistor M82, and a current $I_{BG}-I_{S2}$ flows in a current-to-voltage conversion circuit $b_1$. A current $BI_{BG}$ flows in a current-to-voltage conversion circuit $b_2$ in the module $330_2$. In this manner, in the module $330_0$, the background current $I_{BG}$ detected in the module $330_2$ is subtracted from the signal current $I_{S1}$ of the three-electrode system $140_0$ and output. In the module $330_1$, the background current $I_{BG}$ detected in the module $330_2$ is subtracted from the signal current $I_{S2}$ of the three-electrode system $140_1$ and output. Moreover, the current-to-voltage conversion is performed in the subsequent stage.

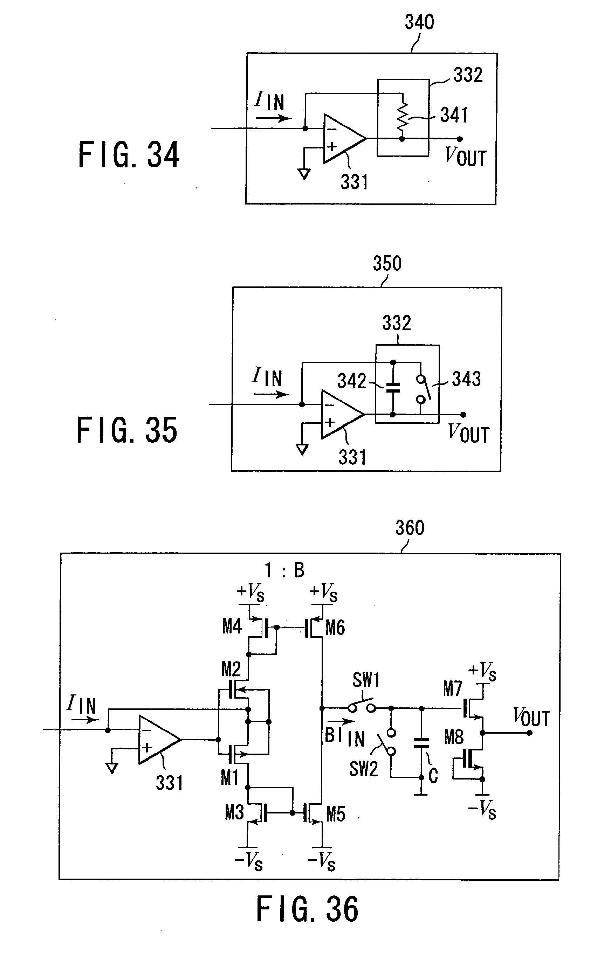

FIGS. 34 to 37 showing the concrete configuration examples of the current-to-voltage conversion circuits $b_0$, $b_1$, and $b_2$. Especially FIGS. 34 and 35 are suitable for a case where an amplification factor B is 1, that is, the current does not have to be amplified in FIG. 33.

FIG. 34 is a diagram showing one example of the current-to-voltage conversion circuit, and a current-to-voltage conversion circuit 340 shown in FIG. 34 is applied to the current-to-voltage conversion circuits $b_0$ to $b_2$. As shown in FIG. 34, the inverting input terminal and output terminal of an operational amplifier 331 are connected via a resistance 341. A voltage $V_{OUT}$ of the output terminal of the operational amplifier 331 is proportional to an input current $I_{IN}$. In the example of the current-to-voltage conversion circuit $b_0$, an output voltage $V_{OUT0}$ of the output terminal of an operational amplifier $331_0$ indicates a value proportional to the input current $I_{S1}-I_{BG}$. In the example of the current-to-voltage conversion circuit $b_1$, an output voltage $V_{OUT1}$ of the output terminal of an operational amplifier $331_1$ indicates a value proportional to the input current $I_{S2}-I_{BG}$. In the example of the current-to-voltage conversion circuit $b_2$, an output voltage $V_{OUT2}$ of the output terminal of an operational amplifier $331_2$ indicates a value proportional to the input current $BI_{BG}$, that is, a value proportional to $I_{BG}$ in case of B = 1.

FIG. 35 is a diagram showing another example of the current-to-voltage conversion circuit, and a current-to-voltage conversion circuit 350 shown in FIG. 35 is applied to the current-to-voltage conversion circuits $b_0$ to $b_2$. As shown in FIG. 35, a switched capacitor including a switch 343 and capacitor 342 is disposed on the inverting input terminal of the operational amplifier 331. A charge flowing into the capacitor 342 from the previous stage is accumulated by the switched capacitor in a state in which the switch 343 is open. When the switch 343 is closed, this charge can be allowed to be discharged. It is to be noted that a principle of the current-to-voltage conversion using the switched capacitor is common to that in the switched capacitor in FIG. 13, and the description is therefore omitted. The voltage $V_{OUT}$ of the output terminal of the operational amplifier 331 is proportional to the input current $I_{IN}$. In the example of the current-to-voltage conversion circuit $b_0$ to which the current-to-voltage conversion circuit 350 shown in FIG. 35 is applied, the output voltage $V_{OUT0}$ of the output terminal of the operational amplifier $331_0$ indicates a value proportional to the input current $I_{S1}-I_{BG}$. In the example of the current-to-voltage conversion circuit $b_1$, the output voltage $V_{OUT1}$ of the output terminal of the operational amplifier $331_1$ indicates a value proportional to the input current $I_{S2}-I_{BG}$. In the example of the current-to-voltage conversion circuit $b_2$, the output voltage $V_{OUT2}$ of the output terminal of the operational amplifier $331_2$ indicates a value proportional to the input current $BI_{BG}$, that is, a value proportional to $I_{BG}$ in case of B = 1.

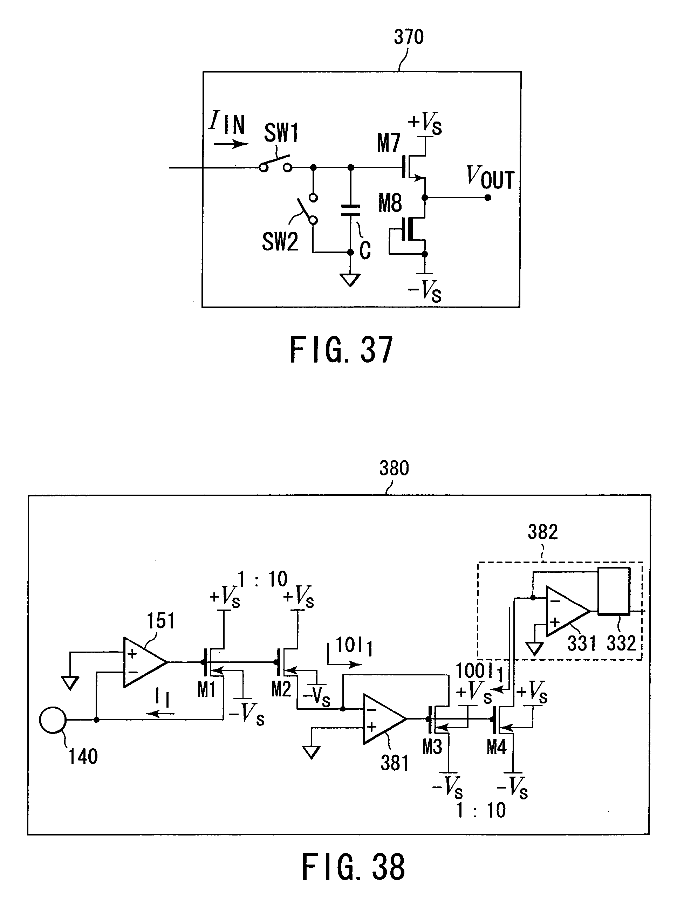

FIGS. 36 and 37 are diagrams showing still further example of the current-to-voltage conversion circuit. A current-to-voltage conversion circuit 360 shown in FIG. 36 further includes a current amplification unit. Therefore, the currents output from the three-electrode systems 140a, 140b, 140c are amplified by B times. By the application of FIG. 36 to the configuration of FIG. 33, a configuration is realized in which a sensor, subtraction section, normalization section, and current-to-voltage conversion section are arranged in order.

The current-to-voltage conversion circuit 360 of FIG. 36 is applied to the current-to-voltage conversion circuits $b_0$, $b_1$, and a current-to-voltage conversion circuit 370 of FIG. 37 is applied to the current-to-voltage conversion circuit $b_2$. It is to be noted that in the configuration in which the circuit shown in FIG. 37 is applied to the current-to-voltage conversion circuit $b_2$, the operational amplifier does not have to be disposed, and the configuration differs from that of the current-to-voltage conversion circuit $b_2$ of FIG. 33 including the operational amplifier $331_2$ and circuit $332_2$.

The current amplification function in FIG. 36 is realized by the current mirror for positive/negative current including the transistors M1 to M6 of FIG. 36, the principle of the current amplification is common to that described with reference to FIG. 13, and therefore the detailed description is omitted. Assuming that the current amplification ratio by the current mirror for positive/negative current is 1:B, a current $BI_{IN}$ flows in the output terminal of the current mirror. Accordingly, the normalization of the detected current is realized.

The gate of the transistor M7 is connected to the output node of the current mirror for positive/negative current via the switch $SW_1$. The source of the transistor M7 is connected to the drain of the depletion mode of N-type MOSFET M8 and the selector 136. The source of the transistor M8 is connected to the gate. This is one of the circuit configurations called the source follower. Needless to say, the buffers may also be used such as the source follower constituted in the other method or the voltage follower. The switch capacitor including the switch $SW_2$ and capacitor C is disposed between the output node of the current mirror for positive/negative current and the transistor M7. The charge flowing via the current mirror is accumulated in the capacitor C by the switched capacitor in the open state of the switch $SW_2$, and can be allowed to be discharged, when the switch $SW_2$ is closed.

Since the method of the open/close control of the switches $SW_1$ and $SW_2$ and the current-to-voltage conversion operation by the method are common to those described with reference to FIG. 13, the detailed description is omitted.

As shown in FIG. 37, the current-to-voltage conversion circuit applied to the circuit $b_2$ has a configuration in which the current mirror and operational amplifier 331 to realize a current amplification function are omitted from the circuit shown in FIG. 36. Since the current-to-voltage conversion operation using the switches $SW_1$ and $SW_2$, capacitor C, and transistors M7 and M8 is common to the operation principle described with reference to FIGS. 36 or 13, the detailed description is omitted. In FIG. 37, since the current mirror is already disposed in the previous stage to realize the current amplification, it is not necessary to dispose the current amplification circuit anew. The output voltage $V_{OUT}$ of the current-to-voltage conversion circuit of FIG. 37 indicates a value proportional to the input current $I_{IN}$. When the current-to-voltage conversion circuit shown in FIG. 37 is applied to $b_2$, the output voltage $V_{OUT2}$, which is proportional to the current B times as large as the current $I_{BG}$ of a three-electrode system $140_c$, is given by the current-to-voltage conversion following the current amplification of B times.

As described above in the example of FIG. 33, the subtraction circuit is preferably used before performing the current-to-voltage conversion in the case of a small area of the electrode. That is possible even if any of circuits shown in FIGS. 34 to 37 is used. In this example, the signal from the three-electrode system $140_c$ to detect the background level is subtracted from the signals from the three-electrode systems $140_a$, $140_b$ to sense the nucleic acid detection level. Simultaneously, the signal from the original background level sensor is also output. Moreover, the current-to-voltage conversion circuit is disposed in the subsequent stage of the subtraction circuit. Accordingly, only the signal components having a strong tendency to be proportional to the electrode area can be measured using the whole dynamic ranges of the current-to-voltage conversion circuit and A/D converter.

It is to be noted that portions of circuits $a_0$ to $a_2$ surrounded with broken lines are preferably disposed in the vicinity in order to reduce mismatch among the devices.

As described above, in accordance with the present embodiment, even when the sensor having a small electrode area is used to perform the quantitative analysis, the background current is subtracted before the current-to-voltage conversion. Accordingly the analysis is possible such that the influence of the background current is relatively reduced as compared with the current which is to be measured and which is proportional to the electrode area. As a result, mismatch of the measured value between the electrode areas is reduced, and high-precision quantitative analysis can be realized.

(Third Embodiment)

A third embodiment relates to a modification of the first embodiment. The present embodiment relates to another embodiment of the module including the current amplification circuit. The present embodiment relates to a configuration obtained by simplification of the current amplification circuit described in the first and second embodiments.

In the first and second embodiments, as shown in FIGS. 5, 13, 33, 36, transistors in the feedback circuit which control a sensor electrode potential to the reference level, and transistors which actually perform the copy operation have been implemented actually in different function blocks for current copy/amplification process. For example, in the example of FIG. 5, the transistors M1 and M2 are implemented in the feedback circuit which controls the sensor electrode potential to the reference level, and the transistors for current copy include a pair of M4, M6, and a pair of M3, M5.

One example of the configuration of the module including the current amplification circuit of the present embodiment is shown in FIG. 38. In a module 380 of FIG. 38, the output terminal of the operational amplifier 151 is connected to the gates of the NMOS transistors M1 and M2. The function realized by the transistors M2 and M4 in FIG. 5 is summarized in the transistor M1 in FIG. 38.

The working electrode of the three-electrode system 140 is connected to the drain of the transistor M1 and the inverting input terminal of the operational amplifier 151. The noninverting input terminal of the operational amplifier 151 is grounded. The source of the transistor M1 is connected to the positive voltage source of $+Vs$, and the bulk of the transistor is connected to the negative voltage source of $-Vs$. The source of the transistor M2 is connected to the positive voltage source of $+Vs$, and the bulk of the transistor is connected to the negative voltage source of $-Vs$. A current amplification ratio realized by the transistors M1 and M2 is 1:10.

The drain of the transistor M2 is connected to the inverting input terminal of a operational amplifier 381 and the drain of the PMOS transistor M3. The output terminal of the operational amplifier 381 is connected to the gates of the PMOS transistors M3 and M4. The bulk of the PMOS transistor M4 is connected to the positive voltage source of

+$Vs$, the source is connected to the negative voltage source of -$Vs$, and the drain is connected to a current-to-voltage conversion circuit 382. A current amplification ratio realized by the transistors M3 and M4 is 1:10.

The current-to-voltage conversion circuit 382 includes a combination of the operational amplifier 331 and circuit 332, and concretely any of the current-to-voltage conversion circuits described with reference to FIGS. 34 to 36 is applied.

In the example of FIG. 38, a polarity of the current to be input is limited to a single polarity of either the oxidation current or the reduction current. However, when the polarity of the current caused by the electrochemical reaction of the intercalating agent is obtained beforehand, it is also possible to prevent an offset current caused by the mismatch between the PMOS transistor and the NMOS transistor from flowing.

Moreover, an ammeter similar to that of FIG. 5 may also be used in place of the current-to-voltage conversion circuit 382, but an input node is preferably virtually grounded by the operational amplifier 331. In this circuit, when the both pairs of the NMOS transistor and the PMOS transistor perform an amplification of ten times, an amplification of 100 times is possible.

The pair of transistors M1 and M2 amplify a current $I_1$ flowing in the transistor M1 by ten times, and a current $10I_1$ flows in the transistor M2. The pair of transistors M3 and M4 amplify the current $10I_1$ which has flown in the transistor M3 from the transistor M2 by ten times, and a current $100I_1$ flows in the transistor M4.

As described above, when the current amplification circuit of the present embodiment is used, the offset current caused by the mismatch between the PMOS transistor and the NMOS transistor can be prevented .

(Fourth Embodiment)

A fourth embodiment relates to a modification of the first embodiment. The present embodiment relates to normalization of the current using the current amplification circuit described in the third embodiment.

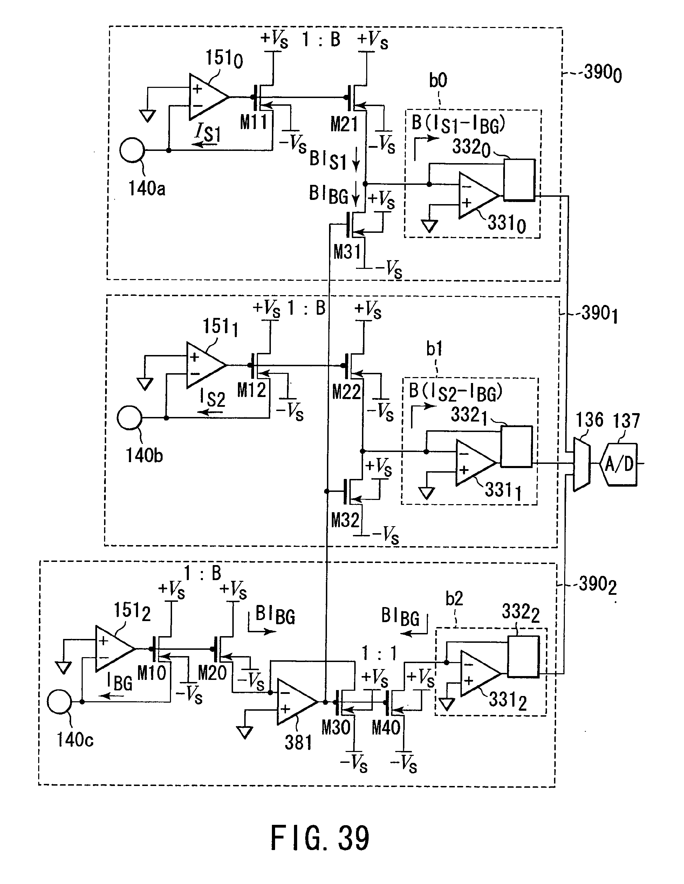

FIG. 39 is a diagram showing one example of the circuit configuration of the module of the present embodiment. The signal outputs of modules $390_0$, $390_1$, $390_2$ including the three-electrode systems $140_a$, $140_b$, $140_c$ having the equal sensor area, that is, the equal area of the working electrode are connected to the selector 136. The module $390_2$ is the module for background current detection for the modules $390_0$, $390_1$. The configurations other than those of the modules $390_0$, $390_1$, $390_2$ are common to those described in the first embodiment, and therefore the detailed description is omitted.

The configuration of the module $390_2$ is common to that of the module 380 of FIG. 38, and the operation is the same. Different respects lie in that the current amplification ratio of transistors M10 and M20 is 1:B, and the current amplification ratio of transistors M30 and M40 is 1:1.

In the module $390_0$, the working electrode of the three-electrode system $140_a$ is connected to the inverting input terminal of an operational amplifier $151_0$ and the drain of an NMOS transistor M11. The noninverting input terminal of the operational amplifier $151_0$ is grounded. The source of the transistor M11 is connected to the positive voltage source of +$Vs$, and the bulk is connected to the negative voltage source of -$Vs$. The source of a transistor M21 is connected to the positive voltage source of +$Vs$, and the bulk is connected to the negative voltage source of - $Vs$.

The drain of the transistor M21 is connected to the inverting input terminal of the operational amplifier $331_0$, circuit $332_0$, and drain of a transistor M31. The source of the transistor M31 is connected to the negative voltage source of - $Vs$, and the bulk is connected to the positive voltage source of +$Vs$. The gate of the transistor M31 is connected to the output terminal of the operational amplifier 381 of the module $390_2$ for background current. Accordingly, the current $BI_{BG}$ amplified by B times in the module $390_2$ is taken out by the transistor M31. The current $I_{S1}$ flowing in the working electrode of the three-electrode system $140_a$ is amplified by B times on a transistor M21 side to indicate $BI_{S1}$. Therefore, the current flowing in the current-to-voltage conversion circuit $b_0$ is $B(I_{S1}-I_{BG})$. Moreover, the voltage proportional to the current $B(I_{S1}-I_{BG})$ is taken out via the output terminal of the current-to-voltage conversion circuit $b_0$.

In the module $390_1$, the working electrode of the three-electrode system $140_b$ is connected to the inverting input terminal of an operational amplifier $151_1$ and the drain of the NMOS transistor M12. The noninverting input terminal of the operational amplifier $151_1$ is grounded. The source of the transistor M12 is connected to the positive voltage source of +$Vs$, and the bulk is connected to the negative voltage source of -$Vs$. The source of the transistor M22 is connected to the positive voltage source of +$Vs$, and the bulk is connected to the negative voltage source of -$Vs$.

The drain of the transistor M22 is connected to the inverting input terminal of an operational amplifier $331_1$, circuit $332_1$, and drain of a transistor M32. The source of the transistor M32 is connected to the negative voltage source of -$Vs$, and the bulk is connected to the positive voltage source of +$Vs$. The gate of the transistor M32 is connected to the output terminal of the operational amplifier 381 of the module $390_2$ for background current. Accordingly, the current $BI_{BG}$ amplified by B times in the module $390_2$ is taken out via the transistor M32. The current $I_{S2}$ flowing in the working electrode of the three-electrode system $140_b$ is amplified by B times on a transistor M22 side to indicate $BI_{S2}$. Therefore, the current flowing in the current-to-voltage conversion circuit $b_1$ is $B(I_{S2}-I_{BG})$. Moreover, the voltage proportional to the current $B(I_{S2}-I_{BG})$ is taken out via the output terminal of the current-to-voltage conversion circuit $b_1$.

It is to be noted that the concrete configurations of the current-to-voltage conversion circuits $b_0$, $b_1$, and $b_2$ are similar to those of the example of FIG. 33 in that the circuits shown in FIGS. 34 to 37 are applied.

It is to be noted that the current amplification factors of the modules $390_0$, $390_1$, $390_2$ are B , but when a plurality of different current amplification factors are substituted to a combination of the modules $390_0$, $390_1$, $390_2$ in accordance with the electrode area, the normalization is possible. That is, the current amplification factor B of a first set of the modules $390_0$, $390_1$, $390_2$ having the electrode area $A_0$ of the working electrode is 1, the factor of a second set of the modules $390_0$, $390_1$, $390_2$ having the electrode area $\alpha A_0$ of the working electrode is $1/\alpha$, and the factor of a third combination of the modules $390_0$, $390_1$, $390_2$ having the electrode area $\alpha^2 A_0$ of the working electrode is $1/\alpha^2$, ... By this setting, the module including the subtraction, normalization, and current-to-voltage conversion can be realized. It is to be noted that in the example of FIG. 39, the subtraction is performed after first performing the current amplification in the circuit.

As described above, according to the present embodiment, the offset current caused by the mismatch between the PMOS transistor and the NMOS transistor can be prevented, and the module which performs the normalization, current amplification, subtraction and current-to-voltage conversion, can be realized.

(Fifth Embodiment)

A fifth embodiment relates to a modification of the first embodiment. In the present embodiment, the normalization in accordance with the electrode area is performed using not only the current mirror but also the capacitor.

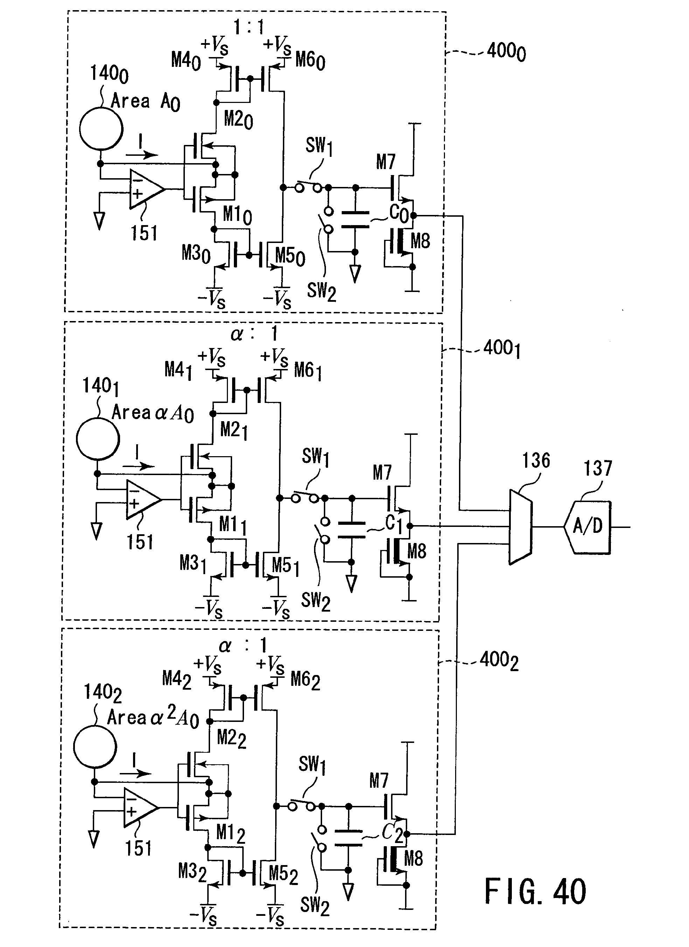

FIG. 40 is a diagram showing one example of the configuration of the module of the present embodiment. Modules $400_0$ to $400_2$ of FIG. 40 are substantially common to the modules $135_0$ to $135_2$ of FIG. 13, the common configuration is denoted with the same reference symbols, and the detailed description is omitted. In FIG. 40, a capacitor $C_0$ of the module $400_0$, a capacitor $C_1$ of the module $400_1$, and a capacitor $C_2$ of the module $400_2$ have different capacitances.

When a sufficient amplification gain of the current mirror included in the normalization circuit cannot be taken because of restrictions on device dimensions, this can be compensated by the reduction of the capacitance of the capacitor in the current-to-voltage conversion circuit. When the working electrode having an electrode area of $A_x = \alpha_x A_0$ is connected to the current mirror having a current amplification factor of $B_x$ times followed by the integrator circuit including a capacitance $C_x$, parameters are determined so as to satisfy the following equation:

$$A_x B_x / C_x = \text{constant.}$$

A list of parameters of the module $400_0$, $400_1$, $400_2$, and so forth given based on a determination method of the parameters is shown in Table 2.

Table 2

| Circuit #X | Area $A_x$ | Current amplification factor $B_x$ | Capacitance $C_x$ | $A_x B_x / C_x$ |
|:---:|:---:|:---:|:---:|:---:|
| 0 | $A_0$ | 1 | $C_0$ | $A_0/C_0$ |
| 1 | $\alpha A_0$ | $\alpha^{-1}$ | $C_0$ | $A_0/C_0$ |
| 2 | $\alpha^2 A_0$ | $\alpha^{-1}$ | $\alpha C_0$ | $A_0/C_0$ |
| 3 | $\alpha^3 A_0$ | $\alpha^{-1}$ | $\alpha^2 C_0$ | $A_0/C_0$ |
| 4 | $\alpha^4 A_0$ | $\alpha^{-2}$ | $\alpha^2 C_0$ | $A_0/C_0$ |

Here, the current amplification factor of $\alpha^{-2}$ times is assumed to be a limit because of design restrictions on preparing circuits whose device sizes are increased while the sensor area size is decreased every $\alpha$ times as shown in Table 2. In this case, assuming that the capacitance is $\alpha$ times or $\alpha^2$ times, any module can function as a module including the normalization circuit in which $A_x B_x / C_x = A_0/C_0$. It is to be noted that $0 < \alpha < 1$.

As described above, in accordance with the present embodiment, even when a sufficient amplification gain of the current mirror is not realized because of the restrictions on the device dimension, the quantitative analysis of the nucleic acid concentration in a broad dynamic range is possible.

(Sixth Embodiment)

A sixth embodiment relates to a modification of the first embodiment. The present embodiment relates to the configuration of a circuit which compensates for a phase shift of the circuit.

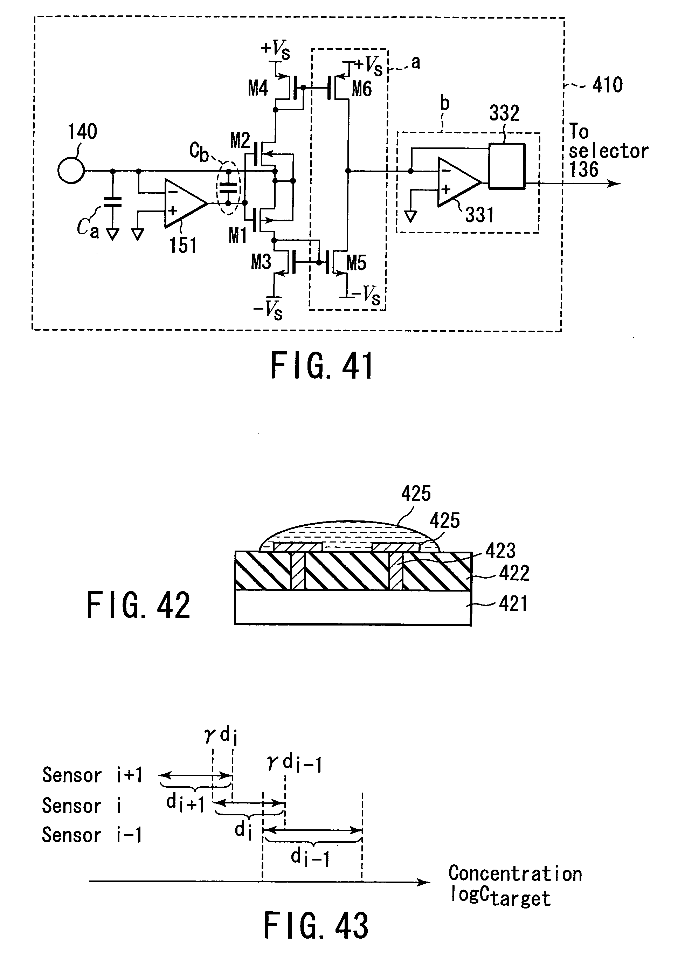

FIG. 41 is a diagram showing one example of the configuration of a module 410 according to the present embodiment. The configuration of the module 410 is substantially common to that of the module $330_2$ of FIG. 33, the common configuration is denoted with common reference numerals, and the detailed description is omitted. That is, the respective configurations of the three-electrode system $140_2$ of the module $330_2$, operational amplifier 151, current mirror for positive/negative current including the transistors $M1_2$ to $M6_2$, and current-to-voltage conversion circuit $b_2$ correspond to the three-electrode system 140 of the module 410, the operational amplifier 151, the current mirror for positive/negative current including the transistors M1 to M6, and the current-to-voltage conversion circuit b. Differences lie in that the capacitor $C_a$ is connected between the three-electrode system 140 and the inverting input terminal of the operational amplifier 151, and a capacitor $C$b is connected between the output terminal of the operational amplifier 151 and the inverting input terminal. Here, the capacitor $C_a$ indicates an equivalent capacitor caused by a solution to be analyzed, and the capacitor $C_b$ functions as a capacitor for phase compensation.

In the present circuit which performs the electrochemical measurement, because the capacity value of the capacitor $C_a$ increases very much in some case, the large capacitor $C_b$ is sometimes required to appropriately perform phase compensation. FIG. 42 is a diagram showing one example of the configuration of the capacitor $C_b$. As shown in FIG. 42, an insulating layer 422 is formed on a substrate 421. Two contact plugs 423 are buried/formed in contact holes disposed in the insulating layer 422, and two metal layers 424 are selectively formed so as to cover the contact plugs 423 on the surface of the insulating layer 422. The metal layers 424 are electrically connected to the substrate 421 via the contact plugs 423. Various integrated circuits are formed on the substrate 421. Moreover, two metal layer 424 surfaces are immersed in a solution 425.

As described above, the capacitor having a large capacity required for compensating for the phase shift in an electrochemical analyzer using the integrated circuit is realized by an electric double layer device generated in a solvent for actually performing the electrolysis. Two metal layers 424 in the figure are used as the electrodes, and immersed in the solution in which the electrochemical measurement is actually performed. That is, one of the metal layers 424 is connected to the inverting input terminal of the operational amplifier 151 via the contact plug 423, and the other metal layer 424 is connected to the output terminal of the operational amplifier 151 via the other contact plug 423. Accordingly, the capacitor equivalent to a large capacity generated in the vicinity of the sensor can easily be realized.

As described above, in accordance with the present embodiment, even when it is difficult to realize the capacitor in the integrated circuit, the configuration in the cell can be used to simply realize the capacitor.

(Seventh Embodiment)

A seventh embodiment relates to a modification of the first embodiment. The present embodiment relates to an embodiment in which ranges of measurable concentrations of electrodes which differ with an electrode area are overlapped to optimize the analysis.

A minimum nucleic acid concentration to impart a condition on which a nucleic acid sensor outputs a signal having a saturation level is defined as an upper end of the range measurable by the sensor, and similarly a maximum nucleic acid concentration to impart a condition on which a signal having a background level is output is defined as a lower end of the range measurable by the sensor. Here, the measurement ranges of the sensors adjacent to each other preferably overlap with each other.

A method of designing the sensor so as to satisfy this condition will be described hereinafter. It is assumed that the number of probes existing on the sensor surface of an *i*-th large sensor ($i = 1, 2, ..., n$-1) is $N_i$, and a dynamic range $d_i$(dec) [$d_i > 0$] of the sensor is a ratio of the concentration of the measurement range upper end to that of the measurement range lower end. Also assuming that a ratio of an upper end to a lower end of a region in which the measurement range of the i-th sensor overlaps with that of a sensor i-1 having an area larger than that of the i-th sensor by one step is d$_{i-1,i}$(dec) and that a ratio d$_{i-1,i}$/d$_{i-1}$ of this d$_{i-1,i}$ to d$_{i-1}$ is a range overlap factor $\gamma$ ($0 \leq \gamma < 1$) given as an optional parameter to a designer, it is preferable to use the chip including the sensor series which satisfies the following relation between the sensors:

$$10^{d_{i-1}(1-\gamma)} = \frac{N_{i-1}}{N_i} .$$

FIG. 43 is an explanatory view of the overlap factor $\gamma$. As shown in FIG. 43, the dynamic ranges of the sensors i-1, i, i+1 are represented by d$_{i-1}$, d$_i$, d$_{i+1}$. The dynamic ranges d$_i$, d$_{i-1}$ of the sensors i and i-1 overlap with $\gamma$d$_{i-1}$. The dynamic ranges d$_i$, d$_{i+1}$ of the sensors i and i+1 overlap with $\gamma$d$_i$. Here, the overlap factor $\gamma$ is preferably $\gamma \leq 0.85$. Here, it is preferable to use the chip including the sensor series arbitrarily set to d$_1$ = d$_2$ = ... = d$_{n-1}$ = d$_n$ = constant.

Moreover, it is preferable to use the chip whose area ratio is constant on the condition that the number $N_i$ of probes existing on the sensor surface is proportional to the area. That is, when the area of a sensor i is defined as $S_i$, preferably $S_{i+1}/S_i = S_i/S_{i-1} = ... = $ constant. Furthermore, it is preferable to use the chip whose area ratio is constant, especially at 0.05 or more and 0.5 or less. That is, preferably $S_{i+1}/S_i = S_i/S_{i-1} = ... \leq 0.5$. When the area excessively largely changes in the sensor series, the overlap of the dynamic ranges is reduced. Conversely, when the area hardly changes, the overlap of the dynamic ranges is excessively enlarged, a large number of sensors are required to achieve a large dynamic range of the whole sensor series, and the apparatus becomes large-scaled. The condition of the area ratio described herein indicates an appropriate condition in this trade-off.

As described above, in accordance with the present embodiment, when the dynamic ranges by the respective electrode areas are overlapped, the optimum quantitative analysis is possible without any measurement leakage.

(Eighth Embodiment)

An eighth embodiment relates to a modification of the first embodiment. The present embodiment relates to an embodiment of a further detailed apparatus configuration of the quantitative analysis.

In accordance with the configuration described in the first embodiment, the electrodes having different areas are mounted on the same substrate in order to quantitatively analyze the concentration of the nucleic acid. Here, the target nucleic acid solution supplied to these electrodes is preferably separated by walls or cell in such a manner that the nucleic acid is not mutually diffused between the electrodes having different areas. Furthermore, a volume of the separated solution is preferably constant regardless of the electrode area, and the number of electrodes immersed in the partitioned solution is also preferably constant regardless of the electrode area. This configuration of the apparatus is a constitutional feature of the present embodiment. This is because the number of target nucleic acid molecules increases relatively compared to the number of probes and the sensitivity can be improved in a smaller electrode, provided that a sufficiently large reaction time is required.

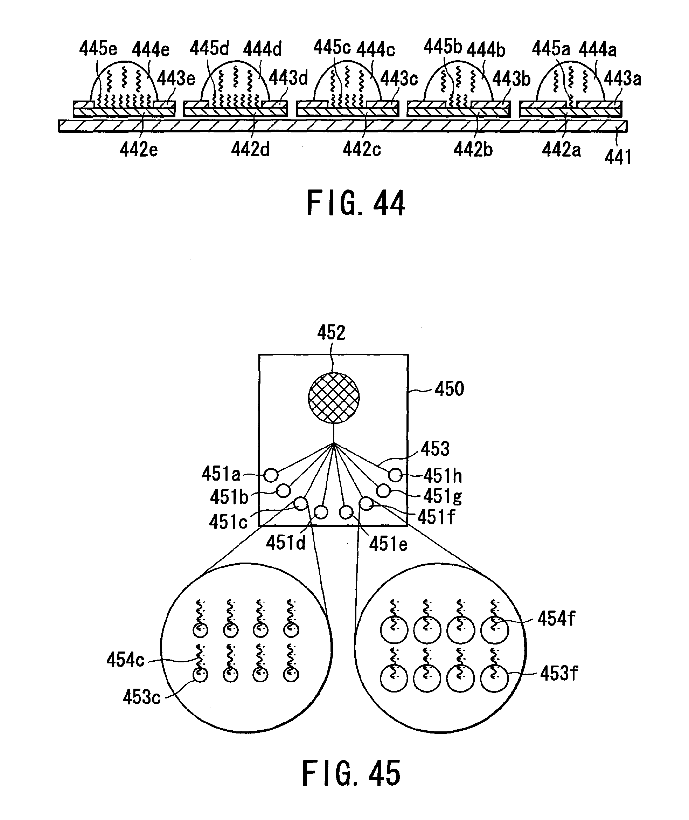

The configuration for realizing the substantial feature of the present embodiment is assumed as shown in FIG. 44 or 45.

FIG. 44 is a diagram showing a main part section of the nucleic acid concentration quantitative analysis chip prepared based on the principle of the measurement apparatus of the present embodiment. As shown in FIG. 44, a plurality of electrodes (working electrodes) 442a to 442e, and passivation films 443a to 443e for selectively exposing parts of the surfaces of the electrodes 442a to 442e are formed on a single substrate 441. A set of the electrode and insulating film form the cell. The passivation films 443a to 443e expose the surfaces of the electrodes 442a to 442e by areas which differ with the cells. This can realize the working electrodes whose surface areas differ with the cells. The cells are immersed in specimen solutions 444a to 444e. Probe nucleic acids 445a to 445e are immobilized on the electrodes 442a to 442e. When these probe nucleic acids 445a to 445e react with the target nucleic acids in the specimen solutions 444a to 444e, the quantitative analysis of the target nucleic acid concentration is possible. The specimen solutions 444a to 444e are independently spotted in the equal volume though no explicit partitions are given. This effectuates a configuration in which the nucleic acid is not diffused mutually among the electrodes having different areas. The volumes of the specimen solutions 444a to 444e separated for each cell are substantially constant regardless of the electrode area, and the number of electrodes immersed in the divided specimen solutions 444a to 444e is also constant regardless of the electrode area.

FIG. 45 is a schematic diagram showing another configuration. As shown in FIG. 45, a plurality of cells s 451a to 451h separated from one another and having the equal volume are disposed on a substrate 450. These cells s 451a to 451h are connected to one target nucleic acid injection port 452 via channels 453. As one example, in FIG. 45, the cells s 451c and 451h are enlarged and shown. A plurality of electrodes 453c having an equal small area are arranged in the cell 451c, and a probe nucleic acid 454c is immobilized on each electrode. A plurality of electrodes 453h having an equal large area are arranged in the cell 451h, and a probe nucleic acid 454h is immobilized on each electrode.

The principle of the present embodiment will be described in more detail from viewpoints of concentration reduction of a quantifiable nucleic acid concentration and extension of a quantifiable nucleic acid concentration range.

A detectable detection object nucleic acid concentration range will hereinafter be described in a nucleic acid detection method in which the nucleic acid probe is immobilized on the substrate surface and hybridization with a detection object nucleic acid is used.

The range of nucleic acid concentration is synonymous with the range of the number of the nucleic acid molecules when an amount of solution for use in detection is constant. In the present embodiment, the nucleic acid concentration range is considered on the basis of the nucleic acid molecule.

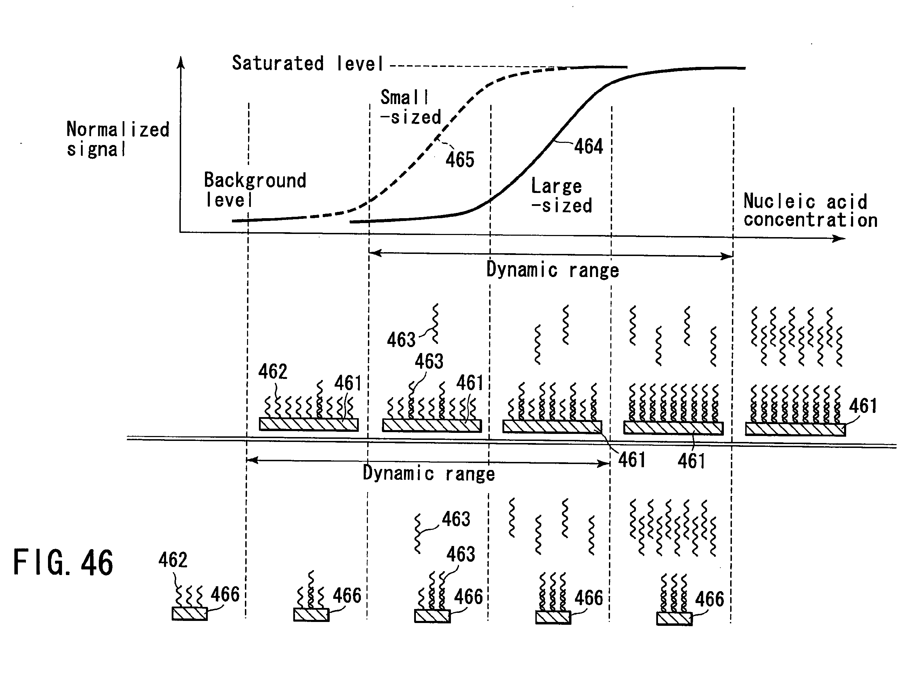

FIG. 46 is an explanatory view of the nucleic acid concentration range of a detectable detection target nucleic acid. In FIG. 46, a graph in the top section of the figure shows a relation between a nucleic acid concentration, that is, the number of nucleic acid molecules contained in a solution having a certain volume, and a normalized signal obtained by normalizing the signal per unit area. Schematics in the middle section of the figure shows the reaction of a probe nucleic acid 462 immobilized on an electrode 461 having a large area to a target nucleic acid 463. Schematics in the bottom section of the figure shows the reaction of the probe nucleic acid 462 immobilized on an electrode 466 having a small area to the target nucleic acid 463. The graph 464 shows a relation between a target nucleic acid concentration expected to be observed on a large-area electrode 461, shown in the middle section, and a normalized obtained signal amount, and the graph 465 shows a relation between a target nucleic acid concentration expected to be observed on a small-area electrode 466, shown in the bottom section, and the normalized obtained signal amount.

As seen from FIG. 46, an upper limit of quantifiable number of nucleic acid molecule is determined by the number of the nucleic acid probe molecule immobilized in a nucleic-acid-probe-immobilized region. A state in which all the nucleic acid probes cause the hybridization with target nucleic acid molecules indicates a quantitative upper limit. The number of nucleic acid probe molecule is determined by the area of the nucleic-acid-probe-immobilized region and the immobilized density of nucleic acid probes. It is possible to set the immobilized density by several factors, but the density is usually set so as to maximize the number of probe molecules that can contribute the hybridization. It is undesirable that the density is excessively large or small. Therefore, when the immobilized density of nucleic acid probes is set to a certain numeric value, the number of immobilized nucleic acid probes is determined by the nucleic-acid-probe-immobilized region area. That is, the upper limit of the quantifiable nucleic acid concentration range is determined by the nucleic-acid-probe-immobilized region area.

On the other hand, the lower limit of quantifiable nucleic acid concentration range is influenced by the fluctuation or noise of the detection signal, and the background signal. However, it can usually be described in the form of 1/10, 1/100, 1/1000 and the like based on the quantifiable upper-limit concentration.

Therefore, in the above-described setting, both the upper and lower limits of the quantifiable nucleic acid concentration range are proportional to the nucleic-acid-probe-immobilized region area.

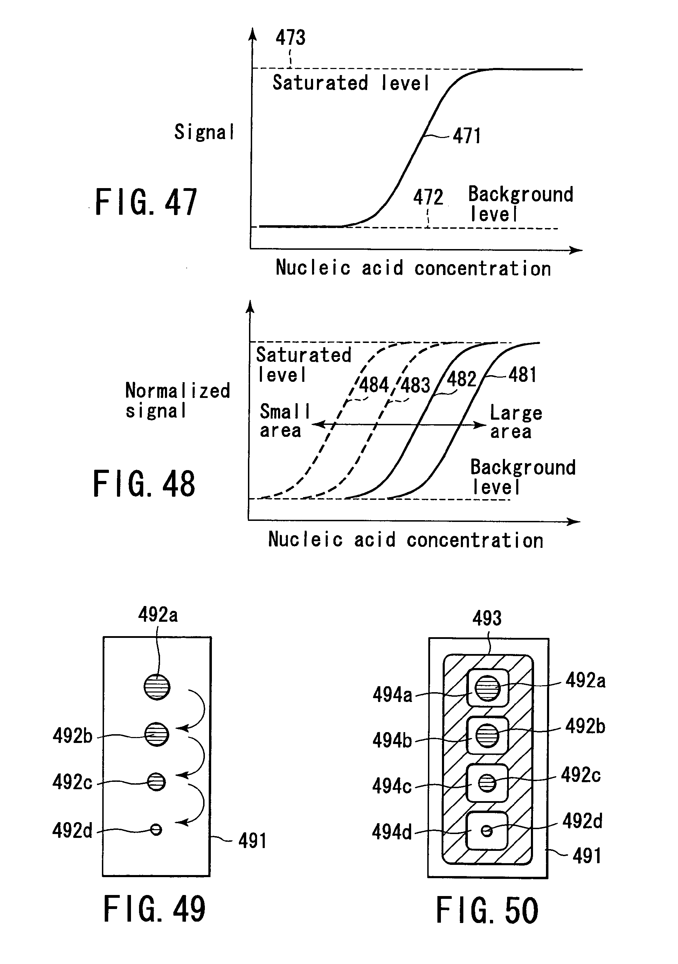

FIG. 47 shows the graph shown in the top section of FIG. 46 in further detail. A graph 471 of FIG. 47 shows a range between a background level 472 and a saturated level 472. The graph 471 is saturated at the background level 472, and a signal amount decreases in the quantifiable concentration range. On the other hand, for the graph 471, the signal becomes constant again in a range which is not more than the quantifiable concentration.

FIG. 48 is a diagram showing a graph example in which the area of nucleic-acid-probe-immobilized region is varied. It is shown that a range in which the signal amount changes, namely a range in which the concentration can be evaluated, changes from the graphs 481 of a larger area to 484 of a smaller area.

Two problems: (1) to shift lower the quantifiable range in nucleic acid concentration domain; and (2) to extend the quantifiable nucleic acid concentration range, will be described using the above-described properties.

(1) Lower Shift of Quantifiable Nucleic Acid Concentration Range

Both the upper and lower limits of the quantifiable nucleic acid concentration range are proportional to the area of the nucleic-acid-probe-immobilized region. Based on this property, the device with a nucleic-acid-probe-immobilized region of small area is utilized. Accordingly, for example, when the area is reduced by one-hundredth, the concentration range shifts two decades. When the area is reduced by ten-thousandth, the concentration range shifts four decades. In this manner, the concentration reduction of the quantifiable nucleic acid concentration can be realized.

(2) Extension of Quantifiable Nucleic Acid Concentration Range

It is supposed that the lower limit of the quantifiable nucleic acid concentration range is 1/100 of the upper-limit concentration. That is, it is assumed that the quantifiable nucleic acid concentration range is two decades. Both the upper and lower limits of the quantifiable nucleic acid concentration range are proportional to the nucleic-acid-probe-immobilized region area. When this is used, the nucleic-acid-probe-immobilized region area decreases by one-hundredth, and the quantifiable nucleic acid concentration range shifts lower by tow decades. Conversely, when the area increases by one-hundred times, the concentration range shifts higher by two decades.

FIGS. 49 and 50 are schematic diagrams of a configuration for extending the nucleic acid concentration range. As shown in FIG. 49, nucleic-acid-probe-immobilized regions 492a to 492d different from one another in area are formed on a substrate 491. The areas of the respective nucleic-acid-probe-immobilized regions 492a to 492d sequentially vary every one-hundredth. The nucleic-acid-probe-immobilized region is determined by the electrode on which the nucleic acid probe is immobilized and the like. As shown in FIG. 50, a sample holding frame 493 is disposed so as to surround each of these nucleic-acid-probe-immobilized regions 492a to 492d up to a predetermined height from the substrate 491. Cell regions 494a to 494d are defined by holes disposed in this sample holding frame 493. These cell regions 494a to 494d have an equal sectional area and height, that is, an equal capacity.

As shown in FIGS. 49 and 50, a device is formed capable of allowing each constant amount of a detection object nucleic acid containing sample to react with respect to each of the nucleic-acid-probe-immobilized regions 492a to 492d whose areas sequentially vary every one-hundredth. Accordingly, quantification is possible in any concentration range. Even when a sample having an unclear concentration is quantified, any of the nucleic-acid-probe-immobilized regions 492a to 492d fits the quantifiable nucleic acid concentration range.

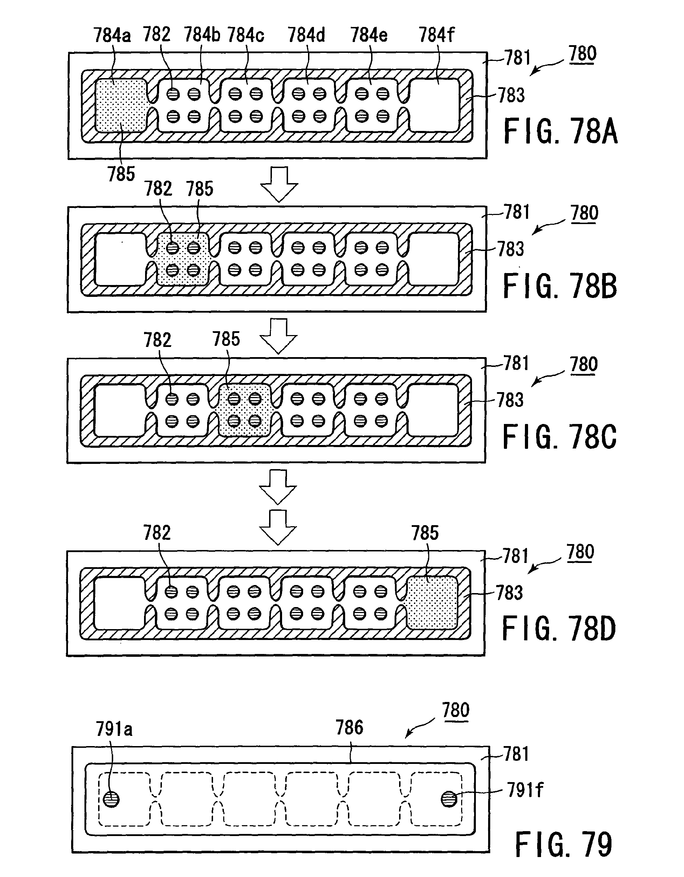

Another configuration for realizing the quantitative analysis of the nucleic acid concentration is shown in FIGS. 78A to 78D. Nucleic-acid-probe-immobilized regions 782 having the equal area are arranged and formed every plurality (every four in FIGS. 78A to 78D) on a substrate 781 having an elongated shape. Moreover, a sample holding frame 783 having the elongated shape is formed to surround these nucleic-acid-probe-immobilized regions 782. The sample holding frame 783 separates the region on the substrate into a plurality of regions, and mutually connects adjacent separated regions via an elongated region. Accordingly, a plurality of cell regions 784a to 784f having the equal area and height, that is, the equal capacity, and channels for connecting the cell regions 784a to 784f to one another can be formed. It is to be noted that the cell regions 784a and 784f are chambers for introducing or discharging the sample, and the nucleic-acid-probe-immobilized region 782 is not disposed. FIG. 79 is a diagram showing that the upper surfaces of the cell regions 784a to 784f are covered with a sample holding frame lid 786. The sample holding frame lid 786 is supported and fixed onto the sample holding frame 783 to function as a lid which covers the cell upper surface. As shown in FIG. 79, sample injection ports 791a and 791f are disposed in accordance with the cell regions 784a and 784f on the opposite ends.

In this manner, an apparatus is formed in which the nucleic-acid-probe-immobilized regions 782 are arranged having the quantifiable concentration range sufficiently lower than that of the specimen nucleic acid that is an object of quantification. Moreover, as sequentially shown in FIGS. 78A to 78D, a specimen solution 785 containing the target nucleic acid is first injected via a sample injection port 791a, and sequentially moved to cell regions 784b, 784c, 784d, 784e, and 784f from 784a. The movement of the specimen solution 785 can be realized, for example, when a pump or the like is used to pressurize the inside of the cell via the sample injection port 791a or to suck a fluid in the cell via the sample injection port 791f. Also in the following embodiment, the solution in the cell is moved on a similar principle.

In the cell region 784b, target nucleic acid molecules cause the hybridization reaction to the nucleic acid probe immobilized on the nucleic-acid-probe-immobilized region 782 and are bonded. Here, since the nucleic-acid-probe-immobilized regions 782 formed on the substrate 781 are in a sufficiently low quantifiable nucleic acid concentration range, the number of target nucleic acid molecules existing in the specimen solution 785 is sufficiently larger than that of immobilized nucleic acid probes. Additionally, the number of target nucleic acid molecules in the solution decreases by the number of hybridized molecules. Similar phenomenon occurs even in second and subsequent nucleic-acid-probe-immobilized regions, and the number of target nucleic acid molecules in the solution gradually decreases. The gradual decrease of the number of target nucleic acid molecules in the solution indicates that the target nucleic acid concentration in the specimen solution decreases. The decrease of the target nucleic acid concentration of the specimen solution indicates that the concentration reaches the quantifiable nucleic acid concentration range of the formed nucleic-acid-probe-immobilized region area in some time. The detection is performed after completely moving all the nucleic-acid-probe-immobilized regions 782. The cell region which is counted from the cell region 784b including the first formed nucleic-acid-probe-immobilized region 782 and in which the signal changes can be analyzed to perform the quantification. The specimen solution 785 which has been treated can be discharged via the sample discharge port 791f.

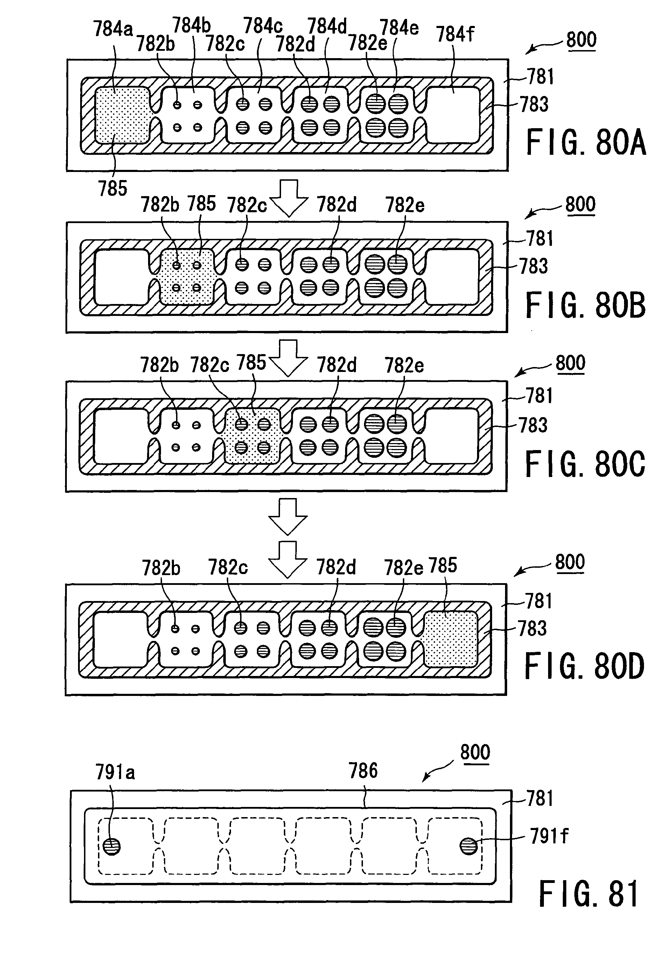

FIGS. 80A to 80D and 81 are drawings showing a chip configuration example for use in a case where the concentration of the target nucleic acid concentration is completely unclear. FIGS. 80A to 80D show top plan views from which the sample holding frame lid 786 is removed, and FIG. 81 shows a top plan view in which the sample holding frame lid 786 is attached. The configuration common to that in FIGS. 78A to 78D and 79 is denoted with the same reference numerals, and the detailed description is omitted. The configurations of nucleic-acid-probe-immobilized regions 782b to 782e are different from those in FIGS. 78A to 78D and 79. In the example of FIGS. 78A to 78D and 79, any of the nucleic-acid-probe-immobilized regions 782 has the equal area. In the example of FIGS. 80A to 80D and 81, a plurality of nucleic-acid-probe-immobilized regions 782b having the equal area are formed in the cell region 784b. Moreover, a plurality of nucleic-acid-probe-immobilized regions 782c having the equal area larger than that of each of the nucleic-acid-probe-immobilized regions 782b are formed in the cell region 784c. Furthermore, a plurality of nucleic-acid-probe-immobilized regions 782d having the equal area larger than that of each of the nucleic-acid-probe-immobilized regions 782c are formed in the cell region 784d. Additionally, a plurality of nucleic-acid-probe-immobilized regions 782e having the equal area larger than that of each of the nucleic-acid-probe-immobilized regions 782d are formed in the cell region 784e. In this manner, the nucleic-acid-probe-immobilized regions having areas which differ with the cells are arranged.

When the rough value of the quantitative target nucleic acid concentration is unclear at all, the configuration shown in FIGS. 80A to 80D and 81 is preferable. In the same manner as in the example of FIGS. 78A to 78D and 79, the specimen solution 785 is sequentially moved from the cell region 784a to 784f through 784b, 784c, 784d and 784e. Moreover, the target nucleic acid in the specimen solution 785 causes the hybridization reaction to the nucleic acid probe in the nucleic-acid-probe-immobilized regions 782b to 782e of the respective cell regions 784b to 784e. In this order, the hybridization reaction takes place in order from the small nucleic-acid-probe-immobilized region to the large region. The target nucleic acid concentration in the specimen solution 785 little decreases in the small-area nucleic-acid-probe-immobilized region. With the increase of the area, the target nucleic acid concentration largely decreases. When this is used, the quantification in a rougher but broader range is possible as compared with the above-described method.

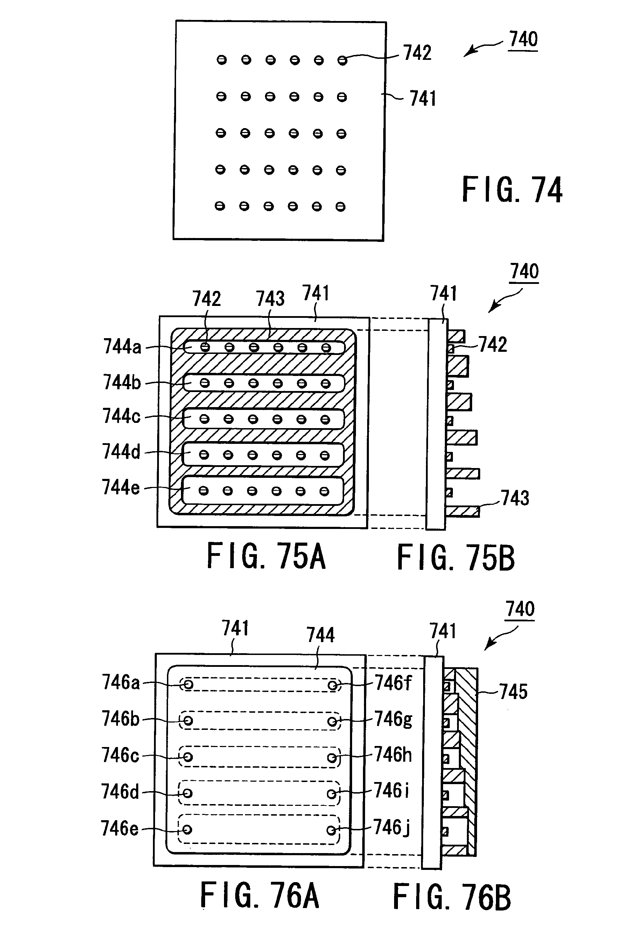

FIGS. 74, 75A, 75B, 76A, and 76B are diagrams showing another chip configuration example. FIG. 74 shows a top plan view from which a sample holding frame 743 and sample holding frame lid 745 are removed, FIGS. 75A and 75B are a top plan view and side view from which the sample holding frame lid 745 is removed, and FIGS. 76A and 76B are a top plan view and side view in which the sample holding frame lid 745 is attached. In the chip example shown in FIGS. 78A to 78D, 79, 80A to 80D, and 81, the method in a case where the constant amount of specimen solution is used. Conversely, in the chip example of FIGS. 74, 75A, 75B, 76A, and 76B, the area of the probe immobilizing region is set to be constant, and a specimen solution amount is varied. Accordingly, the quantitative analysis is possible.

As shown in FIG. 74, nucleic-acid-probe-immobilized regions are formed in a matrix manner on a substrate 741. Moreover, the sample holding frame 743 is formed so as to surround these nucleic-acid-probe-immobilized regions 742, for example, every six regions. A plurality of regions surrounded with the sample holding frame 743 function as cell regions 744a to 744e. The identical number (six regions in FIGS. 75A, 75B) of nucleic-acid-probe-immobilized regions 742 is housed in each of the cell regions 744a to 744e. The cell regions 744a to 744e have different areas and capacities. That is, the cell region 744a has a smallest capacity, and the capacity increases toward 744b, 744c, 744d and 744e. The specimen solution is charged in the respective cell regions 744a to 744e. Therefore, the specimen solution amount changes in accordance with the cell capacity.

When the specimen solution amount is small, the number of nucleic acid molecules included in the solution is small. When the specimen solution amount is large, the number of nucleic acid molecules is large. A detectable nucleic acid molecule range is known from the area of the nucleic-acid-probe-immobilized region 742 formed on the substrate 741. Therefore, it is possible to calculate the concentration of the target nucleic acid from the specimen solution amount used in the reaction in the nucleic-acid-probe-immobilized region 742 in which a detection signal amount changes.

The quantitative analysis method using the chips of FIGS. 74, 75A, 75B, 76A, and 76B can be used together with the above-described methods. That is, the method of varying the cell capacity is usable together with the method of varying the probe immobilizing region area as shown in FIG. 49 or 50, or the method of moving the solution as shown in FIGS. 78A to 78D and 81.

In the method of varying the area of the probe-immobilized region as shown in FIGS. 49 and 50, when a smaller area is formed, a lower concentration is detectable. However, it is technically difficult to form the electrode having the small area in many cases.

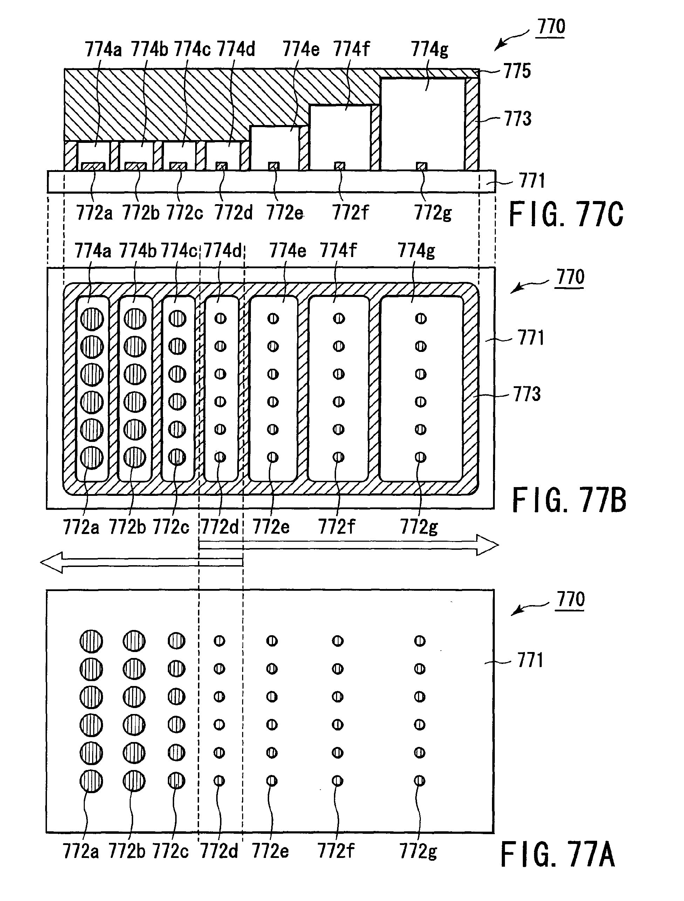

To solve the problem, the chip configuration example shown in FIGS. 77A to 77C is applicable. Nucleic-acid-probe-immobilized regions 772a to 772g are disposed every six regions on a substrate 771. A sample holding frame 773 is formed so as to surround the regions having the equal area among the nucleic-acid-probe-immobilized regions 772a to 772g, and cell regions 774a to 774g are defined.

The cell regions 774a to 774d have the equal cell capacity in the same manner as in the chip example shown in FIG. 50, but the nucleic-acid-probe-immobilized region 772a is largest, and the capacity decreases in order toward 772b, 772c and 772d.

On the other hand, for the cell regions 774d to 774g, in the same manner as in the chip example shown in FIGS. 75A and 75B, the nucleic-acid-probe-immobilized regions 772e to 772g have the equal area, but differ in the cell sectional area and capacity. That is, the cell sectional area and capacity of the cell region 774d are smallest, and the capacity increases toward the cell regions 774e, 774f, 774g.

In this manner, the probe-immobilized regions 772a to 772d are formed on the substrate 771 in order from a large area to an area as small as possible. In this range, the cell capacity, that is, the specimen solution amount is constant. For the probe-immobilized regions 772e to 772g having the area equal to that of the formed probe-immobilized region 772d having the smallest area, the cell sectional area and capacity, that is, the specimen solution amount increase stepwise. By this combination of two methods, the quantifiable range can be enlarged on a low-concentration side. Conversely, when the solution amount gradually decreases with respect to the formed probe-immobilized region having the largest area, it is possible to broaden the quantifiable range on a high-concentration side.

Additionally, since the solution amount has to be increased in order to enlarge the quantifiable range toward lower-concentration range by the method of FIGS. 77A to 77C, a device size increases. To solve the problem, a method including a process of drying the solution is also considered as a similar method. Instead of increasing the solution amount stepwise, the first injected solution is dried, the solution is again injected, and this is repeated. Accordingly, the target nucleic acid condenses , and the number of nucleic acid molecules in the solution increases. Some probe immobilizing regions having the certain area are formed beforehand, and the number of repetitions of the drying and re-injecting is varied stepwise. It is possible to calculate the concentration of the nucleic acid from the number of repetitions in the probe immobilizing region in which the detected signal amount changes.

Next, the chip configuration example embodying the method described herein will be described.

(Configuration Example 1)

Configuration Example 1 shows a chip configuration example in a case where the nucleic acid quantitative analysis is performed by utilizing the device on which the nucleic-acid-probe-immobilized regions having various areas exist.

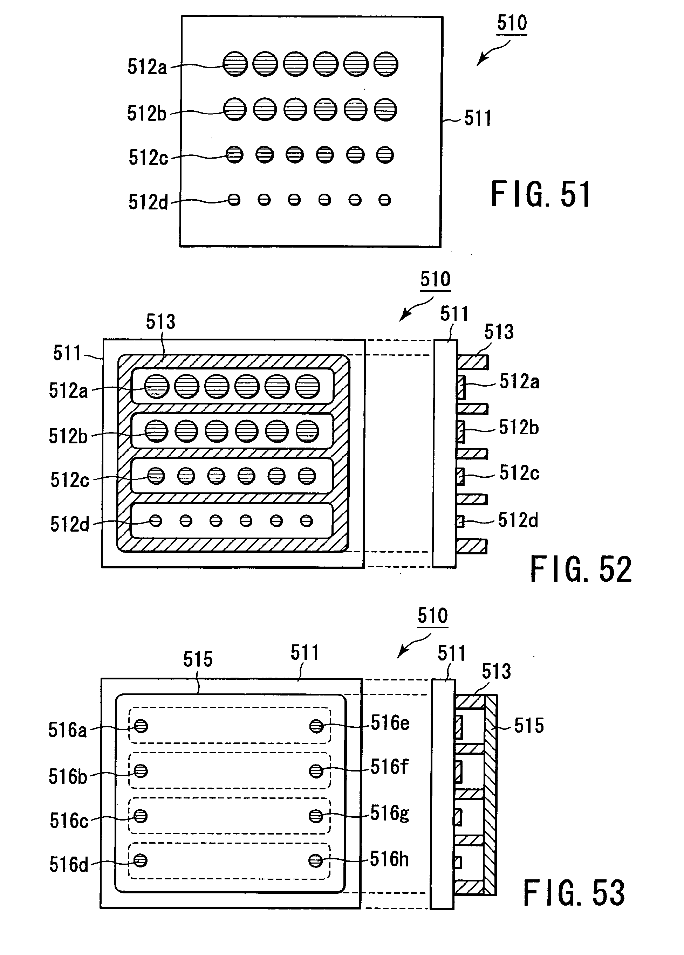

FIGS. 51 to 53 show a chip 510 of Configuration Example 1. Nucleic-acid-probe-immobilized regions 512a to 512d were formed on a substrate 511. The nucleic-acid-probe-immobilized regions 512a to 512d are regularly round, and have four types of areas in which a diameter of 512a is 500 μm, that of 512b is 200 μm, that of 512c is 100 μm, and that of 512d is 50 μm. The regions are formed every six regions. The nucleic acid probes having six different types of nucleotide sequence can be immobilized in each region area. Therefore, a sample mixed with the target nucleic acids having six different types of nucleotide sequences can be detected quantitatively. A sample holding frame 513 for holding the specimen solution is formed on the substrate. The nucleic-acid-probe-immobilized regions 512a to 512d are divided for each area by the sample holding frame 513 to define cell regions 514a to 514d. Furthermore, a sample holding frame lid 515 is formed on the sample holding frame 513. Target nucleic acid sample injection ports 516a to 516d and sample discharge ports 516e to 516h are formed in the sample holding frame lid 515.

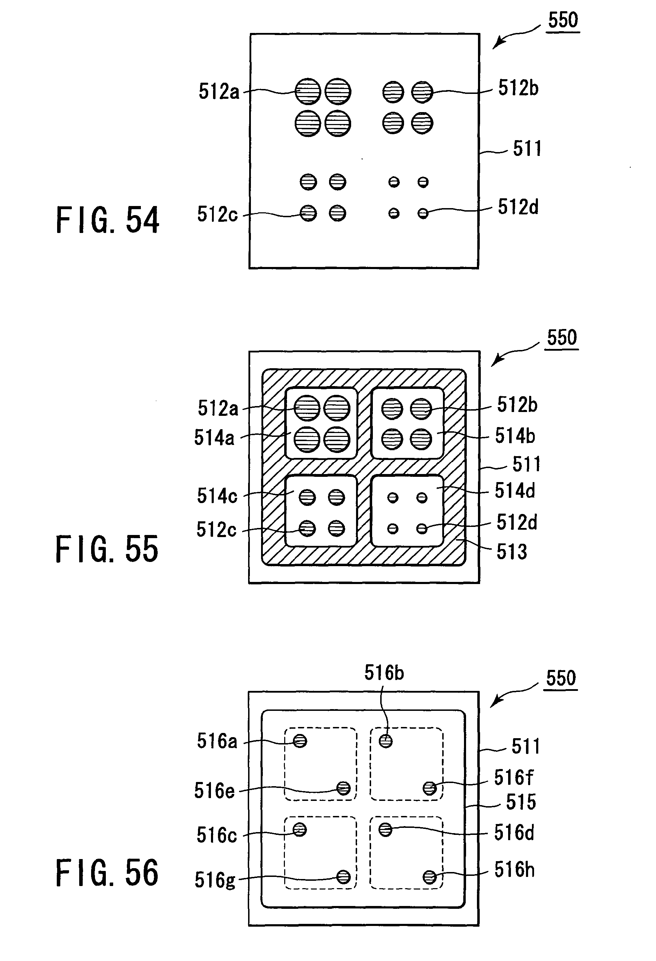

FIGS. 54 to 63C show a modification of the configuration of FIGS. 51 to 53. The same configuration as that of FIGS. 51 to 53 is denoted with the same reference numerals, and the detailed description is omitted.

FIGS. 54 to 56 show the configuration example of a chip 550 in which the nucleic-acid-probe-immobilized regions 512a to 512d are arranged without being aligned in one column. In this configuration example, the nucleic-acid-probe-immobilized regions 512a are longitudinally and transversely arranged every two regions. This also applies to the nucleic-acid-probe-immobilized regions 512b to 512d. Sample holding frame lids 516a to 516h are disposed apart from one another on a diagonal line of the cell regions 514a to 514d.

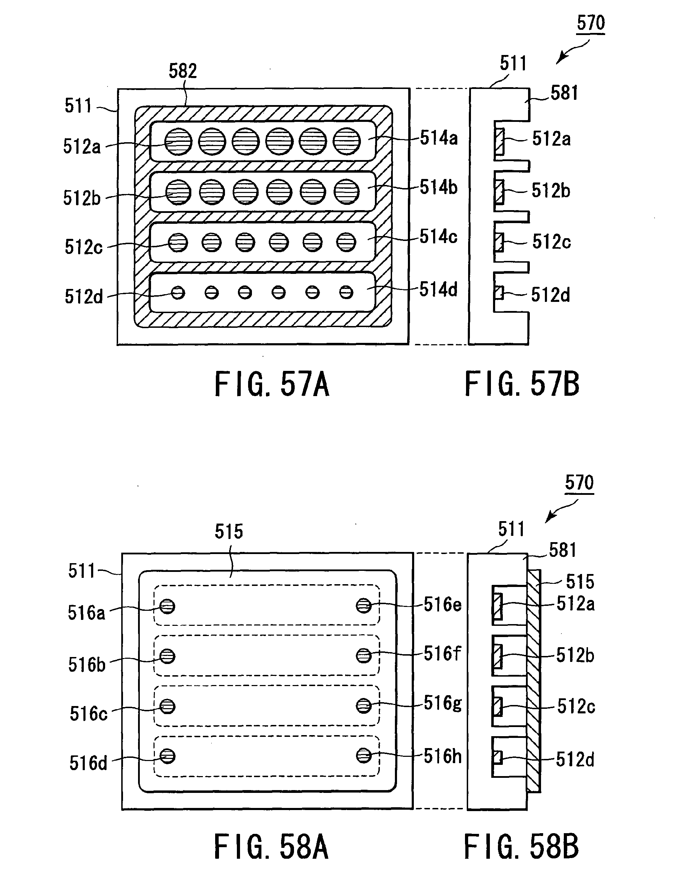

FIGS. 57A, 57B and 58A, 58B show the configuration example of a chip 570. A sample holding frame portion 581 is formed in the substrate 511 itself in the configuration example. A sample holding trench 582 is disposed by the sample holding frame portion 581 to define cell regions 514a to 514d. The other configuration is similar to that of FIGS. 51 to 53.

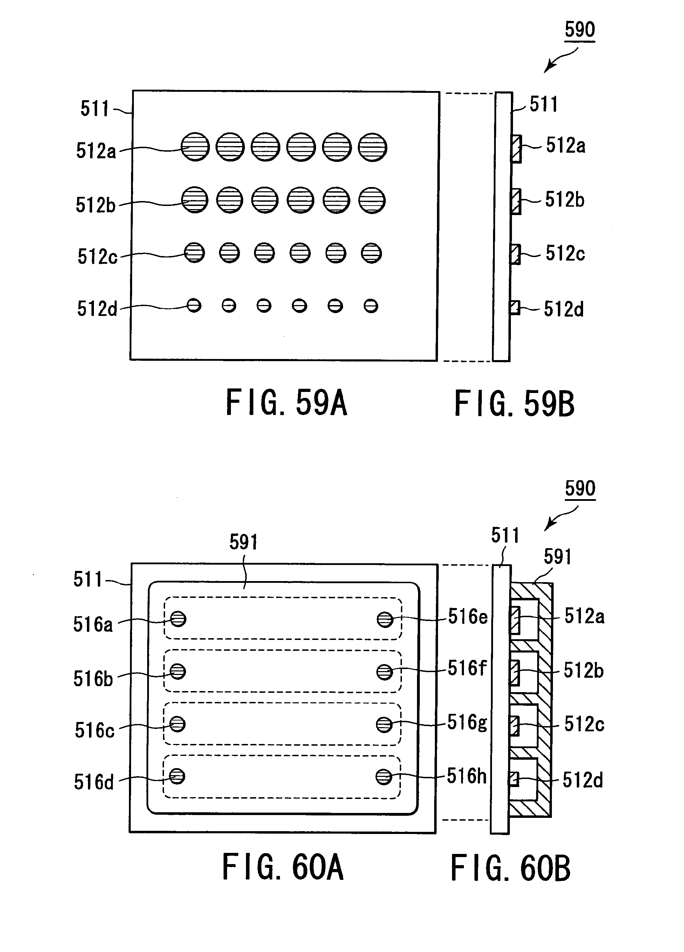

FIGS. 59A, 59B, 60A, 60B show a configuration example of a chip 590 in which the sample holding frame is integrated with the sample holding frame lid. A sample holding frame 591 shown in FIGS. 60A and 60B holds the sample and functions as the lid with respect to the sample holding frame. The other configuration is similar to that of FIGS. 51 to 53.

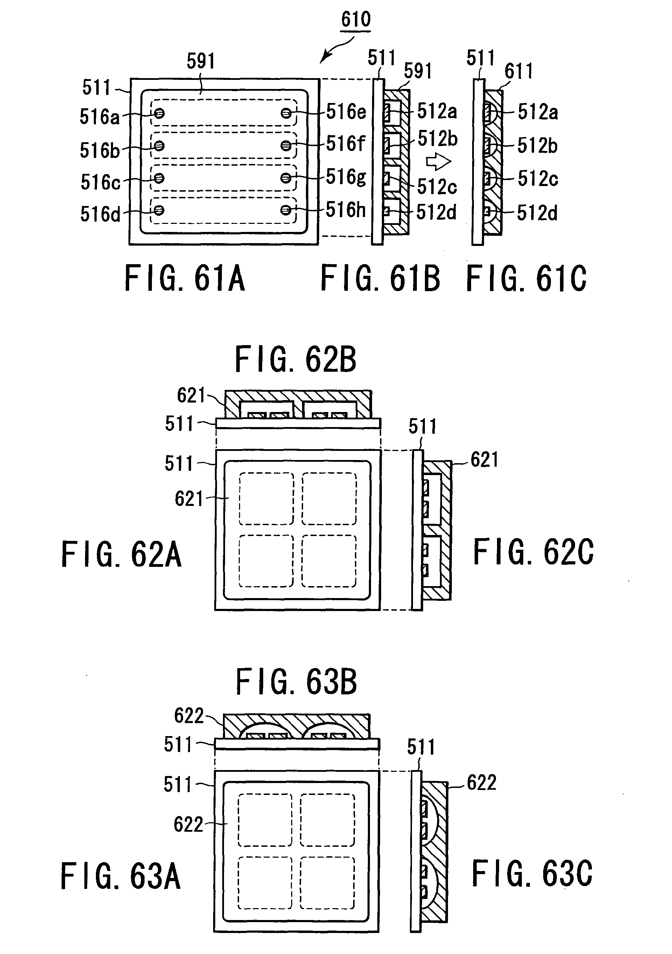

FIGS. 61A to 61C, 62A to 62C, and 63A to 63C show a modification of the sample holding frame lid.

FIGS. 61A and 61B show an example in which the sample holding frame 591 similar to that of FIGS. 59A, 59B is used. For the sample holding frame 591, side walls of the cell regions 514a to 514d are formed vertically to the substrate 511, and upper surfaces are formed horizontally with respect to the substrate 511. On the other hand, as shown in FIG. 61C, the section may also be semicircular.

Moreover, when the configuration of the sample holding frame 591 shown in FIGS. 60A and 60B is applied to that of FIGS. 54 to 56, a configuration is formed as shown in FIGS. 62A to 62C. As shown in FIGS. 62A to 62C, a sample holding frame 621 defines the nucleic-acid-probe-immobilized region in each cell region.

Furthermore, when the configuration of a sample holding frame 611 shown in FIG. 61C is applied to that of FIGS. 54 to 56, a configuration is formed as shown in FIGS. 63A to 63C. As shown in FIGS. 63A to 63C, a sample holding frame 622 having a semicircular section defines the nucleic-acid-probe-immobilized region in each cell region.

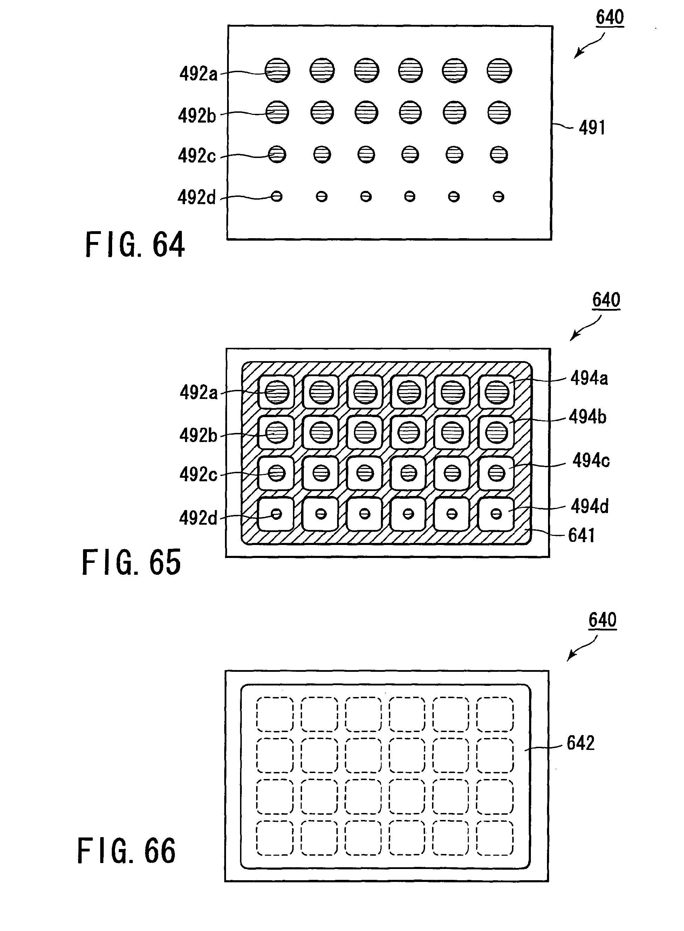

The configuration of FIGS. 54 to 63C is further applicable to that of FIGS. 64 to 82.

FIGS. 64 to 66 show a further chip modification. The basic configuration of a chip 640 shown in FIGS. 64 to 66 is common to that shown in FIGS. 49 and 50, the common configuration is denoted with the same reference numerals, and the detailed description is omitted. In the chip 640 of FIGS. 64 to 66, a plurality of nucleic-acid-probe-immobilized regions 492a to 492d shown in FIGS. 49 and 50 are disposed every plurality (six regions in the figures). Moreover, the nucleic-acid-probe-immobilized regions 492a to 492d are divided every region by a sample holding frame 641. Furthermore, a sample holding frame lid 642 is disposed on the sample holding frame 641. Accordingly, the cell regions 494a to 494d are defined for each of the nucleic-acid-probe-immobilized regions 492a to 492d. This configuration is usable in a case where there are many types of specimen solution samples and the samples are not mixed.

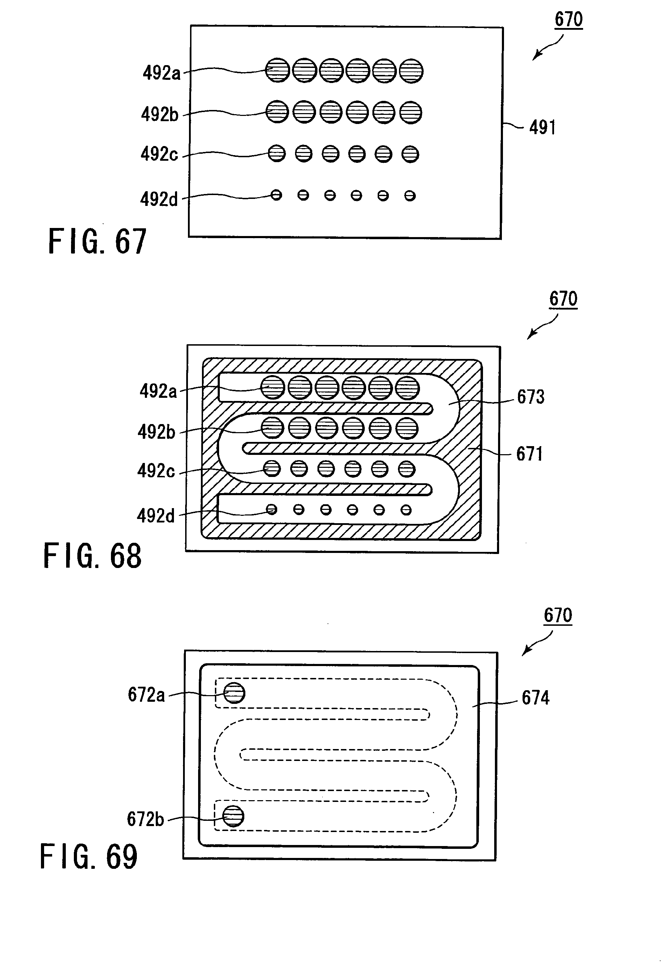

FIGS. 67 to 69 show a further chip modification. The basic configuration of a chip 670 shown in FIGS. 67 to 69 is common to that shown in FIGS. 64 to 66, the common configuration is denoted with the same reference numerals, and the detailed description is omitted. In the chip 670, for the nucleic-acid-probe-immobilized regions 492a to 492d, the regions having the equal area are disposed in the vicinity. A meandered trench is formed in a sample holding frame 671. This trench and a sample holding frame lid 674 disposed on the sample holding frame 671 define a single cell region 673 having a meandered elongated shape. A sample injection port 672a and sample discharge port 672b are formed in positions corresponding to the opposite ends of the cell region 673 of the sample holding frame lid 674. Therefore, the sample spreads over all the nucleic-acid-probe-immobilized regions 492a to 492d with one sample injection.

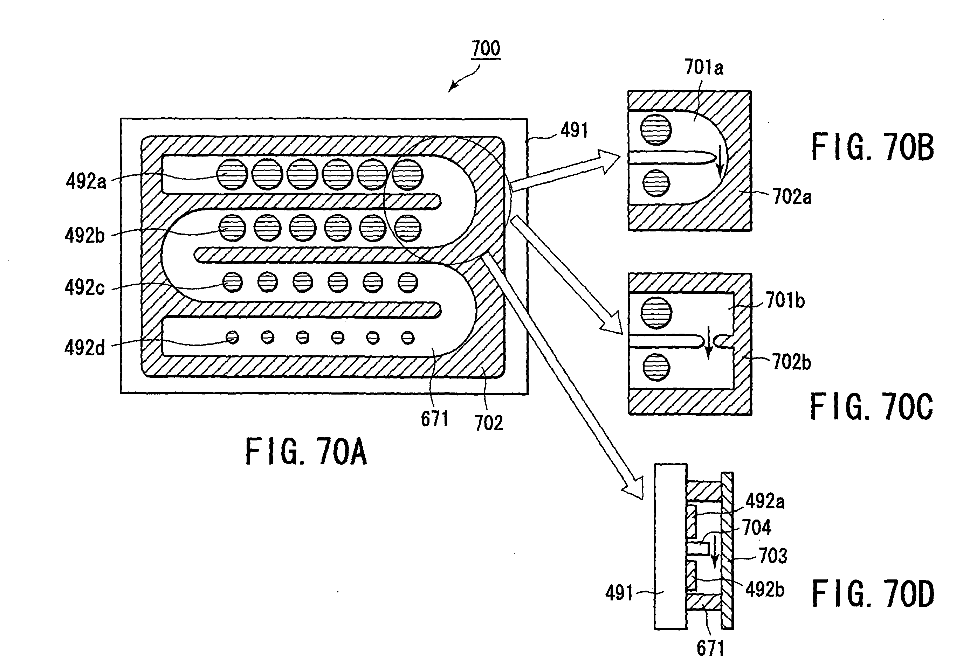

FIGS. 70A to 70D show a modification of FIGS. 67 to 69. FIG. 70A shows the same top plan view as that of FIG. 68. A modification of a bent portion of a cell region 701 of a chip 700 is shown in FIGS. 70B and 70C. The cell region 673 of FIG. 68 has a substantially constant sectional area of the meandered channel. On the other hand, cell regions 701a and 701b shown in FIGS. 70B and 70C are defined by sample holding frames 702a and 702b. In these cell regions 701a and 701b, the meandered channel does not have a constant sectional area. The sectional areas of the cell regions 701a and 701b are reduced in the meandered portions. That is, the channels are narrowed.

Accordingly, for the cell regions 701a and 701b, sample holding regions are divided for each area of the nucleic-acid-probe-immobilized regions 492a to 492d. Moreover, the divided cell regions are bonded to one another via the thin channels.

In FIGS. 70B and 70C, the shapes of the cell regions 701a and 701b seen from the upper surface are narrowed in divided positions. Alternatively, as shown in FIG. 70D, the same sample holding frame 671 as that of FIG. 68 is used, a channel restricting parts 704 is disposed in a dividing position, and the flow of the fluid may partially be restricted. In this case, a sample holding frame 703 is fixed to the sample holding frame 671, but has a gap from the channel restricting member 704.

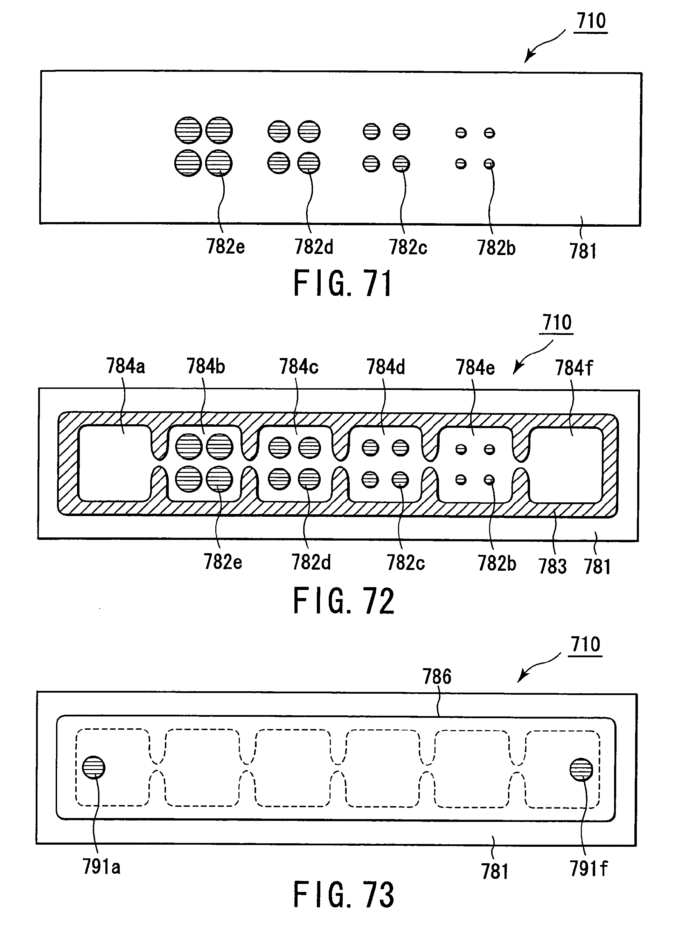

A chip 710 of FIGS. 71 to 73 shows further modification. The configuration is similar to that of FIGS. 80A to 80D and 81, the common configuration is denoted with the same reference numerals, and the detailed description is omitted. The configuration is different in that the nucleic-acid-probe-immobilized regions 782e to 782b are formed in the cell regions 784b to 784e from the sample injection port 791a to the sample discharge port 791f in order from a large area to small area . The other configuration is common to that of FIGS. 80A to 80D and 81.

(Configuration Example 2)

Configuration Example 2 is a chip configuration example in which the device including the cell region for controlling the specimen solution amount is used to perform the nucleic acid quantitative analysis.

FIGS. 74A, 74B, 75B, 76A, and 76B show a chip 740 of Configuration Example 2. The basic configuration has been described above, and is therefore omitted. The cell regions 744a to 744e constitute of the substrate 741, sample holding frame 743, and sample holding frame lid 745 have different sectional areas. In the chip 740, all the nucleic-acid-probe-immobilized regions 742 have the regular circular shape having a diameter of 50 $\mu$m. The cell regions 744a to 744e having five types of sectional areas of 0.002 mm$^2$, 0.02 mm$^2$, 0.2 mm$^2$, 2 mm$^2$ and 20 mm$^2$ were formed. The sectional areas of the cell regions 744a to 744e may be decided by either or both of the height from the substrate 741 and width. The sectional area is shown by a region surrounded with the substrate 741, sample holding frame 743, and sample holding frame lid 745 in the example of FIG. 76B.

FIGS. 77A to 77C show an example of a combination of the configuration of the chip 510 of FIGS. 51 to 53 with that of the chip 740 of FIGS. 74A, 74B, 75B, 76A, and 76B. The basic configuration of FIGS. 77A to 77C has been described above, and is therefore omitted.

For the nucleic-acid-probe-immobilized regions 772a to 772g, the region having the low nucleic acid concentration is in a detectable range in the smaller area. Furthermore, the region having the low nucleic acid concentration is in the detectable range with a more sample amount per area in the nucleic-acid-probe-immobilized regions 772a to 772g. By the combination of these configurations, the nucleic acid quantitative analysis is possible with a small sample amount in a broader range.

(Configuration Example 3)

Configuration Example 3 is a chip configuration example in a case where the nucleic acid quantitative analysis is performed using the device including the cell regions formed in such a manner that the specimen solution can be moved among the nucleic-acid-probe-immobilized regions.

FIGS. 78A to 78D and 79 are diagrams showing one example of a chip 780 of Configuration Example 3. The basic configuration has been described above, and the description thereof is omitted. In the chip 780, all the nucleic-acid-probe-immobilized regions 782 are formed in regular circles each having a diameter of 20 $\mu$m. The specimen solution is moved to the cell region 784b from 784a. After the elapse of a sufficient time for the hybridization reaction of the nucleic acid probe to the target nucleic acid, the solution is next moved to the cell region 784c. The sample is sequentially moved through all the nucleic-acid-probe-immobilized regions 782.

A chip 800 of FIGS. 80A to 80D and 81 shows a modification of the chip 780 shown in FIGS. 78A to 78D and 79. The basic configuration has been described above, and the description thereof is omitted. The nucleic-acid-probe-immobilized regions 782b to 782e having different areas are formed. Moreover, the specimen solution 785 is moved to the larger area from the smaller area. Accordingly, the quantitative range can be broadened as compared with that of the chip 780.

In accordance with the present embodiment, when the specimen solutions are separated from one another for each electrode area , an quantitative analysis precision is improved without causing any nucleic acid reaction among the electrodes having different areas.

(Regarding First to Eighth Embodiments)

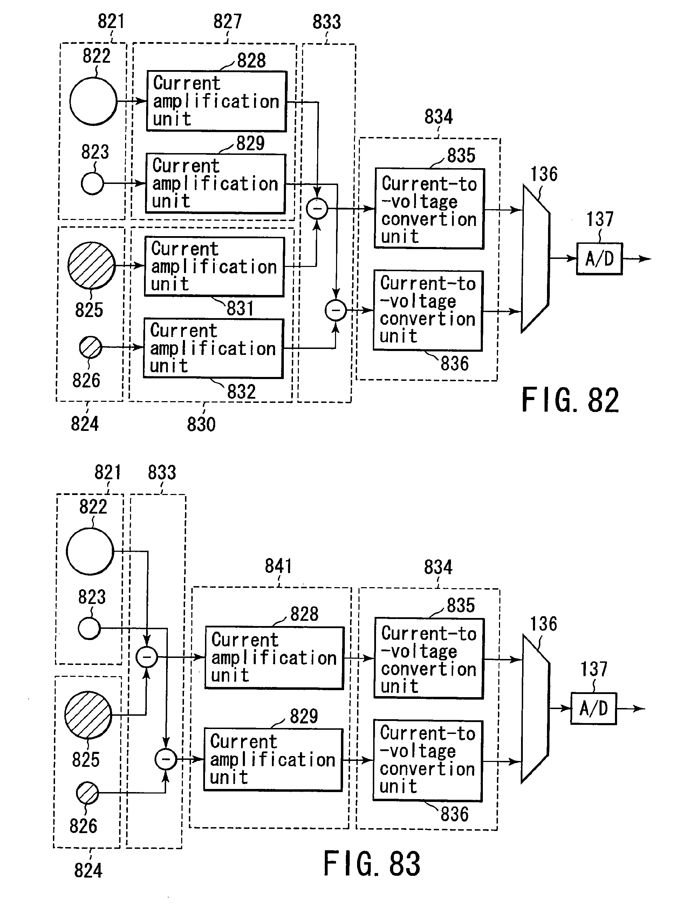

In the configurations of the first to eighth embodiments, the functional blocks of the sensor, normalization, subtraction, current-to-voltage conversion, A/D conversion and the like are shown in FIGS. 82 to 85 described below. It is to be noted that for the sake of convenience of description, FIGS. 82 to 85 show an example in which two sensors 822, 823 for probe current measurement, having different electrode areas, and two sensors 825, 826 for background current measurement, having different electrode areas are arranged, but, needless to say, the present invention is not limited to this. Three or more sensors having different electrode areas may also be arranged.

FIG. 82 is a diagram showing functions of configurations shown in FIG. 39 of the fourth embodiment . As shown in FIG. 82, a nucleic acid detecting sensor section 821, and a background level detecting sensor section 824 are disposed. The nucleic acid detecting sensor section 821 includes a sensor 822 having an electrode area $A_0$, and a sensor 823 having an electrode area $\alpha A_0$ ($\alpha < 1$). The background level detecting sensor section 824 includes a sensor 825 having an electrode area $A_0$, and a sensor 826 having an electrode area $\alpha A_0$.

A normalization section 827 is disposed on an output node of the nucleic acid detecting sensor section 821. The normalization section 827 comprises current amplification sections 828 and 829. The current amplification section 828 amplifies an output current of the sensor 822 by one times, and outputs the current to a subtraction section 833. The current amplification section 829 amplifies an output current of the sensor 823 by $1/\alpha$ times, and outputs the current to the subtraction section 833.

A normalization section 830 is disposed on an output node of the background level detecting sensor section 824. The normalization section 830 comprises current amplification sections 831 and 832. The current amplification section 831 amplifies an output current of the sensor 825 by one times, and outputs the current to the subtraction section 833. The current amplification section 832 amplifies an output current of the sensor 826 by $1/\alpha$ times, and outputs the current to the subtraction section 833.

The subtraction section 833 subtracts the output current of the current amplification section 831 from that of the current amplification section 828 to output the current to a current-to-voltage conversion section 834. The subtraction section 833 also subtracts the output current of the current amplification section 832 from that of the current amplification section 829 to output the current to the current-to-voltage conversion section 834.

The current-to-voltage conversion section 834 comprises two current-to-voltage conversion sections 835 and 826. The current-to-voltage conversion section 835 converts subtraction output currents with respect to the sensors 822 and 825, each of which have the electrode area $A_0$, to voltages to output the voltages to a selector 136. The current-to-voltage conversion section 836 converts the subtraction output currents with respect to the sensors 823 and 826, each of which have the electrode area $\alpha A_0$, to voltages to output the voltages to the selector 136.

The functions of the selector 136 and A/D converter 137 are common to those described in the above-described embodiments.

FIG. 83 is a functional block diagram showing an embodiment in which FIG. 36 is applied to FIG. 33. The configuration common to that of FIG. 82 is denoted with the same reference numerals, and detailed description is omitted. In FIG. 83, the output currents of the sensors 822, 823, 825 and 826 are output to the subtraction section 833. The subtraction section 833 subtracts the output current of the sensor 825 from that of the sensor 822 to output the current to a current amplification section 842 of a normalization section 841. The subtraction section 833 subtracts the output current of the sensor 826 from that of the sensor 823 to output the current to a current amplification section 843 of the normalization section 841.

The current amplification section 842 amplifies the subtraction output current by one times to output the current to the current-to-voltage conversion section 835. The current amplification section 843 amplifies the subtraction output current by $1/\alpha$ times to output the current to the current-to-voltage conversion section 836. The function of the subsequent stage from the current-to-voltage conversion section 834 is common to that of FIG. 82.

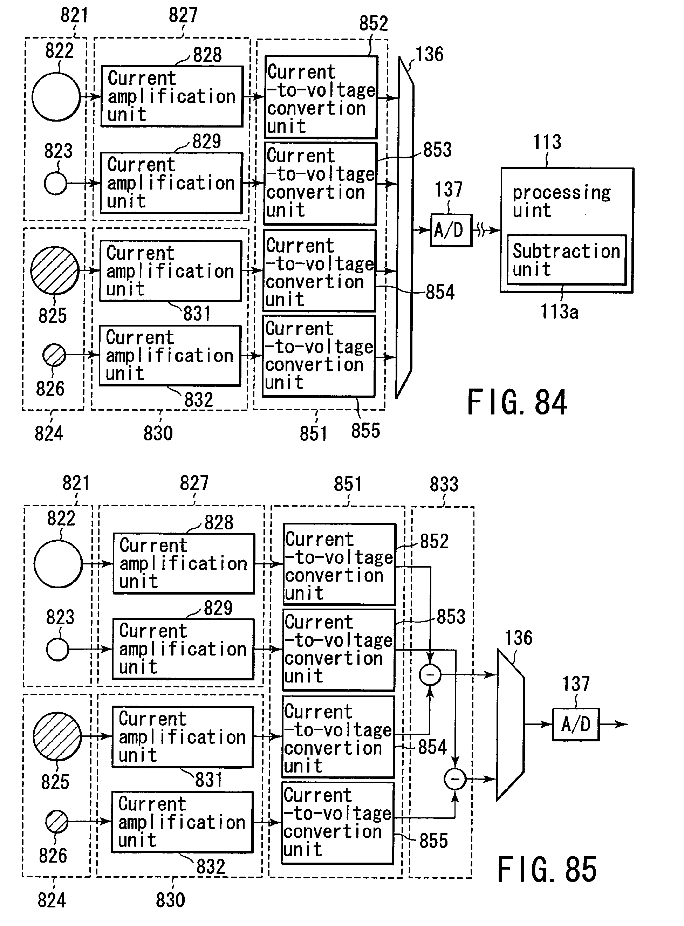

FIG. 84 shows an embodiment in which the subtraction is executed in the processing unit 113 outside the nucleic acid detection chip 12 in the configuration of the first embodiment. The configuration including the nucleic acid detecting sensor section 821, background level detecting sensor section 824, and normalization sections 827 and 830 is common to the example of FIG. 82. The respective output currents of the current amplification sections 828, 829, 831, and 832 are output to current-to-voltage conversion sections 852 to 855. The current-to-voltage conversion sections 852 to 855 convert the respective outputs to the voltages to output the voltages to the selector 136. Each output voltage is output to the processing unit 113 outside the nucleic acid detection chip 12 via the selector 136, and A/D converter 137. The subtraction section 113a in the processing unit 113 subtracts output data of the sensor 825 from that of the sensor 822, and subtracts output data of the sensor 826 from that of the sensor 823.

FIG. 85 is a functional block diagram showing the configuration shown in FIGS. 14, 15, 16, and 19. The configuration including the nucleic acid detecting sensor section 821, the background level detecting sensor section 824, the normalization sections 827 and 830, and a current-to-voltage conversion circuit 851 is common to the example of FIG. 84. The subtraction section 833 subtracts the output voltage of the current-to-voltage conversion circuit 854 from that of the current-to-voltage conversion section 852 to output the voltage to the selector 136. Also, the subtraction section 833 subtracts the output voltage of the current-to-voltage conversion circuit 855 from that of the current-to-voltage conversion section 853 to output the voltage to the selector 136.

It is to be noted that these configurations shown in FIGS. 82 to 85 are illustrations, and the order of the respective configurations may variously be changed.

As described above, according to the present embodiment, the nucleic acid concentration can be measured in a broad dynamic range with high precision.

Industrial Applicability

As described above, the present invention is effective for technical fields of a nucleic acid concentration quantitative analysis chip, nucleic acid concentration quantitative analysis apparatus, and nucleic acid concentration quantitative analysis method in which a concentration of a target nucleic acid contained in a specimen is quantitatively analyzed.

**Claims**

1.    A nucleic acid concentration quantitative analysis chip **characterized by** comprising:

    a plurality of nucleic acid sensors having different sensor areas on which nucleic acid probes each having a nucleic acid complementary to a target nucleic acid are immobilized; and
    a first normalization unit which normalizes first detection signals obtained by the nucleic acid sensors with respect to the respective sensor areas.

2.    The nucleic acid concentration quantitative analysis chip according to claim 1, **characterized by** further comprising:

    a plurality of background level sensors which have the different sensor areas on which the nucleic acid probes having the nucleic acids complementary to the target nucleic acids are not immobilized;
    a second normalization unit which normalizes second detection signals obtained by the background level sensors with respect to the respective sensor areas; and
    a subtraction circuit which subtracts a second output signal of the second normalization unit from a first output of the first normalization unit.

3.    The nucleic acid concentration quantitative analysis chip according to claim 1, **characterized by** further comprising:

    a plurality of background level sensors which have the different sensor areas on which the nucleic acid probes having the nucleic acids complementary to the target nucleic acids are not immobilized;
    a second normalization circuit which normalizes second detection signals obtained by the background level sensors with respect to the respective sensor areas;
    a subtraction unit which subtracts a second output signal of the second normalization unit from a first output of the first normalization unit; and
    a current-to-voltage conversion unit which converts an output signal current of the subtraction unit to a voltage.

4.    The nucleic acid concentration quantitative analysis chip according to claim 1, **characterized in that** the first normalization unit comprises:

    a first current mirror which duplicates and amplifies a first current of the first detection signal detected from the nucleic acid sensor and outputs that amplified current if the first current value is positive; and
    a second current mirror which duplicates and amplifies the first current and outputs that amplified current if the first current value is negative.

**5.** The nucleic acid concentration quantitative analysis chip according to claim 1, **characterized by** further comprising:

a plurality of background level sensors which have the different sensor areas on which the nucleic acid probes having the nucleic acids complementary to the target nucleic acids are not immobilized;
a second normalization circuit which normalizes second detection signals obtained by the background level sensors with respect to the respective sensor areas;
a subtraction unit which subtracts an second output signal of the second normalization unit from an first output of the first normalization unit,
wherein the first normalization circuit comprises:

a first current mirror which duplicates and amplifies a first current of the first detection signals detected from the nucleic acid sensor if the first current value is positive; and
a second current mirror which duplicates and amplifies the first current if the first current value is negative, the second normalization circuit comprises:

a third current mirror which duplicates and amplifies a second current of the second detection signals detected by the background level sensor if the second current value is positive; and
a fourth current mirror which duplicates and amplifies the second current if the second current value is negative, and
the subtraction circuit subtracts a third output current of the third current mirror from a first output current of the first current mirror and subtracts a fourth output current of the fourth current mirror from a second output current of the second current mirror.

**6.** The nucleic acid concentration quantitative analysis chip according to claim 1, **characterized by** further comprising:

a plurality of cells, each of which house one or more of the nucleic acid sensors, which are separated from one another in accordance with the areas of the nucleic acid sensors.

**7.** The nucleic acid concentration quantitative analysis apparatus according to claim 1, **characterized in that** the nucleic acid sensor comprises a first sensor and a second sensor having an electrode area smaller than that of the first sensor, and a first measurement range of the first sensor overlaps with a second measurement range of the second sensor.

**8.** The nucleic acid concentration quantitative analysis apparatus according to claim 1, **characterized in that** the nucleic acid sensor comprises a first sensor and a second sensor having an electrode area smaller than that of the first sensor, a first measurement range of the first sensor overlaps with a second measurement range of the second sensor, and
if a dynamic range $d_1$(dec) of the first sensor is defined as a first ratio of a first upper end to a first lower end of the first the measurement range, the number of first nucleic acid probes of the first sensor is $N_1$, the number of second nucleic acid probes of the second sensor is $N_2$, a second ratio of a second upper end to a second lower end of a nucleic acid concentration region in which the first the measurement range overlaps with the second measurement range is $d_{1,2}$(dec), and a ratio $d_{1,2}/d_1$ of $d_{1,2}$ to $d_1$ is $\gamma$ ($0 \leq \gamma < 1$), the following is satisfied:

$$10^{d_1 (1-\gamma)} = \frac{N_1}{N_2} \, .$$

**9.** The nucleic acid concentration quantitative analysis chip according to claim 1, **characterized in that** the nucleic acid sensor comprises a first sensor and a second sensor having an electrode area smaller than that of the first sensor, a first measurement range of the first sensor overlaps with a second measurement range of the second sensor, and
if a dynamic range $d_1$(dec) of the first sensor is defined as a first ratio of a first upper end to a first lower end of the first the measurement range, the number of first nucleic acid probes of the first sensor is $N_1$, the number of second nucleic acid probes of the second sensor is $N_2$, a second ratio of a second upper end to a second lower end of a nucleic acid concentration region in which the first the measurement range overlaps with the second measurement range is $d_{1,2}$(dec), a ratio $d_{1,2}/d_1$ of $d_{1,2}$ to $d_1$ is $\gamma$ ($0 \leq \gamma < 1$), the following is satisfied:

$$10^{d_1\,(1-\gamma)} = \frac{N_1}{N_2},$$

and the value $\gamma$ satisfies $\gamma \le 0.85$.

10. The nucleic acid concentration quantitative analysis apparatus according to claim 1, **characterized in that** the nucleic acid sensor comprises a first sensor and a second sensor having an electrode area smaller than that of the first sensor, a first measurement range of the first sensor overlaps with a second measurement range of the second sensor, and

if a dynamic range $d_1$(dec) of the first sensor is defined as a first ratio of a first upper end to a first lower end of the first the measurement range, the number of first nucleic acid probes of the first sensor is $N_1$, the number of second nucleic acid probes of the second sensor is $N_2$, a second ratio of a second upper end to a second lower end of a nucleic acid concentration region in which the first the measurement range overlaps with the second measurement range is $d_{1,2}$(dec), a ratio $d_{1,2}/d_1$ of $d_{1,2}$ to $d_1$ is $\gamma$ ($0 \le \gamma < 1$), the following is satisfied:

$$10^{d_1\,(1-\gamma)} = \frac{N_1}{N_2},$$

and the first and second dynamic ranges $d_1$ and $d_2$ satisfy a relation of $d_1 = d_2$.

11. The nucleic acid concentration quantitative analysis apparatus according to claim 1, **characterized in that** the nucleic acid sensor comprises a first sensor and a second sensor having an electrode area smaller than that of the first sensor,

a first measurement range of the first sensor overlaps with a second measurement range of the second sensor, and

if first and second electrode areas of the first and second sensors are defined as $S_1$ and $S_2$ respectively, a relation of $0.05 \le S_2/S_1 \le 0.5$ is satisfied in a case where a number of the nucleic acid probe molecules proportional to the electrode area are immobilized on the first and second sensors respectively.

12. The nucleic acid concentration quantitative analysis chip according to claim 1, **characterized in that** the nucleic acid sensor comprises a first sensor, a second sensor having an electrode area smaller than that of the first sensor, and a third sensor having an electrode area smaller than that of the second sensor, a first measurement range of the first sensor overlaps with a second measurement range of the second sensor, and

if the electrode areas of the first to third sensors are defined as $S_1$, $S_2$, and $S_3$, a relation of $S_2/S_1 = S_3/S_2$ is satisfied in a case where the nucleic acid probes are immobilized on the first to third sensors in proportion to the electrode areas.

13. The nucleic acid concentration quantitative analysis apparatus according to claim 1, **characterized in that** the nucleic acid sensor comprises a first sensor and a second sensor having an electrode area smaller than that of the first sensor,

a first measurement range of the first sensor overlaps with a second measurement range of the second sensor,

if first and second electrode areas of the first and second sensors are defined as $S_1$ and $S_2$, a relation of $0.05 \le S_2/S_1 \le 0.5$ is satisfied in a case where a number of nucleic acid probe molecules proportional to the electrode area are immobilized on the first and second sensors, and

sensor areas of the plurality of nucleic acid sensors substantially form a geometric progression.

14. The nucleic acid concentration quantitative analysis apparatus according to claim 1, **characterized in that** the nucleic acid sensor comprises a first sensor, a second sensor having an electrode area smaller than that of the first sensor, and a third sensor having an electrode area smaller than that of the second sensor, a first measurement range of the first sensor overlaps with a second measurement range of the second sensor, and

if the electrode areas of the first to third sensors are defined as $S_1$, $S_2$, and $S_3$, a relation of $0.05 \le S_2/S_1 = S_3/S_2 \le 0.5$ is satisfied in a case where the nucleic acid probes are immobilized on the first to third sensors in proportion to the electrode areas.

15. A nucleic acid concentration quantitative analysis apparatus **characterized by** comprising:

a plurality of nucleic acid sensors having different sensor areas on which nucleic acid probes each having a nucleic acid complementary to a target nucleic acid are immobilized;

a plurality of background level sensors which have the different sensor areas on which the nucleic acid probes having the nucleic acids complementary to the target nucleic acids are not immobilized;

a first normalization unit which normalizes first detection signals obtained by the nucleic acid sensors with respect to the respective sensor areas;

a second normalization unit which normalizes second detection signals obtained by the background level sensors with respect to the respective sensor areas;

a first current-to-voltage conversion unit which converts a first output signal current of the first normalization unit to a first output voltage;

a second current-to-voltage conversion unit which converts a second output signal current of the second normalization unit to a second output voltage;

an A/D conversion unit which A/D converts the first output voltage to generate first digital data and which A/D converts the second output voltage to generate second digital data; and

a subtraction unit which subtracts the second digital data from the first digital data.

16. A nucleic acid concentration quantitative analysis apparatus **characterized by** comprising:

a plurality of nucleic acid sensors having different sensor areas on which nucleic acid probes each having a nucleic acid complementary to a target nucleic acid are immobilized;

a plurality of background level sensors which have the different sensor areas on which the nucleic acid probes having the nucleic acids complementary to the target nucleic acids are not immobilized;

a first normalization unit which normalizes first detection signals obtained by the nucleic acid sensors with respect to the respective sensor areas;

a second normalization unit which normalizes second detection signals obtained by the background level sensors with respect to the respective sensor areas;

a first current-to-voltage conversion unit which converts a first output signal current of the first normalization unit to a first output voltage;

a second current-to-voltage conversion unit which converts a second output signal current of the second normalization unit to a second output voltage;

a subtraction unit which subtracts the second output voltage from the first output voltage; and

an A/D conversion unit which executes A/D conversion of a third output voltage of the subtraction unit.

17. A nucleic acid concentration quantitative analysis chip **characterized by** comprising:

a plurality of nucleic acid sensors having different sensor areas on which nucleic acid probes each having a nucleic acid complementary to a target nucleic acid are immobilized;

a plurality of background level sensors which have the different sensor areas on which the nucleic acid probes having the nucleic acids complementary to the target nucleic acids are not immobilized;

a subtraction unit which subtracts a second detection signal of the background level sensor from a first detection signal of the nucleic acid sensor; and

a normalization unit which normalizes a subtraction output signal of the subtraction unit.

18. The nucleic acid concentration quantitative analysis chip according to claim 17, **characterized by** further comprising a current-to-voltage conversion unit which converts an output signal current of the normalization unit to a voltage.

19. A nucleic acid concentration quantitative analysis apparatus **characterized by** comprising:

a nucleic acid concentration quantitative analysis chip including:

a plurality of nucleic acid sensors having different sensor areas on which nucleic acid probes each having a nucleic acid complementary to a target nucleic acid are immobilized; and

a first normalization unit which normalizes a detection signal of the nucleic acid sensor with respect to the sensor area to output a normalized signal, and

a nucleic acid concentration calculation device which calculates a nucleic acid concentration based on the normalized signal.

**20.** The nucleic acid concentration quantitative analysis apparatus according to claim 19, **characterized in that** the nucleic acid concentration calculation device compares the normalized signal with a predetermined threshold value to acquire binary bit data with respect to each sensor area, and collates the binary bit data with a judgment table in which binary judgment bit data is associated with a concentration of the nucleic acid with respect to each sensor area beforehand to determine the nucleic acid concentration.

**21.** The nucleic acid concentration quantitative analysis apparatus according to claim 19, **characterized by** further comprising saturated level sensors having different sensor areas on which double stranded nucleic acids composed of the target nucleic acid and the probe nucleic acid are immobilized.

**22.** A nucleic acid concentration quantitative analysis method **characterized by** comprising:

normalizing detection signals of a plurality of nucleic acid sensors on which nucleic acid probes each having a nucleic acid complementary to a target nucleic acid are immobilized and which have different sensor areas with respect to sensor areas to output a normalized signal; and
calculating a nucleic acid concentration based on the normalized signal.

FIG. 1

FIG. 2

FIG. 3

FIG. 4

FIG. 5

FIG. 6

EP 1 530 043 A1

Area A₀    Area αA₀    Area α²A₀    Area α³A₀    . . .

## FIG. 7

Signal intensity

1.0
0.9
0.8
0.7
0.6
0.5
0.4
0.3
0.2
0.1
0.0

Signal

Background

$\alpha^7A_0$  $\alpha^6A_0$  $\alpha^5A_0$  $\alpha^4A_0$  $\alpha^3A_0$  $\alpha^2A_0$  $\alpha A_0$  $A_0$

Sensor area

## FIG. 8

Quantitative
analysis start

Calibration — S1

Measurement — S2

Quantitative
analysis end

## FIG. 9

$$\boxed{\text{Calibration start}}$$

| Immerse sensor in solution T containing no nucleic acid | S11 |

| Current value acquisition | S12 |

Measure solution S containing nucleic acid — S13

| Supply nucleic acid solution $S_i$ having concentration measured beforehand to sensor | S14 |

| Current value acquisition | S15 |

Solution $S_{i+1}$ having next nucleic acid concentration — S16

| Calculation of threshold value | S17 |

S18

Collate with current value obtained in each electrode in each measurement of $S_i$ ($i = 0, 1, \ldots N-1$), judge 1 with normalized current value exceeding threshold value and 0 with value not exceeding threshold value, perform this operation in whole electrode series, acquire one set of obtained data as bit pattern

S19

Bit pattern and concentration have one-to-one correspondence ? — No

Yes

Store threshold value, hybridization conditions (time, temperature and the like), correspondence between bit pattern and concentration as numeric table — S20

## FIG. 10

$$\boxed{\text{Calibration end}}$$

Current value acquisition start

Perform hybridization at constant temperature for certain time — S31

Supply intercalating agent with electrodes different in area to measure current value — S32

Normalize current value with area — S33

Obtain peak value height by fitting — S34

Current value acquisition end

## FIG. 11

Measurement start

Immerse sensor in solution of specimen — S21

Current value acquisition — S22

Compare with threshold value obtained by calibration to obtain bit (0,1) in each electrode — S23

Compare bit pattern of whole electrode series with numeric table obtained by calibration to calculate concentration — S24

Measurement end

## FIG. 12

FIG. 13

Voltage sweeping signal

| Voltage application circuit | $201_1$ |

140b Three electrode system for
background signal measurement

| Current detection circuit and normalization circuit | $160_1$ |

| Voltage application circuit | $201_2$ |

140d Three electrode system
for probe

Selection
signal

| Current detection circuit and normalization circuit | $160_2$ |

| Subtraction circuit |

Data

$202_2$

136

FIG. 14

FIG. 15

FIG. 16

EP 1 530 043 A1

Calibration start

Measure solution S containing nucleic acid — S41

Supply nucleic acid solution $S_i$ having concentration measured beforehand to sensor — S42

Current value acquisition — S43

Solution $S_{i+1}$ having next nucleic acid concentration — S44

Determine threshold value — S45

S46

Collate with current value obtained in each electrode in each measurement of $S_i$ ($i = 0, 1, 2, ... N-1$), judge 1 when normalized current value exceeds threshold value and 0 when value does not exceed threshold value, perform this operation in whole electrode series, acquire one set of obtained data as bit pattern

S47

Bit pattern and concentration have one-to-one correspondence ?    No

Yes

Store threshold value, hybridization conditions (time, temperature and the like), correspondence between bit pattern and concentration as numeric table — S48

Calibration end

FIG. 17

```
        ╭─────────────────────────╮
        │     Current value       │
        │   acquisition start     │
        ╰─────────────────────────╯
                     │
                     ▼
   ┌─────────────────────────────────┐
   │ Perform hybridization at constant│───── S431
   │ temperature for certain time     │
   └─────────────────────────────────┘
                     │
                     ▼
   ┌─────────────────────────────────┐
   │ Supply intercalating agent with  │
   │ electrodes different in area to  │───── S432
   │ measure current value            │
   └─────────────────────────────────┘
                     │
                     ▼
   ┌─────────────────────────────────┐
   │ Normalize background level and   │───── S433
   │ current value with area          │
   └─────────────────────────────────┘
                     │
                     ▼
   ┌─────────────────────────────────┐
   │ Subtract background level        │───── S434
   │ from measured value              │
   └─────────────────────────────────┘
                     │
                     ▼
   ┌─────────────────────────────────┐
   │ Obtain peak by height fitting    │───── S435
   └─────────────────────────────────┘
                     │
                     ▼
        ╭─────────────────────────╮
        │     Current value       │
        │   acquisition end       │
        ╰─────────────────────────╯
```

FIG. 18

Voltage sweeping signal

Voltage application circuit — 2011

140b Three electrode system for background signal measurement

Current detection circuit and normalization circuit — 1601

Voltage application circuit — 2012

140d Three electrode system for probe

Current detection circuit and normalization circuit — 1602

Subtraction circuit — 202

Voltage application side circuit — 2013

140s Three-electrode system for saturated level calibration

Selection signal

Current detection circuit and normalization circuit — 1603

Subtraction circuit — 302

Data

136

FIG. 19

Calibration start

Measure solution S containing nucleic acid — S51

Supply nucleic acid solution $S_i$ having concentration measured beforehand to sensor — S52

Current value and bit pattern acquisition — S53

Solution $S_{i+1}$ having next nucleic acid concentration — S54

Store hybridization conditions (time, temperature and the like), and correspondence between bit pattern and concentration as numeric table — S55

FIG. 20

Calibration end

Measurement start

Supply specimen to sensor — S61

Acquire current value and bit pattern — S62

Compare bit pattern of whole electrode series with numeric table obtained by calibration to calculate concentration — S63

FIG. 22

Measurement end

```
         ╭─────────────────────────╮
         │  Current value and bit  │
         │  data acquisition start │
         ╰─────────────────────────╯
                      │
                      ▼
    ┌───────────────────────────────────┐
    │ Perform hybridization at constant │──── S541
    │ temperature for certain time      │
    └───────────────────────────────────┘
                      │
                      ▼
    ┌───────────────────────────────────┐
    │ Supply intercalating agent with   │──── S542
    │ electrodes  different in area to  │
    │ measure current value             │
    └───────────────────────────────────┘
                      │
                      ▼
    ┌───────────────────────────────────┐
    │ Normalize background level,       │──── S543
    │ measurement current, and saturated│
    │ level with area                   │
    └───────────────────────────────────┘
                      │
                      ▼
    ┌───────────────────────────────────┐
    │ Subtract background level from    │──── S544
    │ saturated level and measured value│
    └───────────────────────────────────┘
                      │
                      ▼
    ┌───────────────────────────────────┐
    │ Obtain saturated level and peak   │──── S545
    │ value of measured value by fitting│
    └───────────────────────────────────┘
                      │
                      ▼
    ┌───────────────────────────────────┐
    │ Determine the half of saturated   │──── S546
    │ level as threshold value          │
    └───────────────────────────────────┘
                      │
                      ▼
    ┌───────────────────────────────────┐
    │ Output 1 for measured value >     │──── S547
    │ threshold value, otherwise 0      │
    └───────────────────────────────────┘
                      │
                      ▼
         ╭─────────────────────────╮
         │  Current value and bit  │
         │  data acquisition end   │
         ╰─────────────────────────╯
```

# FIG. 21

FIG. 23

FIG. 24

**FIG. 25**

FIG. 26

FIG. 27

FIG. 28

Level determining
algorithm start

Acquire data of nucleic acid group $S_i$ (i = 0, 1, 2...N-1) with different concentrations given beforehand ~S81

Divide acquired each current peak value by electrode area $A_j$ (j = 0, 1, 2...M-1), obtain current surface density (area normalized current value) In ~S82

Assume data $I_n(N-1, 0)$ obtained from smallest electrode $A_{M-1}$ (j=0) with nucleic acid $S_{N-1}$ having highest concentration as saturated level, and data $I_0(0, M-1)$ obtained from largest electrode $A_0$(j=$M$-1) with nucleic acid $S_0$ having lowest concentration as background level ~S83

Calculate $I_0(i, j) = \{I_n(i, j) - I_n(0, 0)\} / \{I_n(N-1, M-1) - I_n(0, 0)\}$ ~S84

Fit data series i=1, 2, ... M-1 with sigmoid function
S85

Change assumed saturated level, background level
S87

S86
Fitting error not more than predetermined value ? — No

Yes

Determine assumed values in S84 or S87 as saturated level, background level ~S88

Level determining algorithm end

FIG. 29

FIG. 30

EP 1 530 043 A1

FIG. 31

FIG. 32

FIG. 33

FIG. 34

340

$I_{IN}$

332

341

$V_{OUT}$

331

FIG. 35

350

332

342

343

$I_{IN}$

$V_{OUT}$

331

FIG. 36

360

1 : B

$+V_S$   $+V_S$

M4   M6

M2

$I_{IN}$

SW1

$+V_S$

M7

$V_{OUT}$

$B I_{IN}$

M8

SW2

C

M1

331

M3   M5

$-V_S$

$-V_S$   $-V_S$

**FIG. 37**

**FIG. 38**

FIG. 39

FIG. 40

FIG. 41

FIG. 42

FIG. 43

445e 444e  445d 444d  445c 444c  445b 444b  445a 444a

443e      443d      443c      443b      443a

442e      442d      442c      442b      442a  441

# FIG. 44

452

450

453

451a  451h

451b  451g

451c  451f

451d  451e

454c

453c

454f

453f

# FIG. 45

F I G. 46

FIG. 47

Signal

473
Saturated level

471

472 Background level

Nucleic acid concentration

FIG. 48

Normalized signal

Saturated level

484 483 482 481

Small area ← → Large area

Background level

Nucleic acid concentration

FIG. 49

492a

492b

492c

492d

491

FIG. 50

493

494a — 492a
494b — 492b
494c — 492c
494d — 492d
491

FIG. 51

FIG. 52

FIG. 53

FIG. 54

512a — 512b
511
512c — 512d

FIG. 55

512a — 512b
514a — 514b
511
514c — 514d
512c — 512d
513

FIG. 56

516b
516a — 511
516e — 516f
515
516c — 516d
516g — 516h

FIG. 57A  FIG. 57B

FIG. 58A  FIG. 58B

590

511

512a

512b

512c

512d

## FIG. 59A    FIG. 59B

590

591

511

511

591

516a    516e    512a

516b    516f    512b

516c    516g    512c

516d    516h    512d

## FIG. 60A    FIG. 60B

FIG. 61A   FIG. 61B   FIG. 61C

FIG. 62B

FIG. 62A   FIG. 62C

FIG. 63B

FIG. 63A   FIG. 63C

640

492a

492b

492c

492d

491

FIG. 64

640

492a
492b
492c
492d

494a
494b
494c
494d
641

FIG. 65

640

642

FIG. 66

FIG. 67

FIG. 68

FIG. 69

FIG. 70A

FIG. 70B

FIG. 70C

FIG. 70D

FIG. 71

FIG. 72

FIG. 73

FIG. 74

FIG. 75A

FIG. 75B

FIG. 76A

FIG. 76B

770

774a 774b 774c 774d    774e    774f       774g
775
773

FIG. 77C

772a 772b 772c 772d 772e    772f       772g

774a 774b 774c 774d    774e    774f       774g

770

771

773

FIG. 77B

772a 772b 772c 772d 772e    772f       772g

770

771

772a 772b 772c 772d 772e    772f       772g    FIG. 77A

FIG. 78A

FIG. 78B

FIG. 78C

FIG. 78D

FIG. 79

784a  784b  784c  784d  784e  784f
  782b  782c  782d  782e

FIG. 80A

800
781
783
785

782b 785 782c  782d  782e

FIG. 80B

800
781
783

782b 782c 785  782d  782e

FIG. 80C

800
781
783

782b  782c  782d  782e 785

FIG. 80D

800
781
783

791a  786

FIG. 81

800
781
791f

FIG. 82

FIG. 83

FIG. 84

FIG. 85

## INTERNATIONAL SEARCH REPORT

International application No.

PCT/JP2004/002205

| A. | CLASSIFICATION OF SUBJECT MATTER |
|---|---|

Int.Cl$^7$  G01N27/416, G01N27/327, G01N33/53, G01N37/00, C21M1/00,
          C12M1/34, C12Q1/68

According to International Patent Classification (IPC) or to both national classification and IPC

| B. | FIELDS SEARCHED |
|---|---|

Minimum documentation searched (classification system followed by classification symbols)

Int.Cl$^7$  G01N27/00-27/49, C12N15/00, C12M1/00-3/10, C12Q1/00-3/00,
          G01N33/53

Documentation searched other than minimum documentation to the extent that such documents are included in the fields searched
Jitsuyo Shinan Koho          1922-1996   Toroku Jitsuyo Shinan Koho   1994-2004
Kokai Jitsuyo Shinan Koho    1971-2004   Jitsuyo Shinan Toroku Koho   1996-2004

Electronic data base consulted during the international search (name of data base and, where practicable, search terms used)
JICST FILE (JOIS)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 10-146183 A (Toshiba Corp.), 02 June, 1998 (02.06.98), Full text; Figs. 1 to 16 (Family: none) | 1-22 |
| A | JP 2002-510791 A (California Institute of Technology), 09 April, 2002 (09.04.02), Full text; Figs. 1 to 9 & AU 9935506 A        & EP 1068359 A1 & WO 99/51778 A1 | 1-22 |
| A | JP 2002-195997 A (Toshiba Corp.), 10 July, 2002 (10.07.02), Full text; Figs. 1 to 21 & EP 1211507 A2        & US 2002/39743 A1 | 1-22 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 May, 2004 (24.05.04) | 08 June, 2004 (08.06.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

| | INTERNATIONAL SEARCH REPORT | International application No. |
|---|---|---|
| | | PCT/JP2004/002205 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2002-257730 A (Olympus Optical Co., Ltd.), 11 September, 2002 (11.09.02), Full text; Figs. 1 to 12 & EP 1347285 A          & WO 2002/57756 A1 | 1-22 |
| P,A | JP 2003-274945 A (Toshiba Corp.), 30 September, 2003 (30.09.03), Full text; Figs. 1 to 9 (Family: none) | 1-22 |
| P,A | JP 2003-250088 A (Toshiba Corp.), 05 September, 2003 (05.09.03), Full text; Figs. 1 to 11 (Family: none) | 1-22 |

Form PCT/ISA/210 (continuation of second sheet) (January 2004)